## (12) EUROPEAN PATENT SPECIFICATION

(45) Date of publication and mention
of the grant of the patent:
**03.07.1996 Bulletin 1996/27**

(51) Int Cl.$^6$: **C12N 15/30**, C12N 15/62,
C12P 21/00, A61K 39/012

(21) Application number: **91201751.4**

(22) Date of filing: **24.11.1986**

(54) **Antigenic proteins and vaccines containing them for prevention of coccidiosis**

Antigene Proteine und diese enthaltende Impfstoffe zur Verhütung von Kokzidiose

Protéines antigènes et vaccins les contenant pour la prévention de la coccidiose

(84) Designated Contracting States:
**AT BE CH DE ES FR GB GR IT LI LU NL SE**

(30) Priority: **03.12.1985 US 805301**
**06.12.1985 US 805824**
**11.12.1985 US 807497**
**11.12.1985 US 808013**

(43) Date of publication of application:
**23.10.1991 Bulletin 1991/43**

(62) Application number of earlier application in
accordance with Art. 76 EPC: **86202087.2**

(73) Proprietor: **SOLVAY (Société Anonyme)**
**B-1050 Bruxelles (BE)**

(72) Inventors:
• **Newman, Karel Z., Jr.**
  **Clear Lake, IA 50428 (US)**
• **Gore, Thomas C.**
  **Charles City, IA 50616 (US)**
• **Tedesco, John L.**
  **St. Peters, MO 63376 (US)**
• **Petersen, Gary R.**
  **Charles City, IA 50616 (US)**
• **Brothers, Virginia M.**
  **Albany, CA 94706 (US)**
• **Files, James G.**
  **Belmont, CA 94002 (US)**

• **Paul, Leland S.**
  **Island Lake, IL 60042 (US)**
• **Chang, Ray-Jen**
  **Foster City, CA 94404 (US)**
• **Andrews, William H.**
  **Belmont, CA 94002 (US)**
• **Kuhn, Irene**
  **San Francisco, CA 94117 (US)**
• **McCaman, Michael**
  **San Bruno, CA 94066 (US)**
• **Sias, Stacey R.**
  **San Anselmo, CA 94960 (US)**
• **Nordgren, Robert M.**
  **Charles City, IA 50616 (US)**
• **Dragon, Elizabeth A.**
  **Orinda, CA 94563 (US)**

(74) Representative: **Lechien, Monique et al**
Solvay
**Département de la Propriété Industrielle**
**Rue de Ransbeek, 310**
**B-1120 Bruxelles (BE)**

(56) References cited:
**EP-A- 0 164 176          EP-A- 0 167 443**

• JOURNAL OF BIOLOGICAL CHEMISTRY, vol.
  251, no. 2, 25 January 1976, Baltimore, MD (US);
  R.L. STOTISH et al., pp. 302-307
• PARASITOLOGY, vol. 81, 1980, Cambridge (GB);
  W.M. SHIRLEY, pp. 525-535

**Description**

Background of the Invention

Throughout this application, various publications are referenced by Arabic numerals within parentheses. Full citations for these references may be found at the end of the specification immediately preceding the claims. The disclosures of these publications in their entireties are hereby incorporated by reference into this application in order to more fully describe the state of the art as known to those skilled therein as of the date of the invention described and claimed herein.

The phylum Apicomplexa includes hundreds of different organisms belonging to the order Eucoccidiorida. The genus Eimeria is included within the order of true coccidian agents. Of the organisms belonging to this genus, several species are of recognized importance to the chicken industry. These species include Eimeria tenella, E. maxima, E. acervulina, E. necatrix, E. brunetti, E. mivati, E. mitis and E. praecox.

Differentiation of species is based on the site of infection within the host and oocyst morphology. To date, biochemical markers have not been used for speciation, although differences have been noted for each of the above species.

For avian Eimeria, the entire life cycle is completed within a single host. The life cycle is complex, consisting of asexual and sexual stages, depending upon the Eimeria species involved. The infective stage is the sporulated oocyst. Upon being ingested in contaminated feces, food or water, sporulated oocysts excyst within the digestive tract as a result of the combined action of mechanical shearing and enzymatic hydrolysis of the sporocyst cap. The liberated sporozoites traverse epithelial cells within specific regions of the intestine.

Development begins within the Crypt of Lieberkuhn to the level of first generation meronts ; the meront is a transitional stage consisting of rounded organisms with a more pronounced nucleus, plus increased energy generating and protein synthesizing capacity. Development of first-generation merozoites follows due to multiple fission of meronts. The release of first-generation merozoites destroys the host cell, and the parasites migrate to infect new host cells undergoing a second asexual cycle. Meronts develop to the level of second-generation merozoites destroying additional epithelial cells as they are released. Further destruction of host cells follows with the liberation of the third-generation merozoites. The number of merozoite generations vary from one Eimeria species to another.

Sexual development commences with the production of microgametes and macrogametes through the process of gametogenesis. Liberated microgametes fertilize macrogametes to form zygotes. Development of immature oocysts is followed by rupture of the host cell. Oocysts, released into the lumen of the gut, are passed through the feces to the environment and mature (sporulate) in the presence of atmospheric oxygen.

The process of parasite development is self-limiting if the host ingests no additional oocysts. However, this proves to be an unrealistic expectation in crowded poultry houses.

Disease due to Eimeria can result in severe economic losses associated with diminished feed efficiency and pathologic manifestations.

The pathology of coccidiosis due to E. tenella and E. necatrix is in large part related to the rupture of host cells during the release of merozoites, while host cell rupture during the release of E. maxima oocysts contributes significantly to the pathology seen with that species. Bleeding within the gut is related to rupture of small capillaries servicing the epithelium. It may be difficult to control the progress of disease using coccidiostats, once asexual development is established. Secondary infection often complicates the disease caused by Eimeria. Death can occur within 4-7 days in infected birds infected with E. tenella or E. necatrix. However, death rarely occurs as a result of infection by E. maxima.

A consistent property of the coccidia is that the sporozoites initiate the infection process within very specific tissue sites (39, 45, 57). The site specificity of infection is a characteristic commonly used for speciation of Eimeria. For example, the asexual stages of E. necatrix show a propensity for invasion of epithelial cells residing within the mid-intestine, while sexual stages develop primarily in the cecal pouches.

Much of the work on immunity to coccidiosis has been confined to humoral immunity, more specifically to serum antibodies. Studies have shown a lack of correlation between serum antibody and resistance to disease (59). However, most available data support the contention that a local response with involvement of the secretory immune system or cell mediated immunity (CMI), or both, are involved in the protective response.

Interference with recognition, penetration and/or attachment of pathogens to host cells has a demonstrated protective effect as shown with viral, bacterial and protozoan models. Genetic deletion of key host cell receptors or pathogen attachment features can prevent the initial colonization process (16, 54). Alternatively, secretory antibodies can interfere with the colonization process by binding to, and consequently masking requisite receptors (32, 74). More than one immunoglobulin class has been reported to have the capacity of interfering with the initial colonization process of Eimeria tenella (13). However, recent reports indicate that only production of secretory IgA has been correlated with natural protective immunity (12, 59). Porter and Davis (13) and others (59) reported that secretory IgA neutralizes the extracellular stages of the parasite either by significantly limiting penetration or so debilitating those organisms which did penetrate as to prevent subsequent development.

It has been estimated that an amount approaching $0.5-1.0 billion is spent annually by producers worldwide to combat disease, or to curb the devastating effect of coccidiosis in chickens (39, 52). Even with control measures currently in use, poultry losses are substantial with estimates in the multi-million dollar range (77).

Currently, the most widely used means of controlling Eimeria in chickens is through the application of antiprotozoal chemical feed additives. The specific composition varies with the coccidiostat used, and each product affects only certain stages of the coccidian life cycle (39, 51, 58). Disadvantages of using coccidiostats are many, including short-term residual protection in birds, occasional diminished performance, invocation of resistance to the drug in parasites, and to some extent, safety. Products currently remain on the market for only a few years because of the development of drug resistant strains. This adds considerable pressure on the cost of development and continued manufacture of efficacious products (51).

Protection of birds by immunization has met with some success. Investigators have been able to invoke limited protection using preparations of killed organisms (1, 41, 43). A more effective approach for immunization of chickens has been with the use of a live protozoal product--e.g. Coccivac® (15). The product, being a multivalent composition containing low doses of viable oocysts, is administered in drinking water to invoke a mild parasitemia in birds. A drawback of this product has been occasional depressed performance of birds during the first weeks following administration. Variables such as excessive dosing or moisture content of bedding have even led to severe outbreaks of coccidiosis. See also, U.S. Patent No. 3 147 186 (1964) which concerns the use of viable, sporulated oocysts of E. tenella to immunize chickens and U.S. Patent No. 4 301 148 (1981) which concerns the use of sporozoites of E. tenella for the same purpose.

An alternative means of introducing the live vaccine into broiler houses is by way of the feed. This has been considered in a recent British patent (GB 2 008 404A). Prior to mixing with the feed, fully virulent oocysts of E. tenella are encapsulated in a water soluble polysaccharide to protect against desiccation. The oocysts are in sufficient amounts only to induce subclinical infection. Though the immunizing ability was found to be excellent, no development of this method is foreseen due to questionable field acceptability. However, if attenuated strains of all the important coccidia could be developed, the procedure may be more acceptable.

Efforts have indeed been made to develop Eimeria lines of reduced virulence. Some species have been successfully attenuated through chicken embryo passage (19, 37, 40, 66). These strains have diminished ability to cause disease, yet have retained sufficient immunogenicity to invoke immunity. Some problems do, however, remain with the handling of these strains. As examples, the attenuated variants of E. necatrix have a critical passage limit whereby more or less embryo passage can result in loss of immunogenicity or maintenance of the original virulent form. Furthermore, some attenuated organisms revert to the virulent form upon minimal back-passage through chickens (38, 68). Thus, problems associated with maintaining consistent properties in attenuated organisms are apparent.

Attenuation by precocious selection has also been practiced when Eimeria strains cannot be readily passaged through embryonated eggs. In this process, shed oocysts are harvested late in the prepatent period prior to the onset of heavy oocysts shedding (28, 48, 50, 67). Such selection results in cultures having abbreviated life cycles, and a corresponding diminution in virulence properties (28, 48, 50, 67). Though the trait of precocity for E. tenella (29) and E. aceryulina (49) has been demonstrated to be genetically stable, not enough information is known about this method to assess its usefulness as a tool in the poultry industry.

There is little information available about the surface antigen composition of avian coccidia. Hybridoma cell lines which secrete monoclonal antibodies directed to antigens on the surface of sporozoites of Eimeria tenella have been reported (82). The antigens were not identified, other than that their molecular weights were between 13 and 150 kilodaltons. Additionally, no biological significance or described efficacy in a vaccine was attributed to the antigens. European Patent Publication No. 135 712 also discloses monoclonal antibodies which react with sporozoites of E. tenella. E. tenella sporozoite antigens are disclosed by this publication. Furthermore, European Patent Publication No. 135 073 discloses monoclonal antibodies which react specifically against merozoites and sporozoites of E. tenella. Merozoite antigens derived from E. tenella are described.

Previous work in the laboratory of M.H. Wisher suggests the presence of approximately 16 polypeptides identified by surface iodination of excysted sporozoites of E. tenella and having molecular weights form 20,000 to greater than 200,000 (81). Additionally, European Patent Publication No. 167 443 discloses extracts from sporozoites or sporulated oocysts of E. tenella which may be used as vaccines to protect against coccidiosis. These extracts contain a plurality of polypeptides, one or more of which may be used as an antigen to protect against coccidiosis. Moreover, International Publication No. WO/00528 discloses a cloned gene or fragment thereof from E. tenella which encodes antigenic proteins. These proteins bind with a monoclonal or polyvalent antibody directed against an antigenic protein of avian coccidia.

Subunit approaches to vaccine development have proven successful over the past few years. In such approaches, candidate protective antigens are identified and characterized for the purpose of eventual preparation on a large scale. In studying parasite antigens, one research group used monoclonal antibodies to identify a potential protective antigen on the surface of Babesia bovis (83). A B. bovis antigen of 44,000 daltons has been identified, which when purified and injected into experimental animals afforded some level of protection against primary challenge. An immunologically important 30,000 dalton protein of Toxoplasma gondii has also been identified using monoclonal antibodies (31).

Since mid-1981, Danforth and coworkers have published several papers in which they indicate the possibility of producing monoclonal antibodies toward antigens of avian Eimeria species (9, 10, 11). Similarly, Speer, et al. (69, 70) have demonstrated the development of hybridomas against E. tenella and some physiologic properties thereof. Antibody-secreting hybridomas have been selected on the basis of an indirect fluorescent antibody test (10). The patterns of reaction, as observed with ultraviolet microscopy, have varied depending upon the monoclonal antibody used. Patterns have included exclusive reaction with sporozoites only vs reaction with sporozoites and merozoites ; staining of the anterior portion of the sporozoite vs the entire membrane ; and staining of distinct internal organelles vs non-descript internal staining (11).

Although the preparation of murine-origin hydridomas producing monoclonal antibodies is commonly practiced by those familiar with the art, there is nothing to suggest that the direct and specific selection of sporozoite-neutralizing hybridomas against the species E. tenella and E. necatrix or merozoite-neutralizing hybridomas against the species E. maxima will subsequently identify virulence determinants of these species which may be useful in the development of a subunit vaccine.

This invention concerns the identification, characterization, preparation and use of polypeptide antigens for development of immunity to coccidiosis caused by E. tenella and E. necatrix. Recombinant polypeptide antigens, including fusion proteins, are also described.

The antigens are capable of being precisely dispensed in terms of direct antigenic content and cannot cause disease thus avoiding vaccine strain-related outbreaks and reversions or changes in immunologic properties.

Due to the large economic losses caused by coccidiosis in chickens, vaccines against E. tenella and/or E. necatrix are desirable. Using hybridoma technology, applicants have identified and purified potential protective antigens for use in subunit vaccines. Use of such a subunit vaccine avoids vaccine strain-related outbreaks and reversions or changes in immunological properties associated with the use of a live vaccine.

The quantity of parasite antigens that can be prepared from the organism is quite low and very costly. Recombinant DNA cloning and expression techniques have opened up a new approach to producing large amounts of protective antigens inexpensively. In simplest terms, these techniques require that DNA sequences encoding all or part of the antigen be placed in a cell, under the control of the genetic information necessary to produce the antigenic protein in that cell. The genetic information may be synthetic DNA (17), genomic (e.g., viral) or chromosomal DNA, or cDNA made from the mRNA encoding the antigen. The latter approach is the most direct method for complex organisms such as Eimeria sp.

However, because the cDNA only contains genetic information corresponding to the amino acid sequence of the antigen, it must be inserted into expression vectors that provide the genetic signals necessary for expression of the cDNA gene (i.e., transcription and translation). The antigens can be synthesized either alone or as products fused to another protein in E. coli.

Production of an effective subunit vaccine in E. coli has been reported for foot and mouth disease virus of swine and cattle (33, 66). Foot and mouth disease virus surface antigens were produced as fusion protein antigens in E. coli. Significant levels of virus-neutralizing antibody were raised when cattle and swine were immunized with these antigens. The recombinant DNA-derived antigens gave protection against challenge with foot and mouth disease virus.

In contrast to simple organisms such as foot and mouth disease virus where the genome and surface proteins have been studied extensively, very little is known about the molecular biology of Eimeria. Wang and Stotish (79, 80) reported rapid but transient RNA and protein synthesis in E. tenella during the first 6-8 hours after initiation of sporulation and suggested that all protein and nucleic acid synthesis during sporulation occurs in these first few hours. For example, Stotish et al. (72) reported a 30,000 dalton glycoprotein protein component of sporozoite membranes that was synthesized by unsporulated oocysts and later incorporated into sporozoite membranes during the process of sporulation. Recently, Stotish et al. (73) reported isolation and in vitro translation of RNA from unsporulated oocysts, oocysts during sporulation and from sporozoites. The in vitro translation products ranged from less than 10,000 daltons to greater than 200,000 daltons. Patterns for unsporulated and sporulating oocyst RNA directed-protein synthesis were different, suggesting that different RNA populations may exist during sporulation.

In order to produce cDNA encoding the antigenic proteins, it was necessary to determine when the mRNA encoding the antigenic proteins occurred during the life cycle of E. necatrix. This invention concerns the isolation and characterization of cDNA clones encoding antigenic proteins and the production of engineered antigenic proteins in E. coli. It also concerns the extraction of these proteins produced in E. coli from the insoluble state and the process to make the proteins immunoreactive with monoclonal antibodies. Finally, this invention shows the preparation and use of the bacterially produced antigenic proteins to produce immunity in chickens to coccidiosis cause by E. tenella and/or E. necatrix.

Summary of the invention

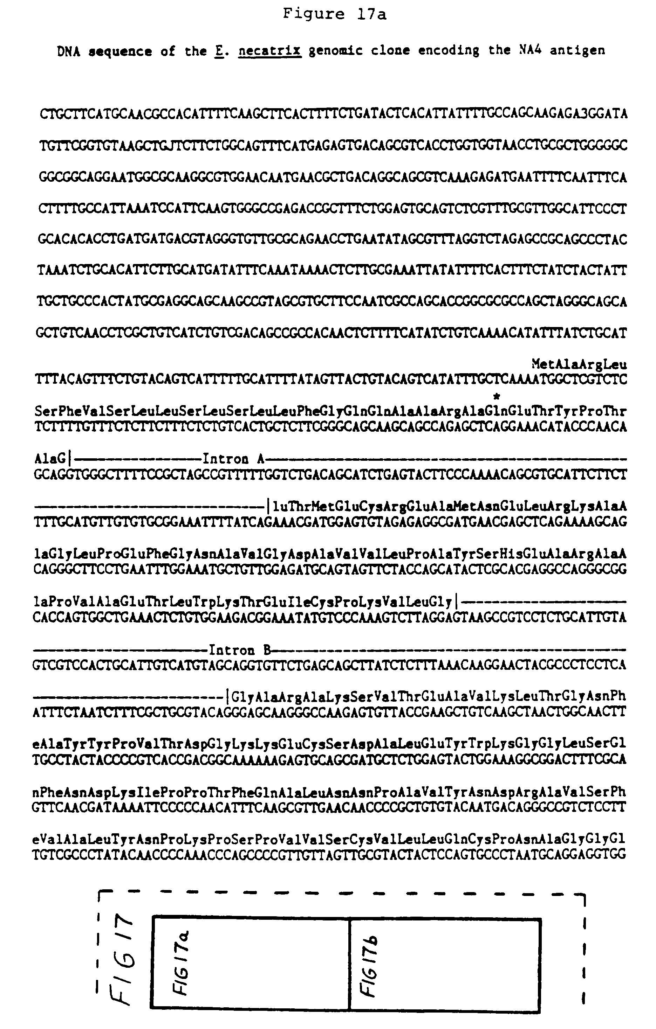

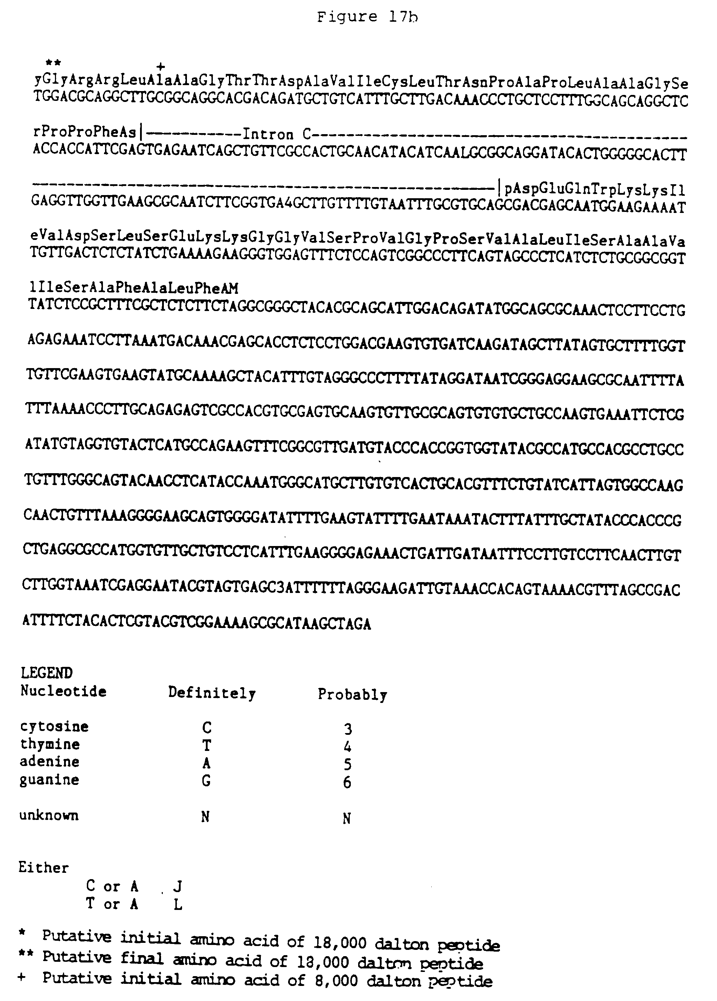

A purified antigenic protein, also referred to herein as the NA4 protein or NA4 antigen, has been isolated from Eimeria necatrix. It is capable of inducing in a chicken an immune response conferring protection against infection by Eimeria necatrix or Eimeria tenella. A homologous antigen found on E. tenella is referred to as the TA4 antigen. The NA4 protein has a molecular weight of about 26,000 daltons and is composed of two polypeptides joined by a disulfide bond. One of the polypeptides is characterized by a molecular weight of about 18,000 and by a blocked N-terminal amino acid ; the other polypeptide is characterized by a molecular weight of about 8,000. The amino acid sequences of both polypeptides are as set forth in Figure 17.

The purified antigenic protein may be prepared by separately recovering it from the sporocyst of E. necatrix. Thus, antigen recovery may be effected by immunoaffinity chromatography or immunoprecipitation utilizing the highly specific monoclonal antibody Ptn 7.2A4/4 produced by hybridoma cell line ATCC No. HB8561. Alternatively, the protein may be prepared by introducing DNA encoding the protein into a suitable host in which the DNA is expressed and from which the protein may be recovered.

Active immunity against infection by E. necatrix and E. tenella may be conferred upon a chicken by administering to the chicken an effective-immunizing amount of the antigenic protein. Preferably, the protein or a fragment thereof is incorporated into a vaccine with a suitable carrier and suitable doses of the vaccine administered to the non-immune chicken.

Brief Description of the Figures

Figure 1 displays the amino acid sequence of the 17,000 dalton polypeptide component of the E. tenella (TA4) antigen determined by microsequencing. Figure 1 also shows the overlapping peptides produced by various chemical and enzymatic digestions.

Figure 2 shows the restriction enzyme map of the E. tenella genomic clone 108-1 encoding the TA4 antigen. Figure 2 also shows the position and orientation of the gene for the TA4 antigen within the 5500 bp E. tenella EcoRI DNA fragment.

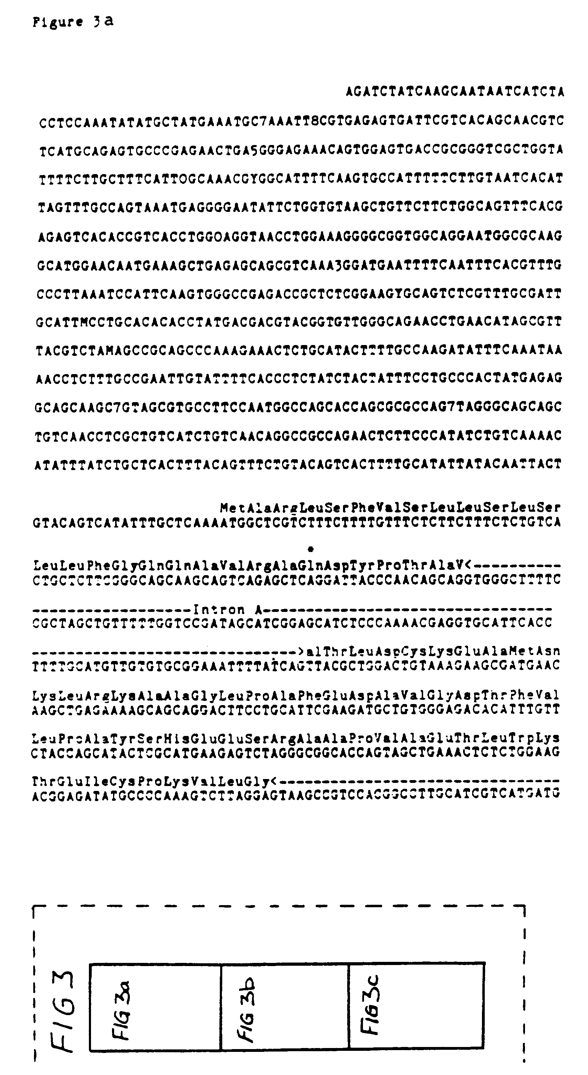

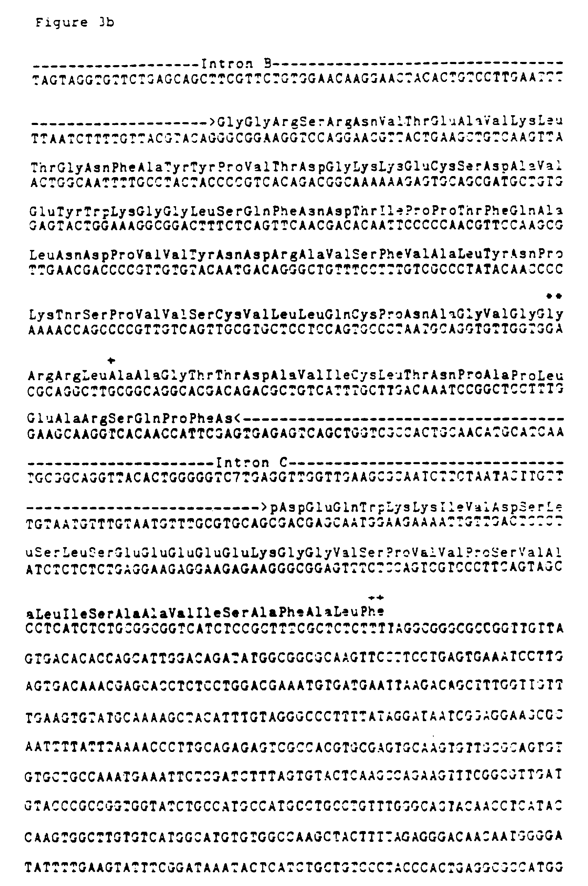

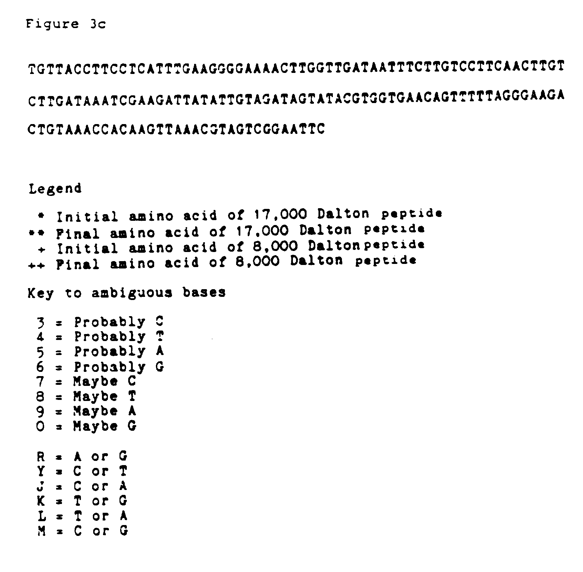

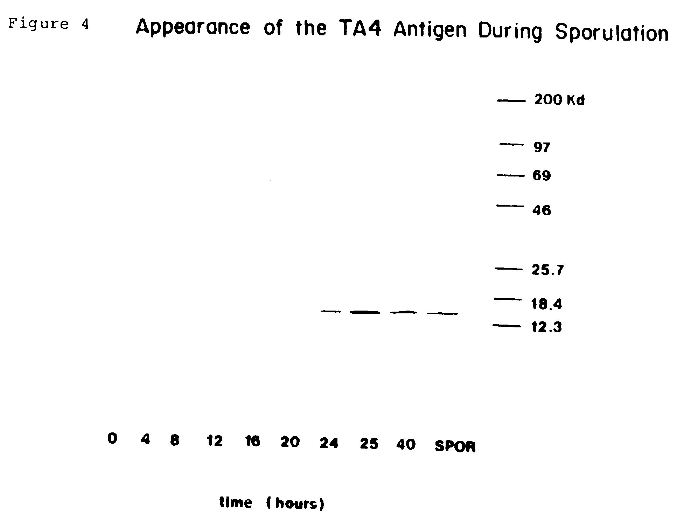

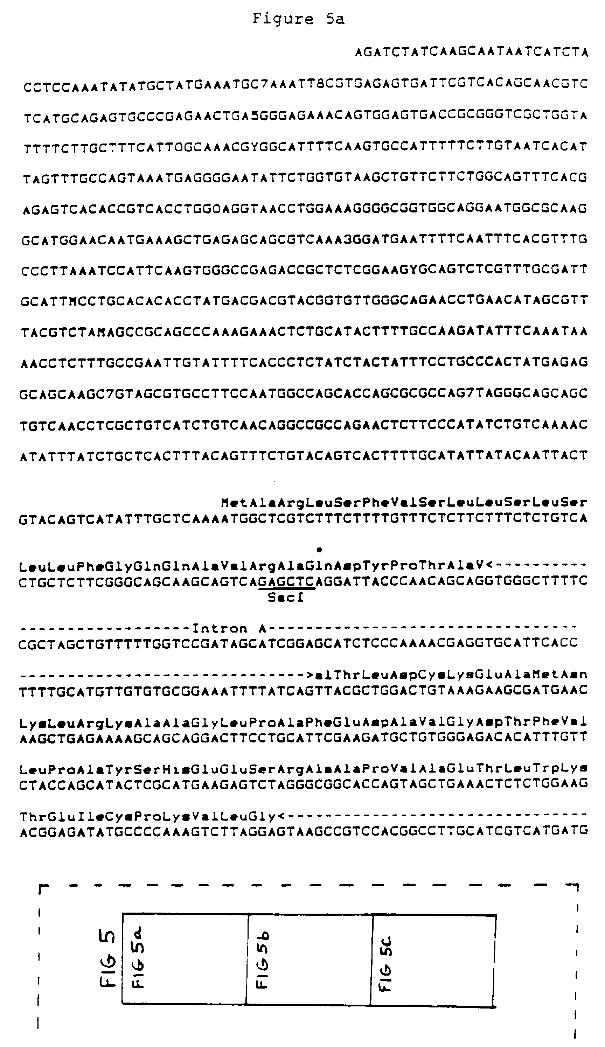

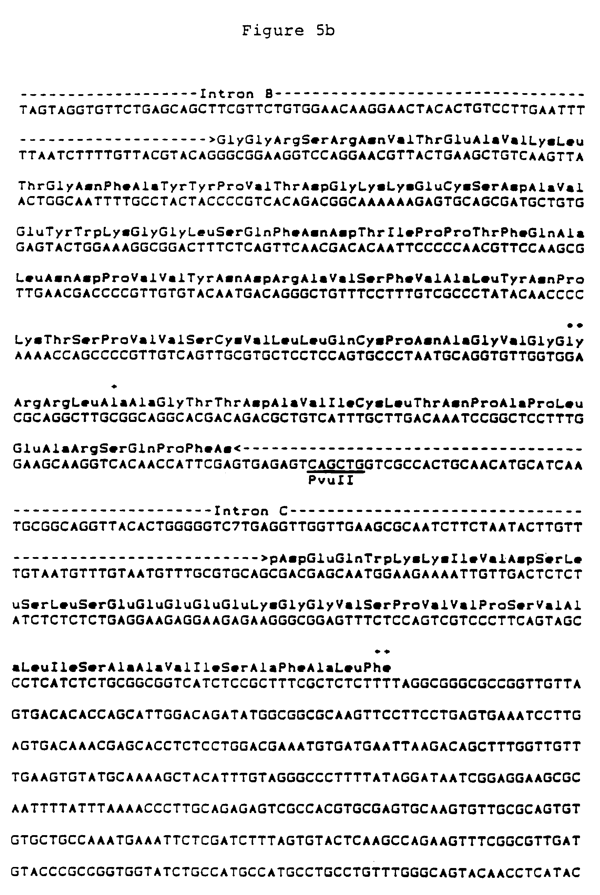

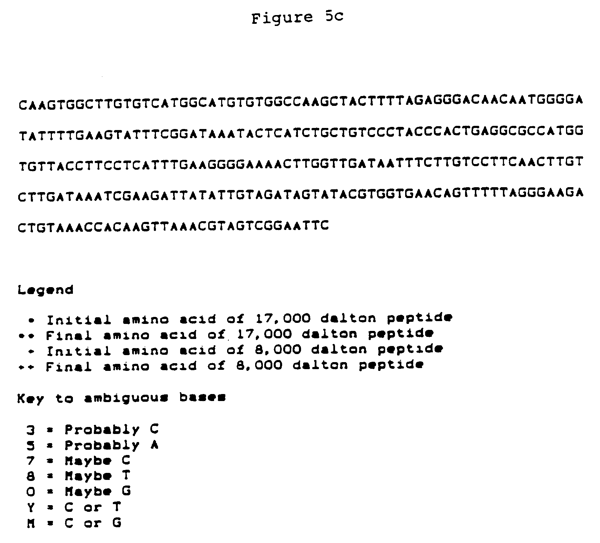

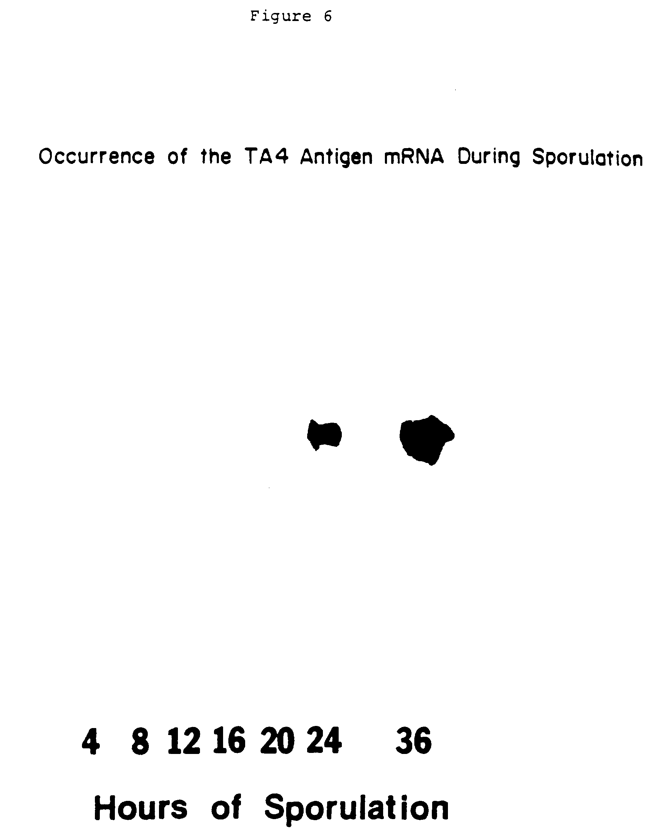

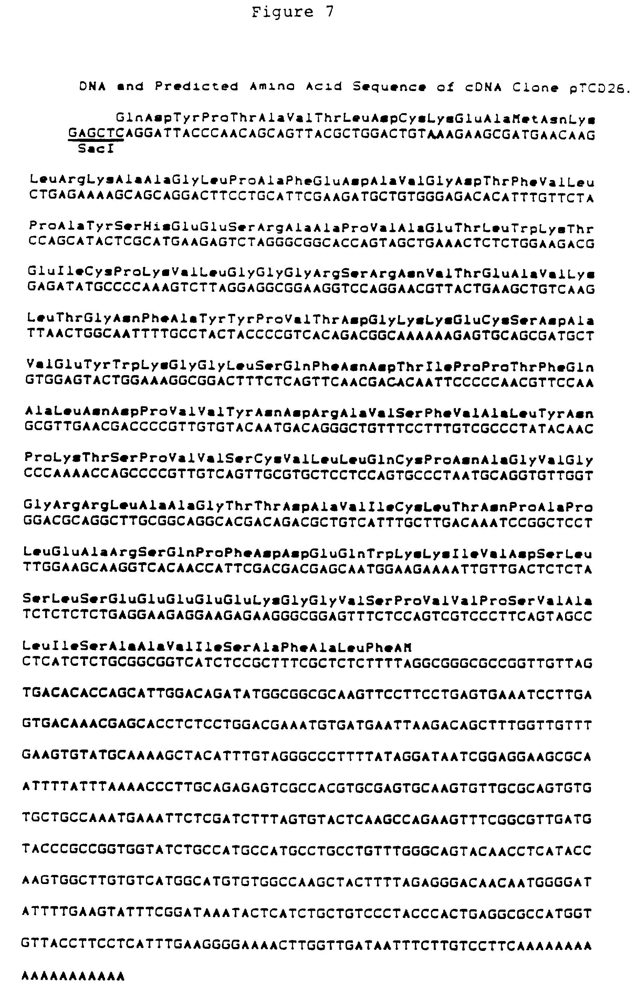

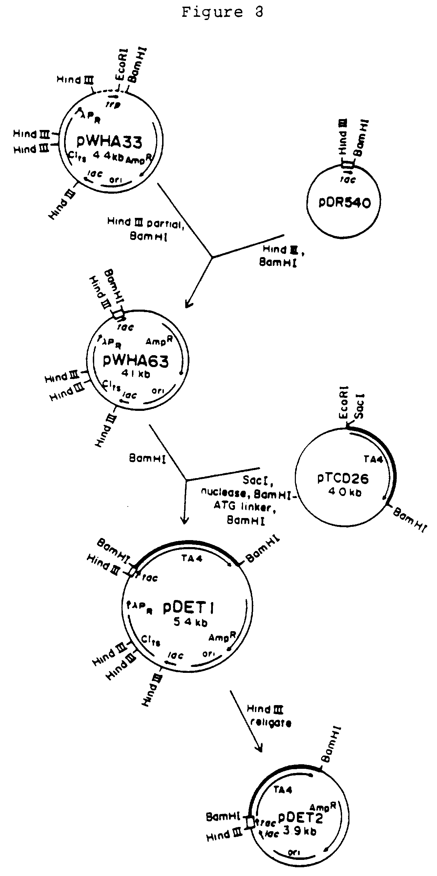

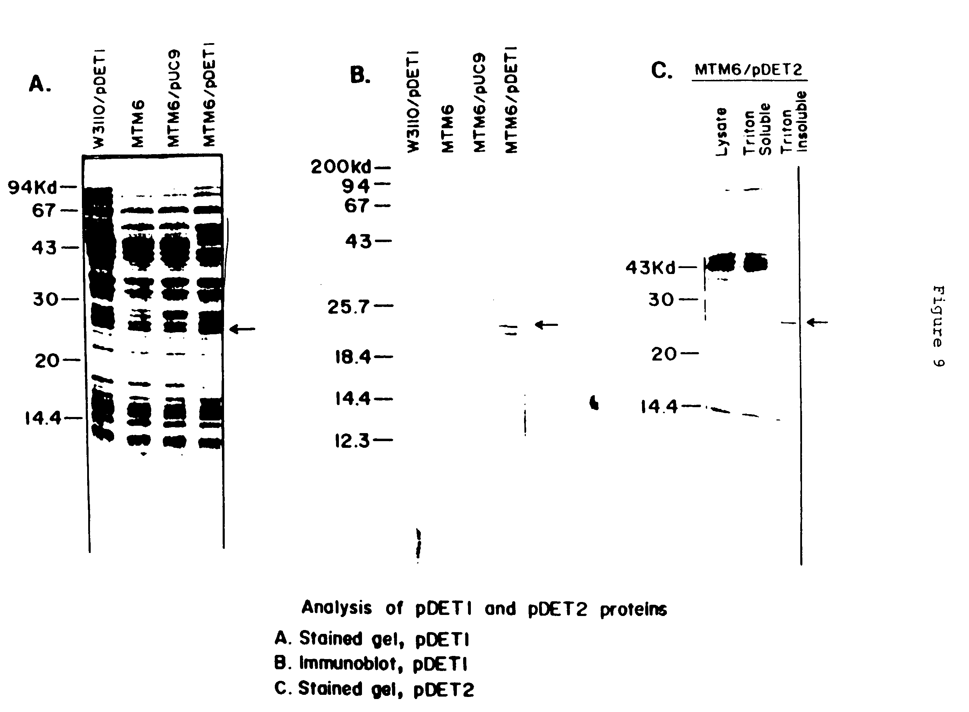

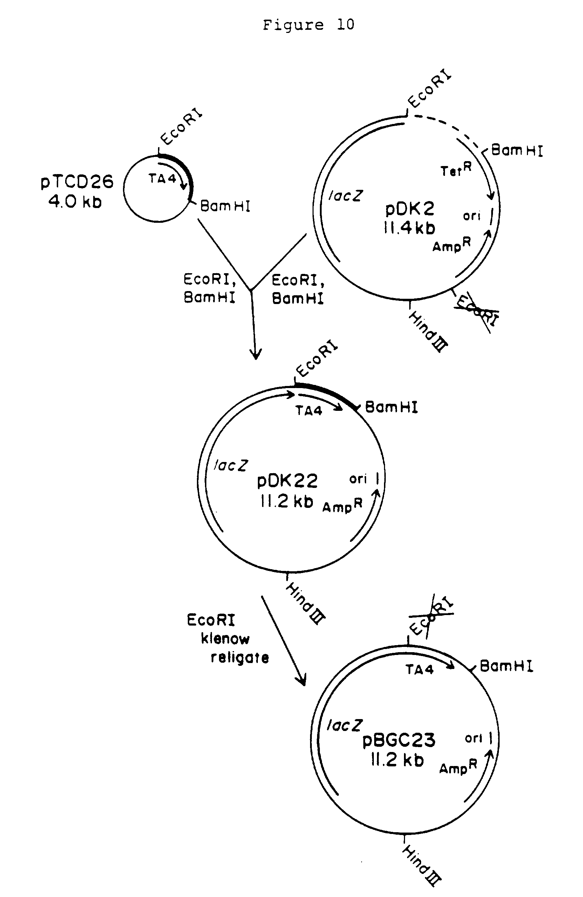

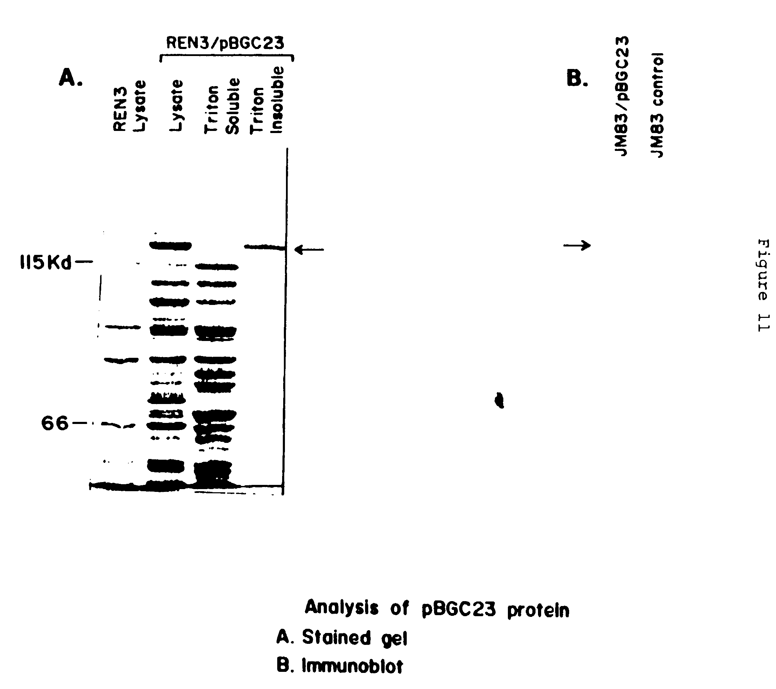

Figure 3 shows the DNA nucleotide sequence of the Bgl II-EcoRI DNA fragment of the genomic clone 108-1 depicted in Figure 2. In addition, Figure 3 shows the amino acid sequence for the signal peptide and the 17,000 dalton and the 8,000 dalton polypeptide components of the TA4 antigen. Figure 3 also shows the introns within the gene.

Figure 16 displays the restriction enzyme map of the E. necatrix genomic clone 7-49 encoding the NA4 antigen and the position and orientation of the gene for the NA4 antigen within the 3900 bp E. necatrix EcoRI DNA fragment.

Figure 17 shows the DNA nucleotide sequence of 2440 bases of the genomic clone 7-49 depicted in Figure 16. This sequence includes the entire HindIII-Ball region shown in Figure 16. Also shown is the amino acid sequence inferred for the E. necatrix NA4 antigen.

Figure 18 shows the amino acid sequence homology between TA4 and NA4 antigens.

Figure 19 displays the homology of the three introns within the E. tenella and E. necatrix genes encoding the TA4 and NA4 antigens respectively.

Figure 20 schematically shows the construction of the recombinant vector pSMAC.

Figure 21 schematically shows the construction of the recombinant vector pSS33.

Figure 22 schematically shows the construction of the recombinant vector pNCD.

Figure 23 schematically shows the construction of expression vectors pTDS1(A) and pTDS2(B).

Figure 24 schematically shows the construction of expression vectors pDDS1(A) and pDDS2(B).

Detailed description of the invention

It should be understood that "antigenic polypeptide" as the term is used herein includes preparations prepared under non-reducing conditions as described herein, characterized by the presence within the preparation of a polypeptide having a defined apparent molecular weight on SDS-PAGE under reducing conditions. When present in such preparations, the polypeptide may be bound to another component or components, e.g. to another polypeptide by one or more disulfide bonds or two or more regions within the polypeptide may be bound to one another, e.g. by a disulfide bond. For those preparations characterized by the presence within them of polypeptides with apparent molecular weights of 18,000 or less on SDS-PAGE under reducing conditions the term "fragment" is also used to describe such preparations on the assumption that the preparations include amino acid sequences contained within the intact antigenic protein. In addition the term "fragment" is used to describe amino acid sequences derived from the antigenic protein by proteolytic digestion.

The present invention provides a purified antigenic protein (hereinafter referred to as NA4) capable of inducing in a chicken an immune response conferring protection against infection by Eimeria necatrix or Eimeria tenella, the protein having a molecular weight of about 26,000 daltons and being composed of two polypeptides joined by a disulfide bond, one of the polypeptides being characterized by a molecular weight of about 18,000 daltons and by a blocked N-terminal amino acid and the other of the polypeptides being characterized by a molecular weight of about 8,000 daltons.

Antigenic analogs of the 26,000 dalton antigenic protein or of its 18,000 dalton and 8,000 dalton polypeptide components are also contemplated. The term "analog" as used herein means any polypeptide, which has an amino acid sequence that differs from that depicted for the 26,000 dalton protein and the 18,000 and 8,000 dalton polypeptides in Figure 17 as a result of substitution of one or more amino acids. Such an analog is contemplated as retaining antigenic properties in that it is capable of inducing an immune response conferring protection against E. necatrix or E. tenella.

The NA4 protein is prepared by first contacting sporocysts of Eimeria necatrix with a detergent under suitable non-reducing conditions in the presence of protease inhibitors so as to solubilize the sporocyst membrane proteins. Next, the protein is separately recovered from the solubilized, sporocyst membrane proteins under suitable non-reducing conditions. The recovery may be effected by partially purifying the solubilized sporocyst membrane proteins by chromatography on DEAE-HPLC followed by preparative SDS gel electrophoresis under suitable non-reducing conditions. The recovery may also be effected by immunoprecipitation or immunoaffinity chromatography with monoclonal antibody Ptn 7.2A4/4 (ATCC No. HB8561).

The present invention contemplates an antigenic polypeptide having an amino acid sequence included within the amino acid sequence of the 26,000 dalton NA4 protein, which is capable of inducing in a chicken an immune response conferring protection against infection by Eimeria necatrix and Eimeria tenella. Such polypeptides include all amino acid sequences which contain an antigenic determinant from the NA4 protein and which are capable of inducing an immune response. Analogs of this polypeptide and monoclonal antibodies directed against it are contemplated. Anti-idiotypic antibodies directed against the monoclonal antibodies are also contemplated.

Antigenic polypeptides which include an amino acid sequence present in the NA4 protein may be produced by various methods, e.g., may be chemically or enzymatically synthesized, may be produced by recombinant DNA methods, may be prepared from the NA4 antigen or may be prepared from the sporocyst or sporozoite of E. necatrix.

The antigens of this invention, in addition to an amino acid sequence included within the amino acid sequence of the NA4 protein, may contain one or more other substances such as polysaccharides, e.g. dextran, or other amino acid sequences, i.e. amino acid sequences not included within the amino acid sequence set forth in Figure 17.

Also contemplated is a fused antigenic polypeptide having an amino acid sequence which includes the amino acid sequence of the 26,000 dalton NA4 protein and an additional amino acid sequence, which is capable of inducing in a chicken an immune response conferring protection against infection by Eimeria necatrix and Eimeria tenella. Analogs of this polypeptide and monoclonal antibodies directed against it are contemplated. Anti-idiotypic antibodies directed against the monoclonal antibodies are also contemplated.

The 18,000 dalton polypeptide of the NA4 antigen may be prepared by first contacting sporocysts of Eimeria necatrix with a detergent under suitable conditions in the presence of protease inhibitors so as to solubilize the sporocyst membrane proteins. Then, separately recovering the polypeptide from the solubilized, sporocyst membrane proteins under suitable reducing conditions. This recovery step may involve partial purification of the solubilized sporocyst membrane proteins by chromatography on DEAE-HPLC chromatography followed by preparative SDS gel electrophoresis under suitable reducing conditions.

The present invention contemplates an antigenic polypeptide which has the amino acid sequence of the 18,000 dalton polypeptide of the NA4 antigen which itself is capable of inducing in a chicken an immune response conferring protection against infection by Eimeria necatrix and Eimeria tenella. Also contemplated are antigenic polypeptides which are fragments of the 18,000 dalton polypeptide or those which consist of the 18,000 dalton polypeptide fused to an additional amino acid sequence. Antigenic analogs of each of these fragments as well as anti-idiotypic antibodies developed against each fragment are also contemplated.

Another method of preparing the NA4 protein comprises preparing a DNA molecule coding for the protein, inserting the DNA molecule into an appropriate expression vector, e.g. a vector containing the PL or lac promoter introducing the resulting expression vector into a suitable host, e.g. E. coli, under appropriate conditions permitting expression of the DNA and production of the protein and recovering the protein so produced.

Another method of preparing the 18,000 dalton polypeptide of the NA4 antigen comprises preparing a DNA molecule coding for the polypeptide, inserting the DNA molecule into an appropriate expression vector, introducing the resulting expression vector, e.g. a vector containing the PL or lac promoter into a suitable host, e.g. E. coli, under appropriate conditions permitting expression of the DNA and production of the polypeptide and recovering the polypeptide so produced.

Messenger RNA may be isolated from sporocysts at many points during the sporulation process. These mRNA samples may then be translated using in vitro (44) or in vivo systems. The translation products may then be immunoprecipitated using the monoclonal antibody (Ptn 7.2A4/4). The mRNA preparation encoding the NA4 antigen may then be used to produce double-stranded cDNA (44). This cDNA may then be inserted into an appropriate cloning vector which may then be used to transform E. coli to generate a cDNA library. This cDNA library may then be screened by colony hybridization techniques using isotopically-labelled oligo-nucleotide probes whose construction is based upon amino acid sequence information from the 18,000 dalton polypeptide component of the NA4 antigen. Vector DNA from bacterial colonies containing nucleotide sequences for the 18,000 dalton polypeptide may then be isolated and the inserted coccidial DNA sequenced (62).

The present invention also provides an antigenic polypeptide which has the amino acid sequence of the 8,000 dalton polypeptide component of the NA4 antigen and which is capable of inducing in a chicken an immune response conferring protection against infection by Eimeria necatrix or Eimeria tenella. Also contemplated are antigenic polypeptides which are fragments of the 8,000 dalton polypeptide or those which consist of the 18,000 dalton polypeptide fused to an additional amino acid sequence. Antigenic analogs of each of these fragments as well as monoclonal antibodies directed against each fragment are contemplated. Anti-idiotypic antibodies directed against the monoclonal antibodies are also contemplated.

A method of conferring upon a chicken active immunity against infection by either Eimeria necatrix or Eimeria tenella or a combination of both comprises administering to a chicken an effective immunizing amount of the NA4 protein, the 18,000 dalton polypeptide, the 8,000 dalton polypeptide, fragments thereof, fusion products thereof, or analogs thereof. Each of these different antigens may be used alone or in combination with one or more of the other antigens. Administration of these materials may be used to increase a relatively low level of immunity in a chicken previously exposed to E. tenella or E. necatrix and may be used in booster vaccinations.

The NA4 antigen or any of the antigenic polypeptides of this invention may be administered to chickens by any of a number of well known methods. Desirably, the administration may involve subcutaneous or intramuscular injection at the back of the neck. The amount of antigen comprising an effective immunizing amount may be any amount from about 0.1 microgram to about 1 mg. The amount of antigen is desirably above about 10 micrograms. The preferred amount of antigen is about 500 micrograms per kilogram of body weight. Alternatively, the administration may be oral (e.g., via capsule) or desirably by injection (e.g., subcutaneous, intradermal, or preferably intramuscular injection). If the mode of administration involves injection, any pharmaceutically acceptable carrier may be employed. Suitable carriers include 0.01 to 0.1M, preferably 0.05M, phosphate buffer or 0.8 percent saline.

A vaccine for conferring upon a chicken active immunity against infection by either Eimeria necatrix or Eimeria tenella or a combination of both comprises per dose an effective immunizing amount of the NA4 protein, the 18,000 dalton polypeptide, the 8,000 dalton polypeptide, fragments thereof, fusion products thereof, or analogs thereof and a suitable carrier. Each different antigen may be used alone or in combination with one or more of the other antigens. An effective immunizing amount is typically above about 0.1 microgram/kg of body weight of the chicken. A method of protecting a chicken against infection by either Eimeria necatrix or Eimeria tenella or a combination of both comprises administering to the chicken a suitable dose of this vaccine.

By administering a suitable dose of such a vaccine to a chicken, the chicken is protected against infection by E. necatrix or E. tenella. The amount of antigenic material per dose should be sufficient to induce production of antibodies to the antigenic material in an animal to which the vaccine is administered. To provide a sufficient degree of immunological response as measured by antibody production and protection, the amount of the antigenic material per dose is desirably above about 20.0 micrograms/kg of body weight of the vaccinated animal. Thus, the amount of antigenic material based upon a 50 gram day-old chick would be above about 1.0 microgram. Presently preferred is a vaccine containing 10 micrograms of antigenic material. In general, the antigen will comprise on a weight basis from about 0.002 percent up to about 0.2 percent of the vaccine and the dose volume will be about 0.1 ml.

This invention also provides the nucleic acid molecule which encodes the NA4 protein and which has the nucleic acid sequence set forth in Figure 17. Further provided are cDNA and mRNA molecules which encode at least a portion of NA4. These nucleic acid molecules may be inserted into a cloning vehicle which may then be inserted into a suitable host cell. A suitable host cell would be a eucaryotic cell, such as a yeast cell or a mammalian cell.

Recombinant cloning vectors are provided which comprise at least a portion of the cDNA molecule which encodes the protein NA4 and cloning vector DNA. The cloning vector DNA is characterized by the presence of a first and a second restriction enzyme site. The cDNA is cloned between these sites.

The recombinant cloning vector DNA may be plasmid DNA. Furthermore, the host cell may be a bacterial cell.

A recombinant cloning vector which comprises plasmid DNA and approximately 90 % of the cDNA which encodes NA4 has been constructed and designated pSMAC. An E. coli host cell which has been transformed with this plasmid has been designated JM83/pSMAC and has been deposited with the ATCC under Accession No. 67241. Another recombinant cloning vehicle, which also includes approximately 90 % of the cDNA encoding NA4 has been constructed and designated pSS33. An E. coli host cell transformed with this vector, designated JM83/pSS33, has been deposited with the ATCC under Accession No. 67242. A further recombinant cloning vehicle which includes approximately 100 % of the cDNA encoding the protein NA4 has been constructed and designated pNCD. This plasmid has been used to transform an E. coli host cell, which has been designated JM83/pNCD and deposited with the ATCC under Accession No. 67266.

Recombinant expression vectors are also provided by the invention. In one embodiment, the recombinant expression vectors are capable of expressing a fused polypeptide comprising at least a portion of the NA4 protein and another amino acid sequence. Such expression vectors have been constructed and designated pTDS1, pTDS2, pDDS1 and pDDS2. These plasmids have been used to transform E. coli cells, designated MH1/pTDS1, MH1/pTDS2 MH1/pDDS1 and JM83/pDDS2 which have been assigned ATCC Accession Nos. 67240, 67264, 67243 and 67265 respectively.

A method of producing the NA4 protein comprises growing host cells having the above-mentioned cloning vehicle inserted under suitable conditions permitting production of the protein and recovering the protein so produced.

Also contemplated is a protein having essentially the same amino acid sequence as the NA4 protein but differing because of expression in a bacterial or other foreign host.

In a further embodiment a composite structure having spatial features common with those of the principal protective structures of the NA4 antigen is substituted for the previously described antigens. One such composition includes an anti-idiotypic antibody developed against the structures of an antibody to the NA4 protein or to one of the antigenic polypeptide of this invention, e.g. the monoclonal antibody Ptn 7.2A4/4, which structures confer specificity toward the respective antigen determinant. Such anti-idiotype antibodies can in themselves be monoclonal in nature, or can be raised as polyclonal antibodies. In the former example, the antibody Ptn 7.2A4/4 can be recovered from hybridoma cell line ATCC No. HB8561, purified and covalently attached to any suitable carrier protein, e.g. the keyhole limpet hemocyanin (KLH). The purified antibody, preferably the purified antibody-KLH complex, is repeatedly injected, preferably with an adjuvant such as Freund's complete adjuvant, into a suitable mammalian lymphocyte donor with Balb/C strain mice as the preferred donor. Hybridomas are developed from lymphocytes of the immunized mice. The hybridomas are screened for antibodies which compete with the NA4 antigen for reaction with the monoclonal antibody Ptn 7.2A4/4, but recognize neither the NA4 antigen nor murine immunoglobulin other than Ptn 7.2A4/4. Such hybridomas secreting anti-idiotype antibodies toward monoclonal antibody Ptn 7.2A4/4 are further expanded and cloned. Production of anti-idiotype antibody can be performed by cultures of cells in any medium suitable for growth of hybridomas and expression of monoclonal antibodies, or growth of antibody producing hybridomas in host animals, with Balb/C mice as the preferred vehicle.

Anti-idiotype antibodies can also be produced by injection of Ptn 7.2A4/4 into animals. One preferred method is to repeatedly inject 500 micrograms of Ptn 7.2A4/4, purified and formulated in a suitable adjuvant, e.g. complete Freunds Adjuvant, into a suitable animal, e.g. a rabbit. After sufficient injections, blood serum is removed after a suitable period of time from such animals. The anti-idiotypic antibodies are then recovered from the blood serum, e.g. by adsorption against normal mouse serum proteins immobilized on an insoluble support such as Sepharose®. Specificity of the resulting antiserum is confirmed by demonstrating reactivity with monoclonal antibody Ptn 7.2A4/4 but none against normal murine antibody.

The anti-idiotype antibodies prepared as above are further purified to the level of IgG fractions. Purified anti-idiotype antibody protein may be administered by any of a number of well-known methods as described for the antigen proper.

Administering an effective amount of the anti-idiotypic antibody of this invention to a chicken provides a method of conferring upon the chicken active immunity against infection by Eimeria necatrix or Eimeria tenella. A vaccine for this purpose comprises an effective immunizing amount of the anti-idiotypic antibody and a suitable carrier. Thus, administering a suitable dose of such a vaccine to a chicken provides a method for protecting the chicken against infection by Eimeria necatrix or Eimeria tenella.

The amount of anti-idiotype antibody per dose must be sufficient to invoke production of an antibody in an animal to whom the vaccine is administered. For induction of an immunological response as measured by antibody production, the amount of anti-idiotype antibody per dose is above 50 micrograms/kg body weight of the vaccinated birds. Thus, the amount of anti-idiotype antibody administered to a 50 g day-old chick would be 2.5 micrograms. Presently preferred is a vaccine containing 25 micrograms of anti-idiotype antibody. In general, the anti-idiotype antibody will comprise on a weight basis from 0.002 percent to up to 0.2 percent of the vaccine, and the dose volume will be 0.2 cc.

A method for obtaining the DNA having the nucleic acid sequence set forth in Figure 17 comprises isolating total genomic DNA from Eimeria necatrix oocysts ; preparing DNA fragments from the genomic DNA so isolated ; ligating the fragments so prepared into an appropriate cloning vector ; subjecting the DNA of the clones so prepared to hybridization with oligonucleotides containing, or complementary to, nucleic acid sequences present within the nucleic acid sequence set forth in Figure 17 to identify appropriate clones ; and isolating from the appropriate clones DNA which encodes the protein and has the nucleic acid sequence set forth in Figure 17.

The NA4 protein is a purified, sporozoite membrane protein derived from the sporocyst of E. necatrix. This protein is an antigen capable of inducing in a non-immune chicken an immune response conferring protection against infection by the intestinal parasites E. necatrix and E. tenella. Although the protein has been derived from E. necatrix, it is contemplated that the protein may be prepared by other methods, e.g., recombinant DNA technology or total organic synthesis and accordingly the invention is not limited to protein prepared directly from E. necatrix but encompasses the protein per se independent of its method of preparation. The protein so prepared may be identical to the structure purified from parasites, or may exist as discrete fragments of the same. It may also exist as a fusion of NA4 with homologous or heterologous sequences. Additionally, it may be presented as an analogue or as an internal image (idiotype) of the same.

The NA4 protein antigen has a molecular weight of about 26,000 and is composed of two polypeptides joined by a disulfide bond. One of the polypeptides is characterized by a molecular weight of about 18,000 and has a blocked N-terminal amino acid. A CNBr fragment of approximately 16,000 daltons has the following partial amino acid sequence : $NH_2$- ? ? Leu ? Lys Ala Ala Gly Leu Pro Glu Phe Gly Asn Ala Val Gly ? Ala Val Val Leu Pro Ala Tyr Ser. The N-terminal amino acid sequence of the 8,000 dalton polypeptide is : $NH_2$- Ala Ala ? Thr ? Asp Ala Val Ile Cys Leu Thr Asn Pro Ala Pro Leu Ala Ala Gly Ser Pro Pro ? Phe ? Asp Glu ? Trp. The complete amino acid sequence of the NA4 protein inferred from the DNA sequence of the gene encoding the NA4 antigen is shown in Figure 17.

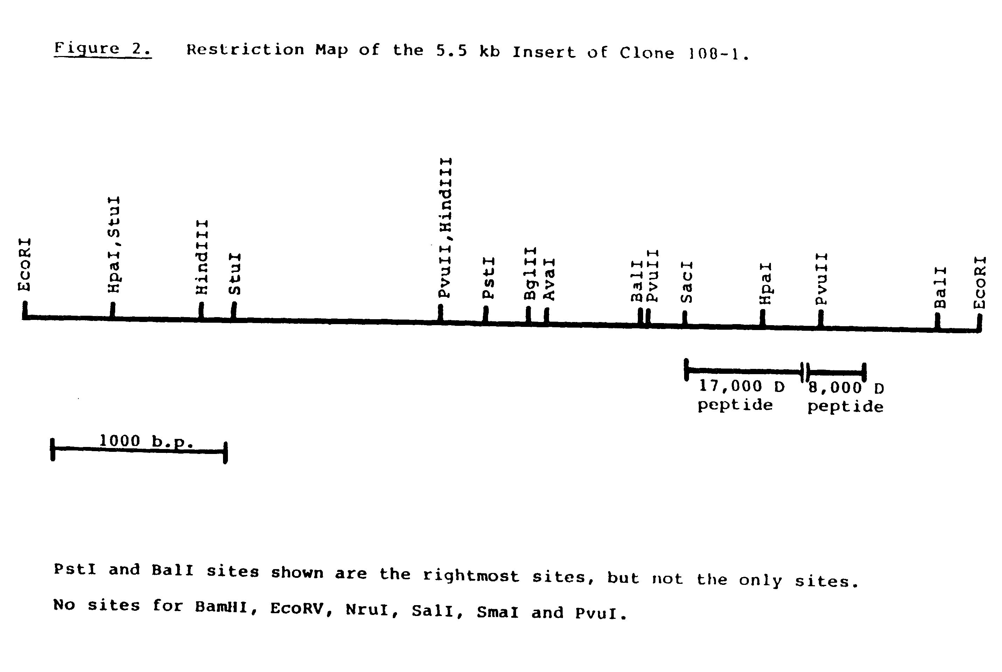

Genomic DNA from E. necatrix has been isolated and cleaved with the restriction endonuclease EcoRI. The restriction fragments were ligated into an appropriate cloning vector, λ-gt wes λ-B, generating a genomic library. The genomic library was then screened by plaque hybridization with a 785bp SacI-PvuII fragment of the E. tenella genomic clone of Figure 2. A genomic clone encoding the NA4 antigen has been isolated and the DNA sequence has shown the two peptides of the NA4 antigen to be encoded by a contiguous nucleotide sequence (Figure 17). Hence the 18,000 and 8,000 dalton peptides are derived from proteolytic processing of a single 26,000 dalton peptide. In addition, the DNA sequence encodes a "signal" sequence typically found at the amino terminus of many secretory or membrane proteins.

The bacterial NA4 proteins can be administered to chickens by any of a number of well known methods. Desirably, the administration can involve subcutaneous intraperitonal or intramuscular injection at the back of the neck, or any convenient form of oral administration. The amount of antigen comprising an effective immunizing amount can be any amount from about 0.1 microgram to about 1 mg. The amount of antigen is desirably above about 10 micrograms. The preferred amount of antigen is about 500 micrograms per kilogram of body weight. Alternatively, the administration can be oral (e.g., via capsule) or desirably by injection (e.g., subcutaneous, intradermal, or preferably intramuscular injections).

If the mode of administration involves injection, any pharmaceutically acceptable carrier can be employed. Suitable carriers include 0.01 to 0.1M, preferably 0.05M phosphate buffer or 0.8 percent saline.

A vaccine for conferring upon a chicken active immunity against infection by Eimeria is provided which comprises an effective immunizing amount of an antigenic material of this invention, i.e., the renatured bacterial NA4 proteins and a suitable carrier. Preferably the effective immunizing amount of the antigenic material in the vaccine is above about 0.1 microgram/kg of body weight of the chicken.

In addition, the carrier desirably also contains a preservative. One particularly suitable preservative is thimerosal (sodium ethylmercurithiosalicylate) which has activity as both a bacteriostat and a fungistat. Desirably, thimerosal is present in the vaccine in a final concentration of $10^{-4}$ percent.

Furthermore, the carrier desirably also contains an immunopotentiator, i.e., a substance which enhances the immune response of the treated animal to the vaccine, including Salmonella minnesota LPS at 10 micrograms/dose. Various immunopotentiators known in the art may be used. The adjuvant presently employed is 94 % Drakeol 6-VR, 5 % Arlacel A, 1 % Tween-80. Arlacel A is a mannide monoleate (Sandria Corp.). It is an irritant which has strong immunopotentiating activity when combined with antigens. Drakeol 6-VR is a hypoallergenic light mineral oil product (Penreco Corp.). Tween-80 is a monoleate derivative of polyoxyethylsorbitan and possesses detergent properties. Other suitable carriers or immunopotentiators include aluminum potassium sulfate, aluminum hydroxide, ligand finding subunits of toxin molecules, bioadhesives, lymphokines and water in oil emulsions.

By administering a suitable dose of such a vaccine to a chicken, the chicken is protected against infection by E. tenella and/or necatrix . The amount of antigenic material per dose should be sufficient to induce production of antibodies to the antigenic material in an animal to which the vaccine is administered. To provide a sufficient degree of immunological response as measured by antibody production and protection, the amount of the antigenic material per dose is desirably above about 20.0 micrograms/kg of body weight of the vaccinated animal. Thus, the amount of antigenic material based upon a 50 gram day-old chick would be above about 1.0 microgram. Presently preferred is a vaccine containing 10 micrograms of antigenic material. In general, the antigen will comprise on a weight basis from about 0.002 percent up to about 0.2 percent of the vaccine and the dose volume will be about 0.1 ml.

EXAMPLE 1

PREPARATION OF EIMERIA NECATRIX AND EIMERIA TENELLA OOCYSTS, SPOROCYSTS AND SPOROZOITES

Coccidia. The purified field isolates of Eimeria necatrix and Eimeria tenella were originally purchased from Dr. Allen Edgar of the University of Auburn. The purity of each isolate was confirmed using oocysts characteristics and histology of infected intestinal tissue. Oocysts size and shape index were within the range of E. necatrix and E. tenella, respectively.

Lesions were scored by the method of Johnson and Reid (30). The lesions in infected birds were typical of each respective isolate. At 5 days post-infection histological examination revealed larger second generation schizonts in the subepithelium of the mid-intestine (E. necatrix) or the ceca (E. tenella). Mortality was experienced with E. tenella and E. necatrix during severe infections (15,000 and 50,000 oocysts respectively). Single oocyst cloning was periodically done to insure purity of each isolate.

Propagation of Oocysts. Pure cultures of each isolate were routinely passaged in 4- tp 6-week old SPF white Leghorn chickens. To avoid extraneous coccidial infections, chickens were reared from 1 day of age in plexiglass isolation units. Oocysts were harvested on day 7 post-infection from the ceca using a trypsin-digest method described by Shirley (66). Sporulated oocysts were typically stored at 24°C in 2 % w/v $K_2Cr_2O_7$.

Isolation of Sporocysts. Sporulated oocysts, (1 x 10$^8$) which had been partially purified from debris by salt floatation, were washed five times in 0.1M phosphate buffered saline, pH 7.4 (PBS) to remove the potassium dichromate preservative. These oocysts were further cleaned by agitation in a 1.05 % sodium hypochlorite solution for 20 minutes followed by five washes in PBS to remove residual sodium hypochlorite and debris. Following the final wash, the cleaned oocysts were resuspended in 10 ml of PBS. Suspended oocysts were then mechanically broken by shaking with an equal volume of glass beads (1.0-1.05mm). The liberated sporocysts were purified from the oocysts walls and from unbroken oocysts by passage over a glass wool column, centrifuged at 3 000 RPM for ten minutes at 4°C and resuspended in 10 ml of PBS.

Preparation of Sporozoites. Freshly sporulated oocysts were cleaned by salt floatation, repeated washing and treatment with 1.05 % sodium hypochlorite solution. Sporocysts were freed by mechanically breaking oocysts with glass beads (1.0-1.05mm). To excyst sporozoites, sporocysts were incubated with trypsin and taurodeoxycholic acid (0.25 and 0.50 % w/v, respectively) for a period of 1 hour at 41°C. Sporozoites thus obtained were rinsed free of excysting fluid by centrifugation and resuspended in Hank's medium. Fresh Hank's medium was used to dilute sporozoites to the working concentration.

EXAMPLE 2

GENERATION, IDENTIFICATION AND CHARACTERIZATION OF HYBRIDOMAS

Monoclonal Antibody. Monoclonal antibodies were derived from hybridomas developed using the method of Van-Deusen and Whetstone (77). Briefly, Balb/C ByJ mice were repeatedly immunized with 10$^6$-10$^7$ intact E. tenella sporozoites. Three days after a final intravenous injection with intact sporozoites, a randomly selected mouse was sacrificed and splenectomized. The splenocytes were separated from fibrous tissue in the organ, and the washed cells fused with the murine plasmacytoma cell line (SP2/OM).

Microneutralization Assay. The microneutralization assay was performed with primary chick kidney cell cultures for E. tenella or embryonic porcine lung cells for E. necatrix. 1- to 2-week-old chicks were sacrificed and aseptically nephrectomized. The cells were plated into 96-well cultures at a density of approximately 10$^4$/well in Earle's LAH medium supplemented with 5 % heat-inactivated fetal calf serum. Cultures were maintained at 41°C in a 5 % $CO_2$ atmosphere. When cell cultures reached a level of approximately 50 % confluency, 50 microliters of hybridoma test or control sample were added to all wells of the plate. Next, about 3 x 10$^4$ sporozoites suspended in 50 microliters of Earle's culture medium were added to all wells of the plate. Twelve to sixteen hours later, the culture supernatant was replaced with fresh Earle's LAH containing 2 % heat inactivated fetal calf serum. The cultures were terminated at 40-44 hours post-infection. Culture supernatant was emptied from the plates at that time. Subsequently, cells were fixed to the plates by the addition of methanol acidified with 5 % glacial acetic acid. The fixed cultures were stained with 0.1 % toluidine blue before examination. Wells were scored as to the approximate percentage level of inhibition of schizogony ; neutralization of parasites by monoclonal antibodies was scored on the basis of the maximum serum dilution still affording complete inhibition of schizont development.

Indirect Fluorescent Antibody Screening. IFA slides were prepared with sporozoites of E. tenella or E. necatrix (about 1 x 10$^6$/well). Slides were air dried several hours to overnight before 10 microliters of 1 % bovine serum albumin (BSA) was added to each well. Five minutes after adding BSA, 20 microliters of test supernatant was added. Supernatants were incubated at 37°C for 20 minutes, followed by three rinses with 0.15M PBS with 0.0005 % Tween-20 (PBS-Tween). Fluorescein conjugated rabbit anti-mouse antibody (diluted 1:40 in PBS) was added to the samples and allowed to incubate at 37°C for 20 minutes. The conjugate was rinsed off three times with PBS-Tween before adding mounting medium and cover slip.

Results. Of the several thousand hybridomas developed against Eimeria tenella, 24 were found to produce neutralizing antibodies toward the sporozoite stage of the parasite. All of the hybridomas studied produced antibodies that recognized membrane bound antigens, although only the antibody produced by one hybridoma recognized an internal membrane antigen.

In vitro neutralizing potency was compared for several supernatants after the initial cloning of the respective cell lines. Supernatant from certain lines demonstrated the greatest relative propensity for neutralizing sporozoites of E. tenella. When antibody content was assessed for each of the supernatants tested, it was determined that twenty-fold less of one antibody (designated Ptn 7.2A4/4) was required to neutralize sporozoites than the second most effective neutralized antibody. Specifically, the amount of Ptn 7.2A4/4 antibody required to neutralize E. tenella is approximately $3.5 \times 10^5$ molecules/sporozoite.

The hybridoma which produces the monoclonal antibody designated Ptn 7.2A4/4 has been deposited with the American Type Culture Collection in Rockville, Maryland, U.S.A. 20852, and identified by ATCC accession No. HB 8561. This deposit was made pursuant to the provisions of the Budapest Treaty on the International Recognition of the Deposit of Microorganisms For The Purposes Of Patent Procedure (hereinafter "Budapest Treaty").

When the monoclonal antibody Ptn 7.2A4 was evaluated with E. necatrix, it was observed that a fluorescent staining pattern, similar to that with E. tenella had developed. The monoclonal was therefore studied in the in vitro neutralization assay against E. necatrix. Said monoclonal antibody was found to possess neutralizing activity against E. necatrix at levels within a comparable range observed with a like number of E. tenella sporozoites.

## EXAMPLE 3

IDENTIFICATION OF THE ANTIGENS OF E. TENELLA RECOGNIZED BY NEUTRALIZING MONOCLONAL ANTIBODY PTN 7.2A4/4

I Labeling of Eimeria Proteins. A total of $2 \times 10^8$ oocysts from E. tenella were processed for iodination. In each case, sporocysts were purified from salt floated, sodium hypochlorite treated oocysts that were broken with glass beads then passed through a glass wool column. Sporocyst membranes were prepared from onehalf of the sporocysts by mechanical breakage in 1 ml 10 mM sodium phosphate, 0.15M NaCl, pH 7.2 (PBS) with glass beads in the presence of protease inhibitors : 0.1mM Phenylmethylsulfonyl fluoride (PMSF), 0.1mM N-tosyl-L-phenylalanine chloromethyl ketone (TPCK), 1mM N-alpha-p-tosyl-L-lysine chloromethyl ketone (TLCK) and 10 KIU/ml aprotinin. The remaining sporocysts were treated with trypsin and taurodeoxychloric acid (total volume = 1 ml) to excyst sporozoites. Both preparations were pelleted at 45,000 RPM for 45 minutes at 4°C and resuspended in 1 ml of phosphate buffered saline (PBS). Care was taken to remove all trypsin - deoxycholate residue from the sporozoites by washing with PBS and 1 mM PMSF prior to ultra-centrifugation.

The one ml samples were put into glass scintillation vials which had been coated with 40 micrograms of IODOGEN (1,3,4,6-tetrachloro-3-alpha,6-alpha-diphenylglycouril) solid phase iodination reagent (24, 53), dried under nitrogen gas and rinsed with PBS. To each tube, 0.5 mCi of $^{125}I$ was added and the samples allowed to incubate for 20 minutes on ice. Afterward, 100 microliters of KI (1 M) was added to each tube to a final concentration of 100 mM, and the reaction was allowed to proceed for an additional 15 minutes on ice. Sporozoite and sporocyst preparations were then diluted to 7 ml with PBS containing 5 mM KI and pelleted at 45,000 RPM for 45 minutes at 4°C.

Extraction of Sporocyst and Sporozoite Membrane Proteins. $^{125}I$ labeled sporocyst and sporozoite pellets from the above high speed centrifugation were resuspended in 1 ml of protein extraction buffer (20 mM Tris-HCl, pH 7.5 ; 50 mM $MgCl_2$ , 25 mM NaCl, 1 % NP40, 1 mM PMSF, 0.1 mM TPCK, 1 mM TLCK and 10 KIU/ml aprotinin). The suspensions were incubated for 60 minutes on ice with occasional vortexing. Insoluble material was separated from the detergent solubilized protein in a microfuge for 15 minutes at 4°C. The supernatants were stored at -70°C.

TCA Precipitation of $^{125}I$ Proteins. Ten microliters of each sample were diluted into 90 microliters of 5 mM KI. Ten microliters of each diluted sample was then added to a solution containing 1 ml of 5 % trichloroacetic acid (TCA), 25 microliters BSA (10 mg/ml) and 5 mM KI and incubated on ice for 30 minutes. The precipitated samples were collected by filtration through glass fiber filters, washed twice with 5 ml of 5 % TCA, 5 mM KI and three times with 5 ml of 95 % ethanol, both at 0°C, and counted in a liquid scintillation counter.

Immunoprecipitation With Monoclonal Antibodies : Fifty microliters of monoclonal antibody were added to 25 microliters of monoclonal antibody dilution buffer (MAB-DIL) : 50 mM Tris-HCl, pH 8.6 ; 150 mM NaCl ; 0.1 % NP-40 ; 0.1 % BSA, RIA grade ; 1 mM TLCK ; 1 mM PMSF ; 10 KIU/ml aprotinin. Twenty microliters of $^{125}I$ labeled protein was then added and the tube vortexed and incubated overnight at 4°C. Rabbit anti-mouse Ig serum (IgA, IgG, IgM) was diluted 1:2 in MAB-DIL and 10 microliters added to each immunoprecipitation tube and incubated 1 hour at 4°C. Protein A-Sepharose (10 % v/v) was diluted 1:4 in monoclonal antibody wash buffer, (MABW) : 50 mM Tris-HCl, pH 8.3 ; 0.05 % NP-40 ; 0.05 % Triton X-100 ; 150 mM NaCl ; 0.02 % $NaN_3$ ; 5 mM KI and 400 microliters added to each tube. The tubes were incubated for one hour at 4°C with gentle rocking. The immunoprecipitation products were washed twice with cold MABW followed by two room temperature washes with MABW. The pellet was resuspended in 50 microliters of SDS-PAGE sample buffer (35), boiled for 5 minutes and microfuged to remove the protein A-Sepharose. Supernatants were counted and analyzed by SDS-PAGE.

Electrophoretic Transfer of Antigens to Nitrocellulose Paper : Uniodinated sporozoite membrane proteins (detergent solubilized as already described) were separately under either reducing or nonreducing conditions by one dimensional sodium dodecyl sulfate polyacrylamide slab gels and electrophoretically tansferred to nitrocellulose paper (75). Electrophoretic blots were processed according to the method of Sharma et al (64) with the exceptions that sera, monoclonal antibodies and the appropriate conjugates (peroxidase conjugated goat anti-chicken IgG, Kirkegaard and Perry, peroxidase conjugated rabbit anti-mouse IgG (Cappel) were employed for blots of reducing gels, and murine monoclonal antibodies used in conjunction with the Vectastain ABC kit for mouse IgG for nonreducing gels (Vector Labs, Burlington, CA). Blots were developed by reacting them with 4-chloro-1-napthol (Sigma ; 660 micrograms/ml) and $H_2O_2$ (0.17 %) for reduced separation or Vectastain reagents for nonreducing separations.

SDS - Polyacrylamide Gel Electrophoresis (SDS-PAGE) of E. tenella Proteins. Total $^{125}$I labeled sporocyst and sporozoite membrane proteins immunosorbed, and immunoprecipitated proteins were analyzed on, 5-25 % exponential or 8-20 % linear gradients SDS-polyacrylamide gels at 25 mA. The gels were dried and exposed to Kodak XAR-5 X-ray film overnight at -70°C. Gels used for staining purposes were visualized by Coomassie (21) or silver staining using the manufacturer's labelled instructions (Pierce Chemical).

Results of Immunoprecipation of E. tenella Antigen with Ptn 7.2A4/4 Monoclonal Antibody. The surfacelabeled E. tenella sporozoite preparation contains two heavily iodinated proteins with apparent molecular weights of 6,500 and 25,000 as judged on reducing SDS-PAGE. The 6,500 dalton protein is readily and specifically immunoprecipitated with monoclonal antibody Ptn 7.2A4/4. Membranes from sporocysts contain two heavily iodinated proteins with apparent molecular weights of 17,000 and 27,000 although several other minor iodinated proteins of various molecular weights are also present. Upon immunoprecipitation of $^{125}$I labeled sporocyst membrane protein the only antigen precipitated following the reaction with the monoclonal antibody Ptn 7.2A4/4 was the 17,000 dalton protein as determined on reducing SDS-PAGE.

Results of Western Blots of E. tenella Antigens with Ptn 7.2A4/4 Monoclonal Antibody. Under the conditions in which the immunoprecipitated, iodinated polypeptides were analyzed on SDS-PAGE as described above, polypeptides linked by disulfide bonds have been separated. However, reduction of disulfide bonds destroys Ptn 7.2A4/4 reactivity on Western blots in both sporocyst and sporozoite membrane preparations. When iodinated sporocyst and sporozoite membrane preparations were run on SDS-PAGE under non-reducing conditions the major radiolabeled species migrates with an apparent molecular weight of 23-25,000. Furthermore, this apparent 23-25,000 dalton species was reactive with monoclonal antibody Ptn 7.2A4/4 by Western blotting. These results suggest that the 17,000 dalton polypeptide and the 8,000 dalton polypeptide are complexed together to form the TA4 antigen. The fact that this other polypeptide component of the TA4 antigen was not observed in immunoprecipitation experiments of iodinated material can be explained by the observation that this other polypeptide does not contain any tyrosines that could be iodinated (see description of the 8,000 dalton polypeptide component of the TA4 antigen in Examples 5 and 6).

EXAMPLE 4

PURIFICATION, IDENTIFICATION AND CHARACTERIZATION OF THE E. TENELLA TA4 ANTIGEN AND FRAGMENTS CONTAINING FRACTIONS THEREOF

Purification of the 17,000 Dalton Peptide Component of the TA4 Antigen. E. tenella sporulated oocysts were resuspended in 10 ml PBS per $10^9$ oocysts and were broken by shaking with an equal volume of glass beads. Membranes were isolated by centrifugation (100,000 x g, 60 min., 4°C) and the proteins were solubilized in 1 % (v/v) NP-40, 10 mM Tris-HCl (pH 7.5), 25 mM NaCl, 1 mM PMSF, 1 mM TLCK, 0.1 mM TPCK and 10 KIU/ml aprotinin. Insoluble material was pelleted with another 100,000 x g spin (60 min., 4°C). The protein was adsorbed to a DEAE-cellulose column equilibrated with 10 mM Tris-HCl (pH 7.7), 0.05 % NP-40 and then washed with this buffer containing 50 mM NaCl. After elution with buffer containing 200 mM NaCl, the 17,000 dalton polypeptide was concentrated by acetone precipitation and the precipitate resuspended in loading buffer, boiled and subjected to electrophoresis in SDS-polyacrylamide (15 %). Conventional SDS-PAGE sample buffer used in this and other experiments contained 62.5 mM Tris-HCl (pH 6.8), 2 % (w/v) sodium dodecyl sulfate, 10 % (w/v) glycerol and 0.001 % (w/v) bromphenol blue. The buffer also contained 5 % (v/v) beta-mercaptoethanol except in experiments in which non-reducing conditions are specified. The 17,000 dalton polypeptide band was identified by staining (Coomassie blue or KCl). The appropriate gel region was excised, the protein electroeluted and concentrated by acetone precipitation. Note that these procedures are denaturing for proteins and peptides bound to each other by disulfide bonds are separated with this method. The 17,000 dalton polypeptide purified by this method was essentially pure.

Purification and Characterization of the TA4 Antigen. As an alternative to purification by gel electrophoresis the sporocyst membrane proteins from the DEAE-cellulose column were dialyzed against 10 mM Tris-HCl, pH8, 0.05 % NP-40 and applied to a DEAE-HPLC column (BioRad) equilibrated in this buffer. The column was eluted with a NaCl gradient (0-300 mM) in the same buffer. The 17,000 dalton polypeptide (identified by its migration on gel electrophoresis) was found in material eluting at 200 mM NaCl. Fractions containing this protein were applied to a hydroxyapatite column (HPHT-BioRad) equilibrated with 30 mM potassium phosphate, pH 6.5, 0.05 % Zwittergent® 3-12 (Calbiochem-Behring, LaJolla, CA) 0.1 mM dithiothreitol. The column was washed with equilibration buffer and developed with a potassium phosphate gradient (0-300 mM) containing 0.05 % Zwittergent® and 0.1 mM dithiothreitol. The 17,000 dalton polypeptide (identified by gel electrophoresis described above) appeared in material eluting at approximately 90 mM potassium phosphate.

Fractions containing the 17,000 dalton polypeptide purified by this method also contained a second peptide of 8,000 daltons. This peptide appears to be linked by a disulfide bridge to the 17,000 dalton polypeptide. If the fractions containing the 17,000 dalton peptide were immunoprecipitated with monoclonal antibody Ptn 7.2A4/4 and the precipitated proteins analyzed by gel electrophoresis under reducing conditions (as above) both the 17,000 and 8,000 dalton polypeptides appear to be immunoprecipitated. Hence, in sporocyst membrane preparations, the 8,000 dalton and 17,000 dalton polypeptides appear to be linked by a disulfide bond (presumably by a cysteine bridge) because the two peptides did not appear on electrophoresis unless a strong reducing agent was present. Under nonreducing conditions, the Ptn 7.2A4/4 reactive species migrates with an apparent molecular weight of 21-24,000.

Preparation of the 11,500 dalton fragment of the TA4 antigen. E. tenella sporocyst membranes were prepared as described above and resuspended in 10 ml of PBS + 1 % Triton X-100. To this 10 ml membrane suspension was added 10 ml of 80 % phenol containing 0.1 % 8-hydroxyquinoline. The suspension was then vortexed at maximum speed for three minutes and centrifuged for ten minutes at 4,000 RPM. The phenol and the flocculent interface were removed and diluted in five volumes of 100 mM ammonium acetate in methanol and allowed to precipitate at -20°C overnight. Following two washes in acetone, the insoluble proteins were agitated for 8 hours in 0.5 % SDS, and insoluble materials removed by centrifugation at 20,000 RPM for one hour at 4°C. The sample was dialyzed extensively against PBS (pH 7.2) containing AG 501-X8 mixed bed resin (1 gm/500 ml). The 11,500 dalton fragment of the TA4 antigen was then immunoadsorbed from the supernatant using the Ptn 7.2A4/4 monoclonal antibody as follows. This polypeptide was shown to be reactive with the Ptn 7.2A4/4 monoclonal antibody by microtiter plate ELISA.

For microtiter plate ELISA polystyrene 96 well clusters (Immulon II) were sensitized with antigen in 10 mM glycine buffered saline, pH 9.6, incubated overnight at 37°C. The wells were washed with 0.15 M PBS with 0.0005 % Tween-2O, blocked with 3 % BSA in PBS-Tween, rewashed, and incubated with Ptn 7.2A4/4 monoclonal antibody diluted in PBS. The wells were washed as before, and then incubated with peroxidase conjugated rabbit anti-mouse IgG serum diluted in PBS. The wells were washed again and then incubated with substrate (2,2'-azino-di-[3-ethyl-benzthiazoline sulfonate]) in the presence of $H_2O_2$. Color development was determined with a Dynatech MR-580 microtiter plate ELISA reader after 15 minutes. The 11,500 dalton fragment of the TA4 antigen was shown to be reactive with the Ptn 7.2A4/4 monoclonal antibody by microtiter plate ELISA.

## EXAMPLE 5

AMINO ACID SEQUENCE OF THE 17,000 AND 8,000 DALTON PEPTIDE COMPONENTS OF THE E. TENELLA TA4 ANTIGEN

Amino Acid Sequence of the 17,000 Dalton Peptide Component of the TA4 Antigen. Amino acid sequencing of the 17,000 dalton peptide was complicated by the finding that the N-terminal amino acid was blocked (i.e. not accessible to Edman degradation (14)). To circumvent this problem the protein was reduced and alkylated and then digested with various chemicals and enzymes. The resulting peptides were purified by reverse phase HPLC (26). The 17,000 dalton polypeptide or the TA4 antigen was digested with CNBr (CN), V8 protease (V), chymotrypsin (CH) and Endoprotease Arg-C (R).

Before protease digestion the purified 18,000 dalton polypeptide or the TA4 antigen was treated with 30 mM dithiothreitol, 6M guanidine-HCl (pH 8) for 1 hour at room temperature. Solid iodoacetamide was added to a final concentration of 100 mM, the pH was readjusted to 8 and the sample was incubated for 1 hour at room temperature. Following reduction and alkylation, samples were purified from reagents either by P6DG (BioRad, Richmond, CA) spin columns equilibrated in 0.1M MOPS, pH 7.5, 0.1 % SDS or by reverse phase HPLC.

For CNBr digestion, the protein sample was treated with 1 % CNBr in 70 % formic acid for 20 hours at 4°C. The sample was evaporated to dryness in a Savant Speedvac centrifuge and redissolved in 0.1 % trifluoroacetic acid (TFA) or 0.1 % TFA, 20 % acetonitrile ($CH_3CN$). V8 digestion was performed in 0.1 % SDS, 0.1 M MOPS pH 7.5 for 2 hours at room temperature at a ratio of 50 micrograms 17,000 dalton polypeptide : 1 microgram V8. After digestion, the samples were precipitated with 4 volumes of acetone at -20°C overnight. The acetone precipitates were redissolved as described above. Chymotrypsin digestion was performed in 0.05 % Zwittergent® 3-12, 0.1M $NH_4HCO_3$, pH 7.8 for 1 hour at 37°C at a ratio of 50:1, 17 000 dalton peptide:chymotrypsin. Samples were acidified with TFA for peptide purification. Arg-C digestion was performed in 0.05 % Zwittergent® 3-12, 0.1M $NH_4HCO_3$ pH 7.8 for 2 hours at 37°C at a ratio of 15:1, 17,000 dalton peptide : Arg-C. After acetone precipitation overnight at -20°C, the peptides were mainly in the acetone supernatant. The supernatant was evaporated and the samples redissolved as described above. Peptides were purified on a Vydac C4 column (the Separations Groups, Inc., Hesperia, CA) and eluted with a 0-100

% $CH_3CN$ gradient in 0.1 % TFA.

Amino acid sequencing was performed using a gas phase sequencer (Applied Biosystems, Inc., Foster City, CA) according to the procedure of Hunkapiller et al (25). Phenylthiohydantoin (PTH) derivatized amino acids were analyzed by HPLC (8).

The N-terminal amino acid was determined directly by removing the blocking agent. The 17,000 dalton peptide was treated with pyroglutamate aminopeptidase (5:1 protein:PAP) in 0.1M potassium phosphate (pH 8.0), 10 mM EDTA, 5 % glycerol, 5 mM dithiothreitol, 0.05 % Zwittergent™ 3-12 for 1 hour at 37°C. After treatment, the amino acid sequence could be determined directly suggesting that the N-terminal amino acid glutamine is cyclized to form the blocked residue pyrrolidone carboxylic acid.

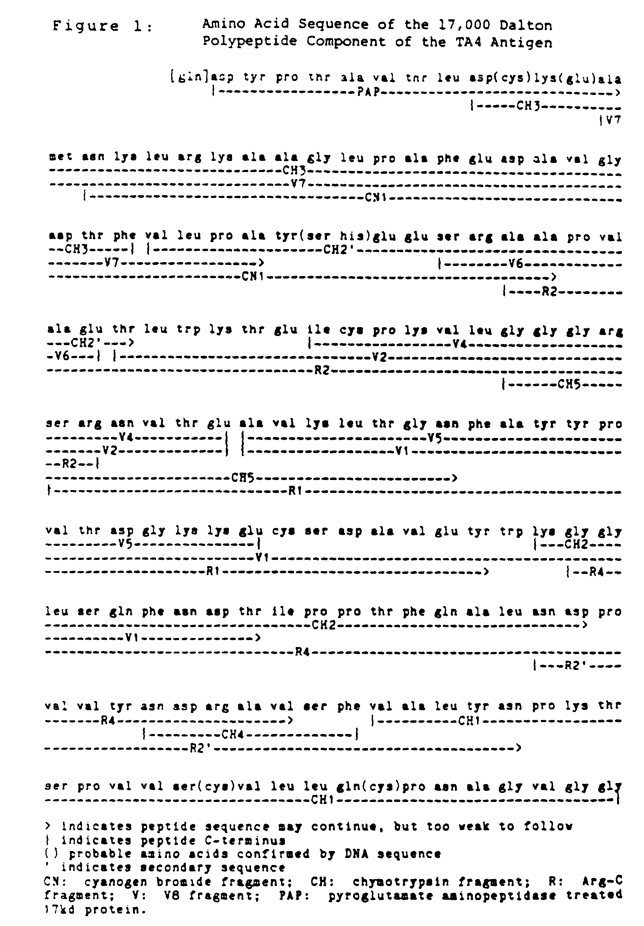

The complete amino acid sequence for the 17,000 dalton peptide component of the TA4 antigen is shown in Figure 1.

Partial Amino Acid Sequence of the 8,000 Dalton Peptide Component of the TA4 Antigen. When the purified 8,000 dalton peptide (derived from the TA4 antigen by reduction and alkylation) was subjected to Edman sequencing the N-terminal amino acid sequence could be determined directly. A partial amino acid sequence of the N-terminal region of the peptide is shown below.

$$NH_2 - \text{ala ala gly thr thr asp ala val ile cys}$$
$$\text{leu thr asn pro ala pro leu glu ala}$$
$$\text{arg ser gln pro phe asp asp glu}$$

EXAMPLE 15

USE OF E. TENELLA TA4 ANTIGEN AND AN 11,500 DALTON FRAGMENT THEREOF TO ELICIT SPOROZOITE NEUTRALIZING SERUM RESPONSE AND PROTECTIVE RESPONSE AGAINST E.TENELLA IN CHICKENS

Eliciting Sporozoite Neutralizing Serum Response Against E. tenella Using the TA4 Antigen. The TA4 antigen used in these experiments was prepared from sporocysts by methods described in Example 4 for the preparation of the nonreduced intact TA4 antigen. Purity and identify of the protein was confirmed by SDS-PAGE and immunoreactivity with monoclonal antibody Ptn 7.214/4 prior to use in chickens.

Vaccine preparations were formulated at a level of one volume antigen to three volumes of oil carrier consisting of about 5 % Arlacel A, 94 % Drakeol 6-VR, 1 % Tween 80 so that each 0.1 ml dose contained approximately 15 micrograms of TA4 antigen. When necessary, antigen was diluted with PBS (pH7.2) to the level desired for formulation. Chickens received 0.1 ml dose by intramuscular route in the neck muscle. Antigen was administered two more times by the same route using the same amount at two-week intervals.

Three days prior to each administration of protein, and eleven days after the final administration, birds were bled for collection of serum samples. Heat inactivated sera were tested independently in the sporozoite microneutralization assay as described in Example 2. Neutralization of parasites by serum was scored on the basis of the maximum serum dilution affording 50 % inhibition of schizont development.

The results as set forth below in Table I indicate that whereas nonvaccinated birds receiving carrier only had no demonstrable neutralizing antiserum titers against E. tenella sporozoites, birds receiving three doses of the antigen had demonstrable neutralizing antiserum titers.

Table I

| TA4 Antigen Induced Sporozoite Neutralization Assay Data Sporozoite Neutralization Titers (ND 50 %)[d] | | | |
|---|---|---|---|
| Serum Samples | Highest | Lowest | Median Titer |
| Prebleed[a] | N.D.[b] | N.D. | N.D. |
| Nonvaccinate Controls (n=9) | N.D. | N.D. | N.D. |
| Carrier Only (n=14) | N.D. | N.D. | N.D. |
| Carrier/Protein | | | |
| Vaccine (n=15) | 1:32 | N.D. | 1:8 |
| Immune serum[c] (Whole Sporozoite Vaccinates | --- | --- | 1:32 |

[a] Serums from birds within each treatment group were pooled and tested

[b] N.D. = No detectable neutralization

[c] Pooled serum from several birds

[d] 50 % neutralization dose

Eliciting a Protective Response in Chickens Using the TA4 Antigen. Sixty-three (63) days after the final vaccination, some birds were challenged, orally with 1,000 sporulated E. tenella oocysts. This was followed the next day with 3,000 sporulated E. tenella oocysts also given orally. Caecal lesions were scored 5 days after the final challenge. The results are tabulated below in Table II.

Table II

| Protection of TA4 Antigen Vaccinated Birds Against E. tenella Coccidiosis | |
|---|---|
| | Lesion Score $\overline{X} \pm$ S.D. |
| Nonvaccinate Controls (n=17) | $3.4 \pm 0.6$ |
| Adjuvant Only (n=5) | $4.0 \pm 0.0$ |
| TA4 Antigen/Adjuvant Vaccinates (n=8) | $2.4 \pm 1.3$ |

Eliciting Sporozoite Neutralizing Serum Response Against E. tenella Using the 11,500 dalton fragment of the TA4 Antigen. The immunogen used in these experiments was prepared from sporocysts by phenol extraction as described in Example 4. Purity and identity of the protein was confirmed by SDS-PAGE and immunoreactivity with monoclonal antibody Ptn 7.2A4/4 prior to use in chickens.

Lyophilized purified antigen was dissolved in 0.15 M phosphate buffered saline and emulsified in three parts carrier consisting of about 5 % Arlacel A, 94 % Drakeol 6-VR, 1 % Tween-20 at a final antigen concentration of 70 micrograms/ml. Chickens received about 14 micrograms protein/0.2 cc dose by intramuscular route in the neck muscle. Antigen was again administered two weeks later by the same route using the same amount.

One day prior to each administration of protein, and two weeks after the second administration of protein, birds were bled for collection of serum samples. Heat inactivated sera were tested independently in the sporozoite microneutralization assay as described in Example 2.

The results as set forth below in Table III indicate that whereas nonvaccinated birds receiving carrier only had no demonstrable neutralizing antiserum titers against E. tenella sporozoites, birds receiving two doses of antigen had demonstrable neutralizing antiserum titers of up to 1:81.

Table III

| Sporozoite Neutralizing Assay Data Sporozoite Neutralization Titers (ND 50 %)a | | | | |
|---|---|---|---|---|
| Serum Samples | Bleeding | Highest | Lowest | Median Titers |
| Prebleed | 0 Week | 1:3 | 1:3 | 1:3 |
| Nonvaccinate | 2 Weeks | 1:3 | 1:3 | 1:3 |
| Controls | 4 Weeks | 1:3 | 1:3 | 1:3 |
| Carriers only | 2 Weeks | 1:3 | 1:3 | 1:3 |
| | 4 Weeks | 1:3 | 1:3 | 1:3 |
| Carrier/Protein | 2 Weeks | 1:3 | 1:3 | 1:3 |
| Vaccine | 4 Weeks | 1:81 | 1:3 | 1:9 |
| Immune Serum** (Whole Sporozoite vaccine) | - | - | - | 1:81 |

\* 5 birds per group
\** Pooled serum from several birds
a A 50 % neutralizing dose

Eliciting a Protective Response in Chickens Using the 11,500 Dalton Fragment of the TA4 Antigen. Birds received approximately 3 micrograms of antigen in the aforementioned carrier one time in the neck muscle. A second group of birds received the carrier substance only. A final group of nonvaccinate (sentinel) birds were housed with each of the two aforementioned groups. Birds were exposed to coccidia by being housed in E. tenella contaminated cages. Approximately two weeks later, the birds were examined and found to have been infected by E. tenella. The results ares shown in Table IV below.

Table IV

| Protection of Vaccinate Birds Against Coccidiosis by E. Tenella | | |
|---|---|---|
| Treatment | Lesion Score $\overline{X} \pm$ S.D. | N° of Deaths |
| Adjuvant only (n=5) | $3.8. \pm 0.4$ | 2 |

Table IV (continued)

| Protection of Vaccinate Birds Against Coccidiosis by E. Tenella | | |
|---|---|---|
| Treatment | Lesion Score $\bar{x}$ ± S.D. | N° of Deaths |
| Antigen vaccination (n=5) | 1.0 ± 0.8 | 0 |
| Sentinal Birds (n=6) | 4.0 ± 0.0 | 6 |

Because the conditions described above closely mimic the natural means of exposure to E. tenella in the field, the data presented show clear evidence of the usefulness of the invention for protection against coccidiosis due to E. tenella.

Demonstration that Neutralizing Serum Antibodies of Chickens Recognize the 17,000 Dalton Polypeptide Component of the TA4 Antigen. Analysis of serum antibody specificity for the 17,000 dalton polypeptide component of the TA4 antigen was performed using Western blots (7,59). All chicken sera with demonstrable neutralization titers to E. tenella sporozoites were shown to possess immunoglobulins with specificity for the 17,000 dalton peptide component of the TA4 antigen; conversely, no sera from nonresponding or control birds had specificity for the 17,000 dalton polypeptide or any other sporozoite protein.

Demonstration that Neutralization Serum Antibodies of Chicken Compete With Monoclonal Antibody Ptn 7.2A4/4. Sera from vaccinated birds with demonstrable neutralization titers to E. tenella sporozoites, as well as corresponding control sera were tested for the ability to compete with antibody Ptn 7.2A4/4 for binding sites on sporozoite membranes. Polystyrene 96 well clusters (Immulon II) were sensitized with 50 microliters of sporozoite membrane proteins in 10 mM glycine buffered saline, pH 9.6, at a level of approximately 100 micrograms total protein/ml. Serial two-fold dilutions of sera were prepared in 0.15 M phosphate buffered saline with 0.0005 % Tween-20 containing a 1:80 dilution of alkaline phosphatase conjugated to Ptn 7.2A4/4 and then transferred to the sensitized plates at a final volume of 75 microliters/well. After incubation at 37°C for 30 minutes, the plates were rinsed free of unreacted materials using PBS-Tw. Afterward, substrate consisting of the sodium salt of P-phosphonitrophenol dissolved in 1M diethanolamine buffer at a level of 1 mg/ml was added to each well of the plate to a final volume of 100 microliters. The resultant reaction product was monitored spectrophotometrically. From the study, it was possible to ascertain that sera from birds responding to the vaccination as evidenced by neutralization and immunoblots also contained antibody which competed with monoclonal antibody Ptn 7.2A4/4. This experiment provides direct evidence that antigen purified from sporozoite membranes by either immunoaffinity chromatography using monoclonal Ptn 7.2A4/4 or conventional chromatography is capable of stimulating an immune response in chickens to the epitope defined by monoclonal Ptn 7.2A4/4.

EXAMPLE 16

USE OF E. TENELLA PROTEIN TO ELICIT SPOROZOITE NEUTRALIZING SERUM RESPONSE AGAINST E. NECATRIX IN CHICKENS

Heat inactivated sera from birds vaccinated with the 11,500 dalton containing preparation of the E. tenella TA4 antigen (Example 4) were pooled and tested in the neutralization assay (Example 2) substituting embryonic porcine lung cells. The results are as listed in Table V below.

Table V

| Treatment | Neutralization Titer |
|---|---|
| Non-immune chicken serum | 1:6 |
| TA4 Antigen Vaccination | 1:24 |
| E. tenella whole sporozoite immune serum | 1:48 |

The data demonstrate the development of an elevated serum neutralization titer against E. necatrix when birds receive the purified 11,500 dalton fragment of the TA4 antigen. Because it has been previously demonstrated that administration of the TA4 antigen results in the elevation of serum neutralizing titers to E. tenella, and that administration of the TA4 antigen results in protection from E. tenella challenge, and since E. necatrix sporozoite neutralization titers are elevated by the administration of TA4 antigen, one skilled in the art would predict that protection against E. necatrix challenge will also result from administration of the TA4 antigen.

EXAMPLE 20

IDENTIFICATION OF THE ANTIGENS OF E. NECATRIX RECOGNIZED BY THE MONOCLONAL ANTIBODY Ptn 7.2A4/4

$^{125}$I Labeling of Eimeria Proteins. A total of 2x10$^8$ oocysts from E. necatrix were processed for iodination. In each case, sporocysts were purified from salt floated, sodium hypochlorite treated oocysts that were broken with glass beads then passed through a glass wool column. Sporocyst membranes were prepared from one-half of the sporocysts by mechanical breakage in 1 ml PBS with glass beads in the presence of protease inhibitors : 0.1 mM Phenylmethylsulfonyl fluoride (PMSF), 0.1 mM N-tosyl-L-phenylalanine chloromethyl ketone (TPCK), 1 mM N-alpha-p-tosyl-L-lysine chloromethyl ketone (TLCK) and 10 KIU/ml aprotinin. The remaining sporocysts were treated with trypsin and taurodeoxycholic acid (total volume = 1 ml) to excyst sporozoites. Both preparations were pelleted at 45,000 RPM for 45 minutes at 4°C and resuspended in 1 ml of phosphate buffered saline (PBS). Care was taken to remove all trypsin - deoxycholate residue from the sporozoites by washing with PBS and 1 mM PMSF prior to ultra-centrifugation.

The one ml samples were put into glass scintillation vials which had been coated with 40 micrograms of IODOGEN® solid phase iodination reagent (24, 53), dried under nitrogen gas and rinsed with PBS. To each tube, 0.5 mCi of $^{125}$I was added and the samples allowed to incubate for 20 minutes on ice. Afterward, 100 microliters of KI (1 M) was added to each tube to a final concentration of 100 mM, and the reaction was allowed to proceed for an additional 15 minutes on ice. Sporozoite and sporocyst preparations were then diluted to 7 ml with PBS containing 5 mM KI and pelleted at 45,000 RPM for 45 minutes at 4°C.

Extraction of Sporocyst and Sporozoite Membrane Proteins. $^{125}$I labeled sporocyst and sporozoite pellets from the above high speed centrifugation were resuspended in 1 ml of protein extraction buffer. The suspensions were incubated for 30 minutes on ice with occasional vortexing. Insoluble material was separated from the detergent solubilized protein in a microfuge for 15 minutes at 4°C. The supernatants were stored at -70°C.

TCA Precipitation of $^{125}$I Proteins. Ten microliters of each sample were diluted into 90 microliters of 5 mM KI. Ten microliters of each diluted sample was then added to a solution containing 1 ml of 5 % trichloroacetic acid (TCA), 25 microliters BSA (10 mg/ml) and 5 mM KI and incubated on ice for 30 minutes. The precipitated samples were collected by filtration through glass fiber filters, washed twice with 5 ml of 5 % TCA, 5 mM KI and three times with 5 ml of 95 % ethanol, both at 0°C, and counted, for 10 minutes in a liquid scintillation counter.

Immunoprecipitation With Monoclonal Antibodies : Fifty microliters of monoclonal antibody were added to 25 microliters of MAB-DIL. Twenty microliters of $^{125}$I labeled protein was then added and the tube vortexed and incubated overnight at 4°C. Rabbit anti-mouse Ig serum (IgA, IgG, IgM) was diluted 1:2 in BAB-DIL and 10 microliters added to each immunoprecipitation tube and incubated 1 hour at 4°C. Protein A-Sepharose (10 % v/v) diluted 1:4 was added and the tubes were incubated for one hour at 4°C with gentle rocking. The immunoprecipitation products were washed twice with cold MABW followed by two room temperature washes with MABW. The pellet was resuspended in 50 microliters of SDS-PAGE sample buffer (35), boiled for 5 minutes and microfuged to remove the protein A-Sepharose. Supernatants were assayed for radioactive counts and analyzed by SDS-PAGE.

SDS-Polyacrylamide Gel Electrophoresis (SDS-PAGE) of E. Necatrix Proteins. Total $^{125}$I labeled sporocyst and sporozoite membrane proteins immunoadsorbed, and immunoprecipitated proteins were analyzed on, 5-25 % exponential or 8-20 % linear gradient SDS-polyacrylamide gels at 25 mA. The gels were dried and exposed to Kodak XAR-5 X-ray film overnight at -70°C. Gels used for staining purposes were visualized by Coomassie (21) or silver staining using the manufacturer's labelled instructions (Pierce Chemical).

Results of Immunoprecipitation of E. Necatrix Antigen with Ptn 7.2A4/4 Monoclonal Antibody. The surface-labeled E. necatrix sporozoite preparation contains two heavily iodinated proteins with apparent molecular weights of about 6,500 and 25,000 as judged on reducing SDS-PAGE. The 6,500 dalton protein is readily and specifically immunoprecipitated with monoclonal antibody Ptn 7.2A4/4. Membranes from sporocysts contain two heavily iodinated proteins with apparent molecular weights of about 18,000 and 26,000 although several other minor iodinated proteins of various molecular weights are also present. Upon immunoprecipitation of $^{125}$I labeled sporocyst membrane protein the only antigen precipitated following the reaction with the monoclonal antibody Ptn 7.2A4/4 was the 18,000 dalton protein as determined on reducing SDS-PAGE.

EXAMPLE 21

PURIFICATION, IDENTIFICATION AND CHARACTERIZATION OF THE E. NECATRIX NA4 ANTIGEN

Purification and Characterization of the NA4 Antigen - E. necatrix sporulated oocysts were resuspended in 10 ml PBS per 10$^9$ oocysts and were broken by shaking with and equal volume of glass beads. Membranes were isolated by centrifugation (100,000 x g, 60 min., 4°C) and the proteins were solubilized in 1 % NP-40, 10 mM Tris-HCL (pH 7.5), 25 mM NaCl, 1 mM PMSF, 1 mM TLCK, 0.1 mM TPCK and 10 KIU/ml aprotinin. Insoluble material was pelleted by centrifugation (100,000 x g spin, 60 min., 4°C). The sporocyst membrane proteins were adsorbed to a DEAE-HPLC column (BioRad) equilibrated in 20 mM Tris-HCl, pH 8.1, 0.05 % Zwittergent® 3-12. The column was eluted with a NaCL gradient (0 - 500 mM) in this buffer containing 0.1 mM dithiothreitol. The NA4 identified by its migration on gel electrophoresis, was found in material eluting at approximately 275 mM NaCl.

Fractions containing the NA4 antigen were pooled and concentrated using a Centricon® 10 microconcentrator (Amicon Corp., Danvers, MA). The concentrate was diluted with approximately 10 volumes of 0.01 % (w/v) SDS and reconcentrated to lower salt and dithiothreitol levels. The sample was diluted in loading buffer-containing 62.5 mM Tris-HCl (pH 6.8), 2 % (w/v) sodium dodecyl sulfate, 10 % (w/v) glycerol and 0.001 % (w/v) bromphenol blue, boiled and subjected to electrophoresis in 15 % SDS-polyacrylamide gels. Under these nonreducing conditions, an approximately 26,000 dalton NA4 antigen was identified by KCl staining (21). The appropriate region of the gel was excised and the protein was eluted by shaking the gel for 4 hours at room temperature in 1 ml of 10 mM $NH_4HCO_3$, 0.02 % (w/v) SDS. The NA4 antigen prepared by this method was essentially pure.

When the NA4 antigen was analysed by SDS-PAGE under reducing conditions (i.e. 5 % (v/v) B-mercaptoethanol in the sample buffer) the NA4 antigen appears to contain two polypeptides of 18,000 and approximately 8,000 daltons. In sporocyst membrane preparations, the 18,000 and 8,000 dalton polypeptides therefore appear to be linked by a disulfide bond.

Purification of the E. Necatrix Antigen By Immunoadsorption Techniques for In Vivo Testing. Immunoadsorption of E. necatrix NA4 antigen was done according to the procedure of Kasper et al with some modifications (31). Briefly, total E. necatrix sporocyst membrane, as described earlier in this example was incubated overnight at 4°C in the presence of Ptn 7.2A4/4 monoclonal antibody. The resultant mixture was then rocked at 4°C in the presence of goat anti-mouse antisera for two hours followed by reaction with protein A Sepharose (Sigma ; St. Louis, MO) under these same conditions. This suspension was poured into a glass column and washed with PBS until base-line adsorbance was achieved in order to remove unbound protein. Nonspecifically bound protein was removed with alternate washes of PBS (pH 8.0) and acetate buffer (0.1 M, pH 4.0). Specifically bound antigen was eluted with 60 mM Tris-HCl, pH 6.8 containing 2 % SDS. This was followed by subsequent passage of antigen over a Sephadex G-200 column equilibrated and eluted with this same buffer. Sodium dodecyl sulfate was removed by passage over an ExtractiGel D® column (Pierce ; Rockford, IL).

EXAMPLE 22

PARTIAL AMINO ACID SEQUENCE OF THE 18,000 AND 8,000 DALTON PEPTIDE COMPONENTS OF THE E. NECATRIX NA4 ANTIGEN

Amino Acid Sequence of the 18,000 dalton peptide component of the NA4 Antigen. Amino acid sequencing of the 18,000 dalton peptide was complicated by the finding that the N-terminal amino acid was blocked (i.e. not accessible to Edman degradation (14). To circumvent this problem, the NA4 antigen was digested with CNBr and an approximately 16,000 dalton CNBr fragment was purified by reverse phase HPLC (26). For CNBr digestion approximately 10 micrograms of protein was dissolved in 2 % CNBr in 70 % formic acid overnight at 4°C. The sample was evaporated to dryness in a Savant Speed-vac centrifuge and redissolved in 0.1 % TFA. The large CNBr fragment was purified on a Vydac C4 column (the Separations Group, Hesperia, CA) and eluted with a 0-100 % $CH_3CN$:isopropanol 2:1 gradient in 0.1 % TFA.

Amino acid sequencing was performed using a gas phase sequencer (Applied Biosystems, Inc., Foster City, CA) according to the procedure of Hunkapiller et al (25). Phenylthiohydantoin (PTH) derivatized amino acids were analyzed by HPLC (8). The partial amino acid sequence for the large CNBr fragment is shown below

NH$_2$ – _?_ _?_ Leu _?_ Lys Ala Ala Gly Leu Pro Glu Phe Gly Asn Ala Val Gly _?_ Ala Val Val Leu Pro Ala Tyr Ser.

.

Partial Amino Acid Sequence of the 8,000 Dalton Peptide Component of the NA4 Antigen. The N-terminal amino acid sequence of the 8,000 dalton peptide component of the NA4 antigen could be determined directly by sequencing the NA4 antigen. The purified NA4 antigen eluted from the SDS-PAGE gel was concentrated approximately 6-fold using a Centricon® 10 microconcentrator. To remove glycine in the sample that was eluted from the SDS gel 20 volumes of water was added twice to the concentrate and the sample was then reconcentrated. The concentrated sample was applied directly to the sequenator. A partial amino acid sequence of the N-terminal region of the peptide is shown below :

```
NH₂ - Ala Ala _?_ Thr Thr? Asp Ala Val Ile Cys Leu Thr Asn
Pro Ala Pro Leu Ala Ala Gly Ser Pro Pro? Phe _?_ Asp Glu _?_ Trp.
```

EXAMPLE 23

ISOLATION AND CHARACTERIZATION OF A GENOMIC DNA CLONE ENCODING THE E. NECATRIX NA4 ANTIGEN

Isolation of DNA from E. Necatrix Sporulated Oocysts. Sporulated oocysts (5 x 10⁸) were washed and DNA from sporocysts was isolated as described in Example 6.

Construction of the E. Necatrix Genomic Library in Bacteriophage λgt wes λB. The E. necatrix genomic DNA library in bacteriophage λgt wes λB (26) was constructed as described in Example 6. 15 micrograms of EcoRI digested DNA arms were ligated to 3 micrograms of EcoRI digested E. necatrix DNA using T4 DNA ligase. 1 microgram of the ligated DNA was packaged in vitro into phage particles producing a library of 2 x 10⁶ recombinant phage particles.

Screening the E. necatrix Genomic DNA Library. Nitrocellulose filter replicates of recombinant phage of the E. necatrix genomic DNA library were screened with the 785 base pair Sac I - Pvu II fragment of the E. tenella genomic clone 108-1-2 which had been nick translated with [³²P]-dATP. Positive plaques that hybridized to the nick-translated probe were picked, plaque purified and DNA was prepared as described previously. Positive phage 7 was grown up on a larger scale for purification and characterization of the E. necatrix DNA insert.

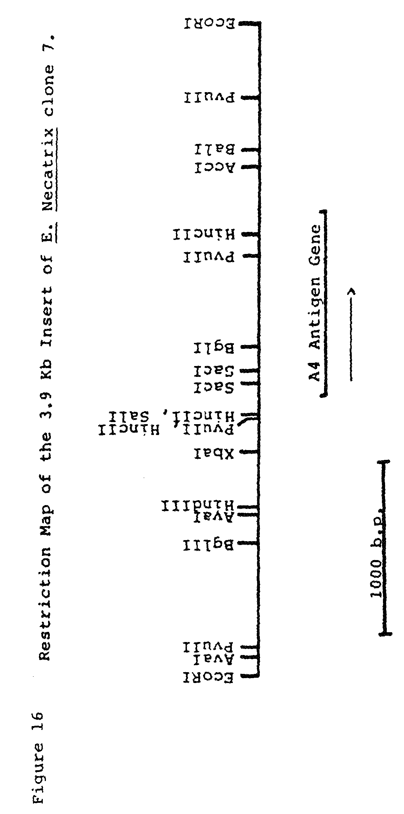

Detailed Characterization of the Genomic Clone Encoding the 18,000 Dalton Peptide - Restriction Map. The 3,900 bp EcoRI fragment insert of clone 7 was subcloned from the phage vector into plasmid pUC9 (78) to produce clone 7-49. This recombinant plasmid was digested with a variety of restriction endonucleases to determine the position of key restriction sites in the genomic DNA clone. The position of restriction sites within the DNA was needed to determine the location and orientation of the 18,000 dalton peptide gene and to develop a strategy to sequence the EcoRI genomic DNA fragment. The restriction map is presented in Figure 16. The location and orientation of the gene for the 18,000 dalton peptide is shown on this map.

DNA Sequence Analysis of Subclone 7-49. The fragment of clone 7-49 containing the gene for the 18,000 dalton peptide component of the E. necatrix NA4 antigen was sequenced by the dideoxy method of Sanger (62) using various restriction enzyme fragments. Primers for DNA synthesis included oligonucleotides COD 92, 94 and 108 as well as other synthetic oligonucleotides. The DNA sequence is shown in Figure 17.

Structure of the Gene Encoding the E. necatrix NA4 Antigen. The DNA sequence agrees with that predicted by the partial amino acid analysis.

Based on polyacrylamide gel electrophoresis under non-reducing conditions both the E. necatrix and E. tenella antigens have an apparent molecular weight of 25-26,000 daltons. Electrophoresis under reducing conditions has shown that both antigens are composed of tow polypeptides linked by a disulfide bond. Comparison of the E. necatrix gene to the E. tenella gene suggests the gene structure is similar to the E. tenella gene in the three features discussed previously, namely : (1) the gene encodes a 23 amino acid signal peptide ; (2) there are three introns within the gene and (3) the gene encodes a 26,000 dalton peptide which has the same proteolytic processing site (Arg-Arg-Leu) to produce 18,000 and 8,000 dalton peptides.

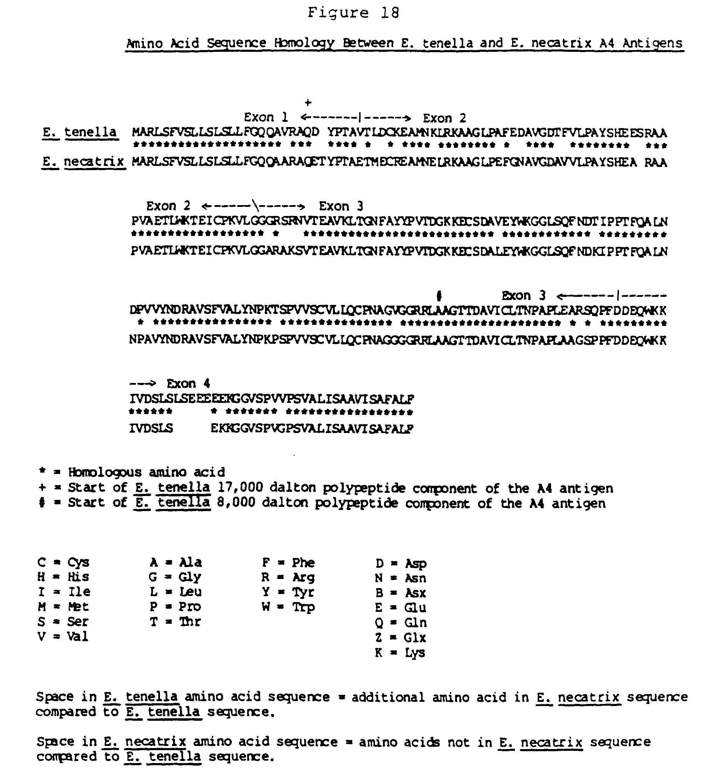

From analysis of the DNA sequence of the E. necatrix gene compared to the E. Tenella gene, similarities and differences between the two proteins can be inferred. Figure 18 shows the alignment of the E. tenella and E. necatrix genes and the predicted amino acid sequences. The 3 intron entrance/exit sites are preserved in both genes. The two A4 antigen proteins show 86 % homology in their amino acid sequences. All cysteine amino acid residues and presumably disulfide bonds are preserved. The E. necatrix protein shows an insertion of one amino acid between positions 2 and 3 of the mature 17,000 dalton polypeptide of the E. tenella protein. In addition, the E. necatrix protein lacks the serine residue that is at position 45 in the E. tenella protein and the amino acids corresponding to positions 223 to 228 in the mature E. tenella protein.

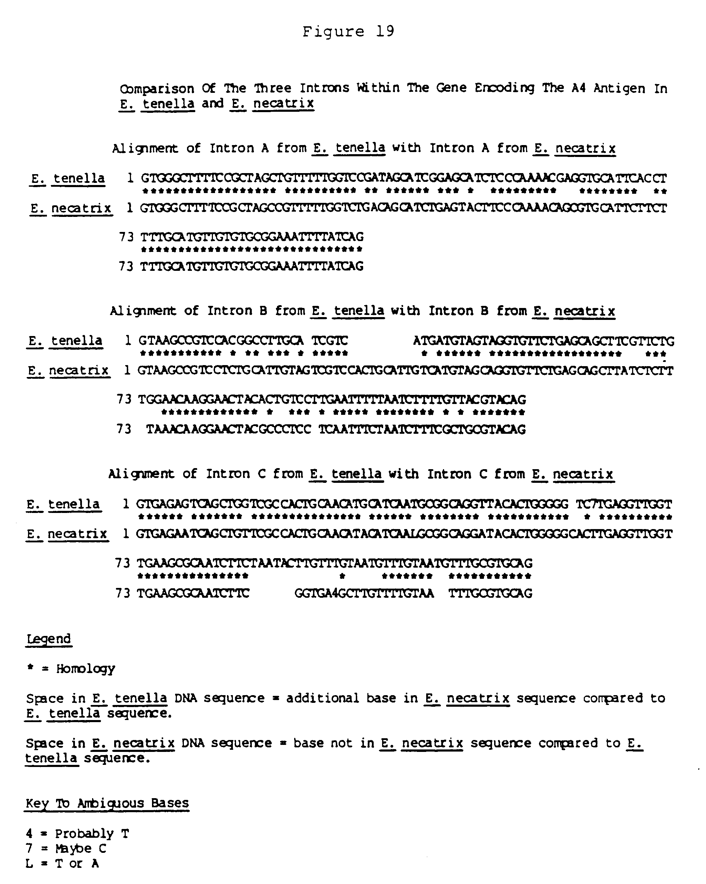

Figure 19 shows the alignment of the three introns within the genes. Intron A is 101 bp in both species and shows 89 % sequence homology. Intron B is 114 bp in E. tenella and 122 bp in E. necatrix with 74 % homology. Intron C is 124 bp in E. tenella and 117 bp in E. necatrix with 77 % sequence homology; Thus, the introns are clearly different.

EXAMPLE 24

ISOLATION AND IDENTIFICATION OF mRNA ENCODING The NA4 ANTIGEN

Before cDNA encoding the NA4 antigen could be synthesized it was necessary to determine when the mRNA encoding the NA4 antigen was present during sporulation. 2.5 x $10^8$ oocysts were sporulated at 30°C, with gentle mixing for 40 hours. The sporulating oocysts were centrifuged at 7-800 x g for 10 minutes and the supernatant was removed. The pellets were quick-frozen in a dry ice/methanol bath and then stored at -70°C until RNA was isolated.

Each pellet was thawed in approximately 10 volumes of 5 M guanidine thiocyanate, 20 mM Tris-HCI pH 7.5, 10 mM EDTA, 5 % (v/v) beta-mercaptoethanol and oocysts were rapidly broken by shaking vigorously with an equal volume of 1.0 mm glass beads for 10 minutes. After bringing the samples to 2 % (w/v) N-lauroylsarcosine they were centrifuged at approximately 8,000 x g at room temperature to remove debris. RNA was isolated from the supernatant by sedimentation through a CsCl cushion (76).

The RNA pellet was resuspended in 20 mM Tris-HCL pH 7.5, 50 mM EDTA pH 8.0, 0.2 % SDS, 100 units/ml RNasin® (Promega Biotec, Madison, WI, 10mM betamercaptoethanol). After extracting twice alternatively with phenol-chloroform:isoamyl alcohol (24:1) and chloroform:isoamyl alcohol (24:1) the RNA was precipitated and stored in ethanol at -20°C. Approximately 85-150 micrograms of total RNA was isolated from (0.5-1.0 x $10^9$) oocysts.

PolyA-containing RNA was isolated by oligo-dT cellulose chromatography (2). Total RNA was loaded on an oligo-dT cellulose column (Type 3, Collaborative Research, Inc; Lexington, MA) in 10 mM Tris-HCl pH 7.5, 1 mM EDTA, 0.2 % (w/v) SDS, 0.4 M LiCl. RNA was eluted at 40°C in the same buffer without LiCl. Approximately 10 micrograms $A^+$ RNA was isolated from 5.0 x $10^8$ oocysts.

Before polyA RNA could be used as a template for cDNA synthesis, it was necessary to demonstrate the presence of the mRNA encoding the NA4 antigen. The presence of the NA4 antigen mRNA was demonstrated by hybridizing polyA RNA from fully sporulated (40 hours) oocysts with DNA from the clone encoding the TA4 protein. Ten micrograms of total RNA isolated from the sporulated oocysts was electrophoresed through gels containing formaldehyde (44). The RNA was transferred to nylon filters for Northern blot analysis. The nylon filters were probed with a [$^{32}$p] nick-translated (44) DNA fragment consisting of the first approximately 300 bp of the TA4 cDNA fragment consisting of the first approximately 300 bp of the TA4 cDNA clone. The DNA sequence of this fragment is over 80 % homologous with its corresponding regions in the NA4 gene sequence and therefore is chosen as an appropriate probe. The mRNA encoding the NA4 antigen was indeed present in the oocysts sporulated for 40 hours. These experiments demonstrate that mRNA from oocysts sporulated for 40 hours could be used to make cDNA encoding the NA4 antigen.

EXAMPLE 25

ISOLATION AND CHARACTERIZATION OF A cDNA CLONE ENCODING THE NA4 ANTIGEN

cDNA

The nucleotide sequence encoding the NA4 antigen was to be used as a gene in an easily grown cell such as E. coli to produce an NA4 protein for vaccination of chickens against coccidioisis caused by certain Eimeria. There are three regions of the NA4 gene (Figure 17) in which the DNA sequence does not coincide with the protein sequence. These three sequeces are introns typically found within the coding regions of many eukaryotic genes. However, since genes containing introns would not express the proper protein in E. coli it was necessary to isolate a cDNA clone encoding the NA4 antigen. This clone contains a continous coding sequence for the NA4 antigen.

Synthesis of cDNA

Briefly, the sporulated oocyst mRNA isolated as described in Example 24 was transcibed into cDNA using a cDNA synthesis kit purchased from Amersham (Amersham Corporation, Arlington Heights, IL) and used according to their instructions. From 2 micrograms of mRNA we obtained approximately 400 ng cDNA.

Construction of the NA4 cDNA Library

The cDNA was resuspended in 20 microliters of 6 mM Tris-HCI, 6 mM $MgCl_2$ pH 7.4, 6 mM beta-mercaptoethanol. To clone the cDNA into a library, restriction sites were used that had been determined from NA4 genomic clone DNA sequence. A SacI site is immediately upstream of the N-terminal glutamine of the mature 18,000 dalton subumit of the NA4 antigen, and a second SacI site lies 60 bp downstream from the first. The cDNA was digested with 12 units of SacI in the presence of 6 mM Tris-HCI (pH 7.4), 6 mM $MgCl_2$, and 6 mM beta-mercaptoethanol for 150 minutes at 37°C.

For cloning the major portion of the cDNA, i.e., from the second SacI site to the end of the gene, a pUC18 (56) was used. The vector has beer digested with SacI and SmaI. SmaI provided the blunt end site necessary for ligation of the 3' end of the cDNA. Tile ligation reaction was performed using about 40 ng of vector DNA and 40 ng of cDNA. Ligations were done overnight at 12°C in a ligase buffer of 50 mM Tris-HCI (pH 7.8), 10 mM $MgCl_2$, 20 mM dithiothreitol, 1.0 mk rATP using one unit of T4 DNA ligase.

The recombinant DNA molecules were then introduced into Escherichia coli K-12 strain MH1 by transformation. The transformed bacteria were spread on agar plates containing the antibiotic ampicillin at a concentration of 50 micrograms/ml. Since the plasmid pUC18 (56) contains the ampicillin resistance gene, only those bacteria which acquired a recombinant plasmid survived. These bacteria each grew and divided to form a bacterial colony. Each cell in the colony is a descendant of the original parental cell and contains the same recombinant plasmid. Approximately 20,000 clones were obtained from 20 nanograms of the SacI-digested cDNA used to make recombinant plasmids.

Identification of NA4 cDNA Clones

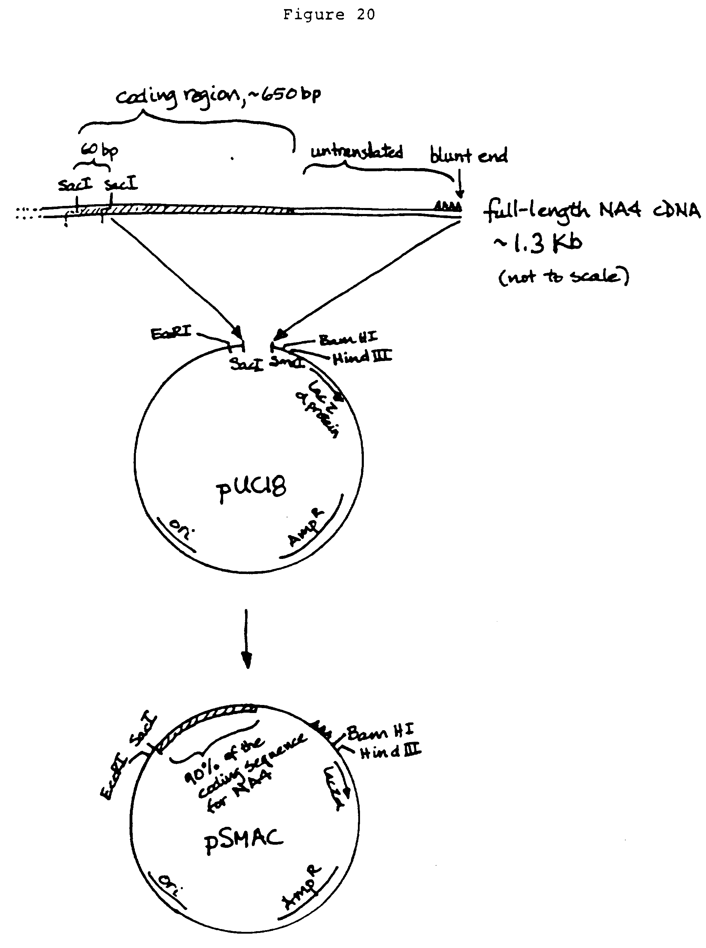

This cDNA library was screened by colony hybridization using the high density screening method described by Grunstein and Hogness (20). The same 300 bp fragment of the TA4 cDNA clone which had been used to hybridize to the mRNA previously described was purified and labeled with $^{32}$p by nick-translation (44). Positive clones were identified, purified and plasmid DNA was isolated for further analysis. Restriction analysis of the positive cDNA clone pSMAC agreed with the map predicted from the NA4 genomic clone. The cDNA insert of the clone designated as pSMAC was sequenced by dideoxy sequencing using oligonucleotide primers made to correspond to the genomic clone (62). The construction of pSMAC is illustrated in Figure 20. This cDNA clone was transformed into an E. coli strain JM83, and the strain designated as JM83/pSMAC was deposited with the American Type Culture Collection, Rockville, MD, and assigned ATCC Accession N° 67 241. This deposit was made pursuant to the Budapest Treaty.

The DNA sequence agreed with that predicted from the genomic clone.

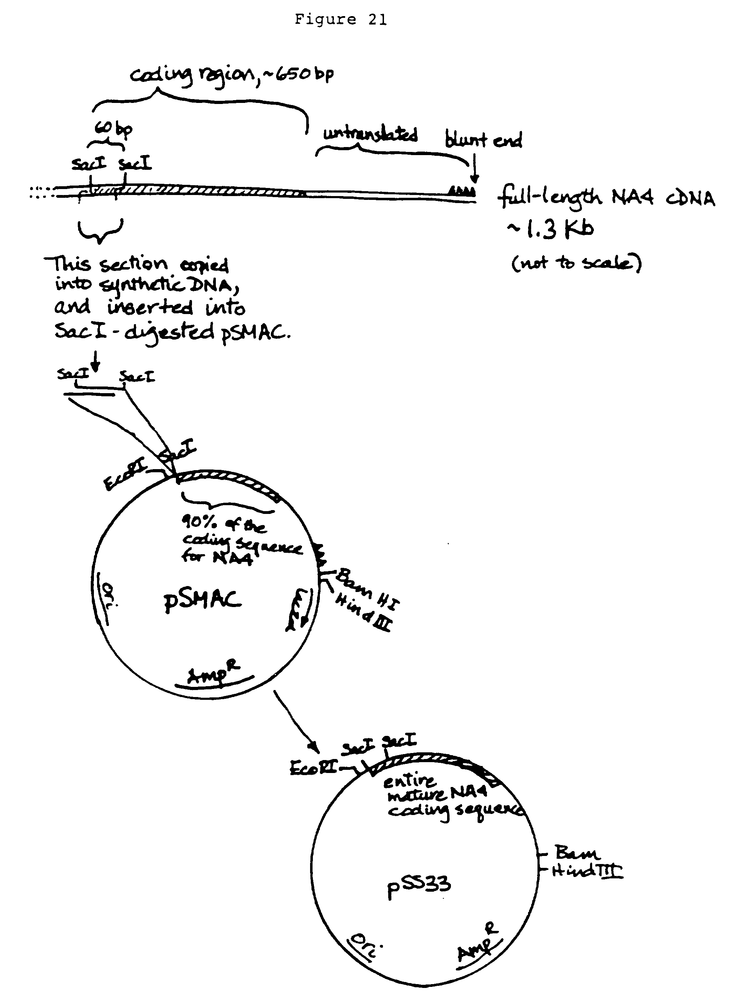

The plasmid pSMAC encodes over 90 % of the cDNA, but lacks the beginning 60 bp of cDNA sequence for the mature NA4 protein. For purposes of simplicity, we chose to synthesize these 60 bp of cDNA using the sequence predicted from the NA4 genomic clone 7. Two oligonucleotides, COD 391 and COD 392, were synthesized on a Biosearch 8600 DNA Synthesizer (Biosearch, San Rafael, California), purified by HPLC, mixed in equimolar amounts, heated to 90° for 5 minutes and allowed to cool to 22°C. The annealing of these two oligonucleotides forms a DNA fragment with SacI ends whose sequence is identical to the sequence of the Na4 gene between the two SacI sites near the 5' end of the gene. This synthetic fragment was then ligated into SacI-digested pSMAC, the resultant recombinant molecules were transformed into MHI and transformants were screened by DNA sequencing to determine which clone had the SacI-SacI 60 bp fragment in the correct orientation to encode the full-length NA4 cDNA. pSS33 is this plasmid. The construction of pSS33 is illustrated in Figure 21.

In order to facilitate the construction of NA4 cDNA expression plasmids, a second plasmid which also encodes the full-lenght NA4 cDNA was constructed. This plasmid is pNCD. pNCD contains an additional nucleotide base pair inserted into the pUCl8 sequence immediately upstream of the SacI site which marks the 5' end of the NA4 cDNA in pSS33. The addition of this base pair shifts the reading frame so that when the EcoRI site of either bovine prochymosin (pWHA93) or E. coli betagalactosidase (pDK2) the reading frame will be maintained and a fusion protein of prochymosin -NA4 or betagalactosidase-NA4 can be produced.

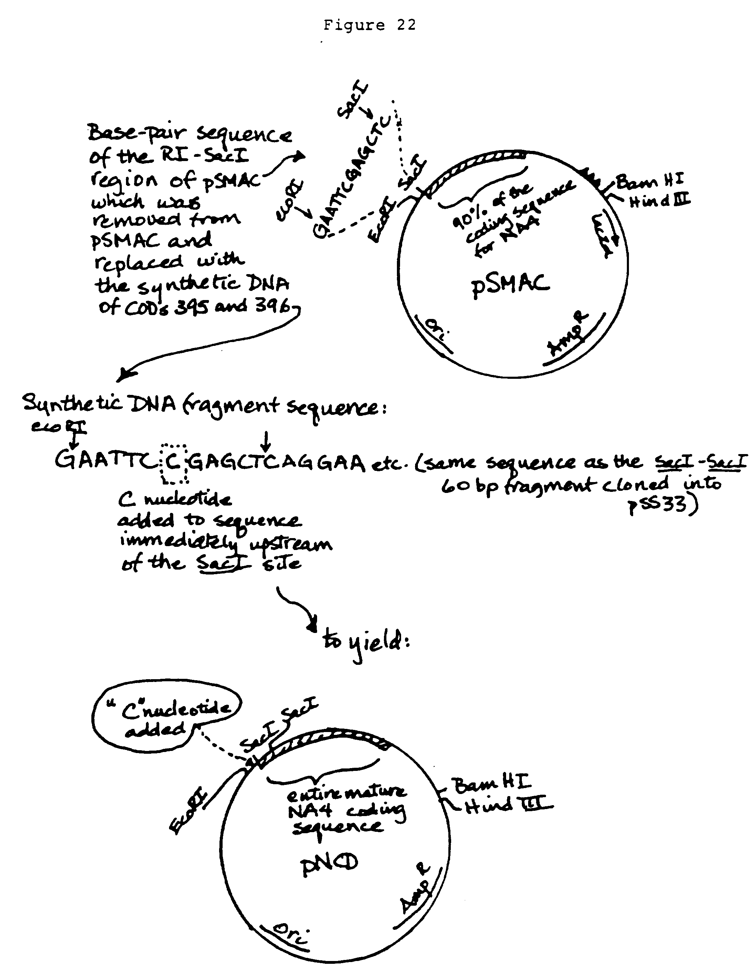

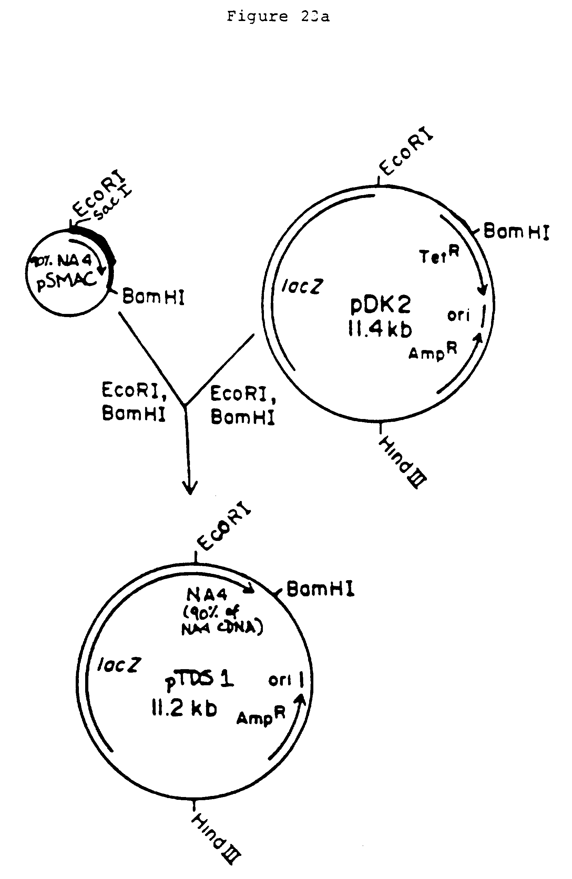

pNCD was derived from pSMAC in a manner analogous to that used to create pSS33. Synthetic oligonucleotides COD395 and COD396 were made, purified, and annealed. The DNA fragment they form when annealed has an EcoRI end and a SacI end. This fragment was ligated into EcoRI-SacI digested pSMAC, and the resultant recombinant plasmids transformed into E. coli K-12 strain Mhl. The construction of pNCD was verified by di-deoxy sequencing. The construction of pNCD is illustrated in Figure 22. pNCD was transformed into E. coli host cell JM83 and deposited with the ATCC under Accession N° 67 226.

EXAMPLE 26

EXPRESSION OF THE cDNA DERIVED NA4 ANTIGEN GENE IN E. COLI

Construction of cDNA Derived NA4 Expression Plasmids

The cDNA clone provides the gene for synthesis of the NA4 protein in bacteria. However, the cDNA does not contain the proper signals to allow transcription and translation in E. coli. Therefore, the cloned cDNA was inserted into expression vectors that contain a stong promoter(s) for RNA polymerase and a ribosome binding site to initiate protein synthesis in E. coli upstream of the inserted cDNA. As used herein, the phrase NA4 protein refers to the expression product of the NA4 cDNA encoded by any derivative of pSS33 or pNCD, or any recombinant NA4-derived material produced in a bacterial host cell. The phrase NA4 antigen refers to the naturally-occurring material as expressed by the genomic NA4 DNA, as present on the surface of the sporozoite or purified away from sporozoites.

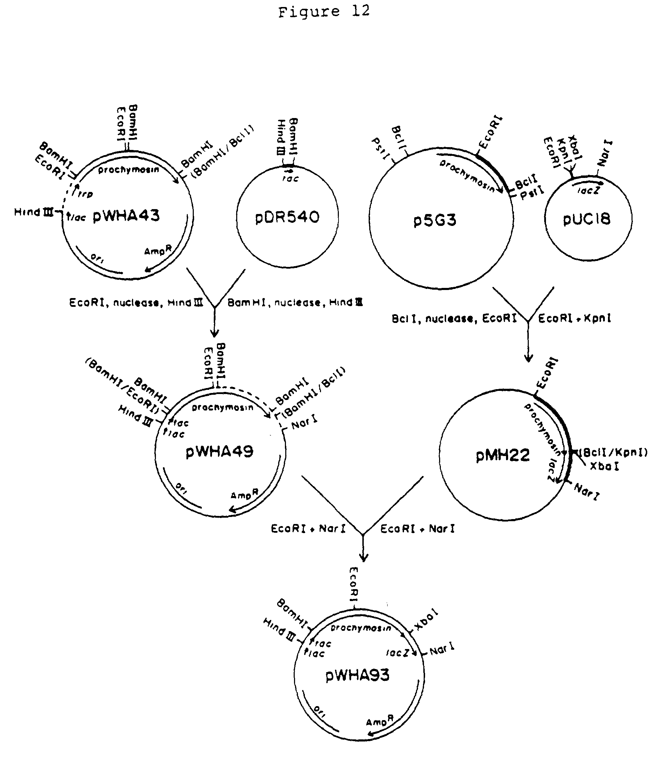

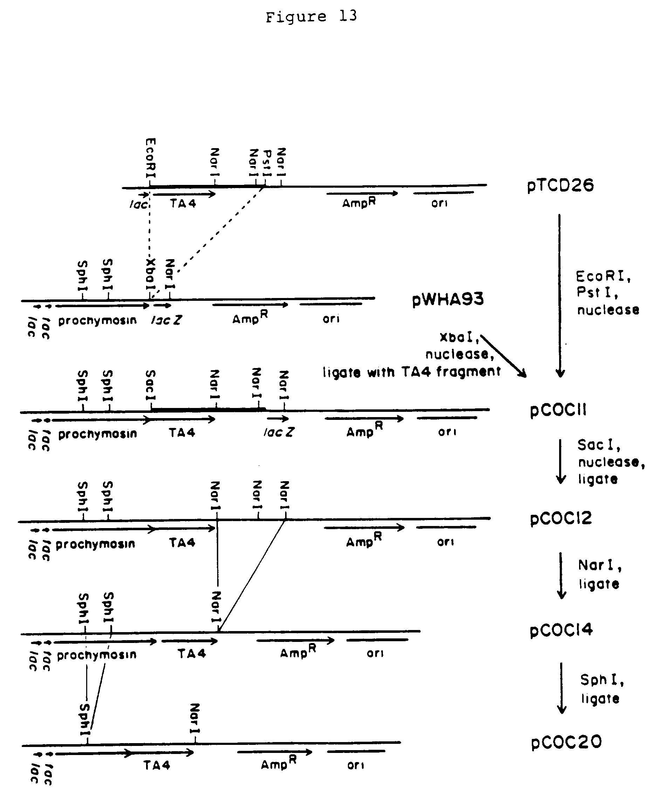

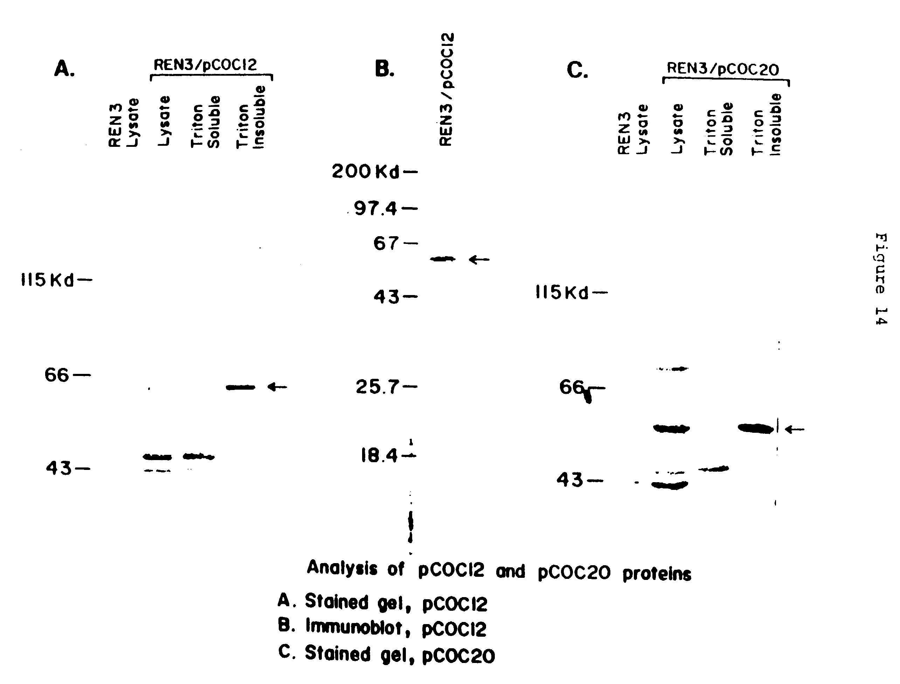

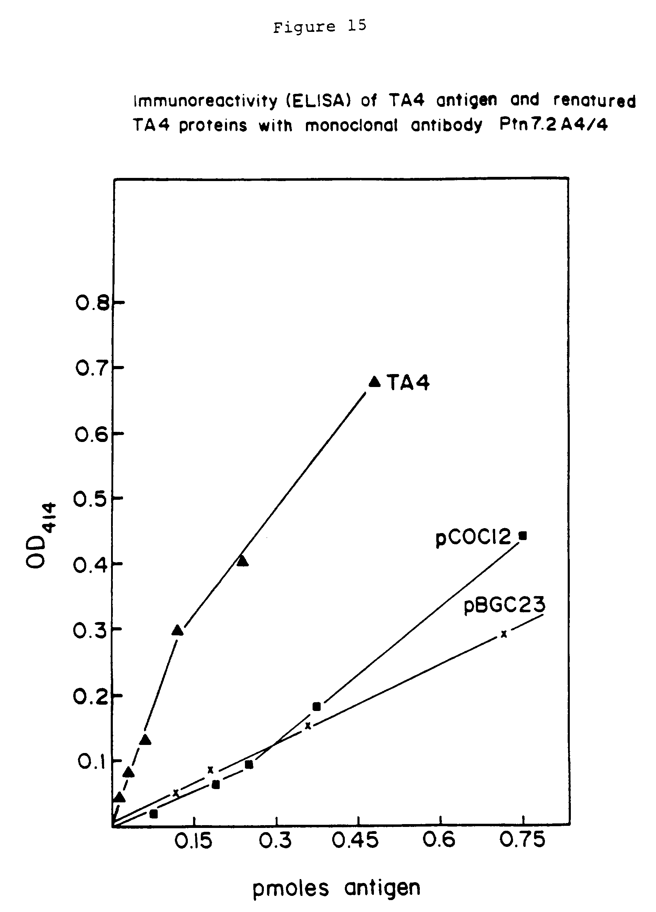

Expression vectors pWHA93 and pDK2 were constructed so that genes could be inserted into them to obtain expression in E. coli. Other suitable plasmids known to one skilled in the art could also be used. Plasmids pWHA93 and pDK2 are two examples of suitable plasmids. The pWHA93 plasmid contains two promoters, lac and tac (the tac promoter is from plasmid pDR450 ; 34 ; Pharmacia Molecular Biology Division, Piscataway, NJ), each of which could direct transcription of an inserted gene. The structure of plasmid pWHA93 is diagrammatically shown in Figure 12.

Because the expression levels of the analogous E. tenella protein TA4 were far higher when expressed as fusion proteins rather than directly expressed, the NA4 protein was stabilized by fusion to other proteins. Any suitable protein could be utilized for this protein fusion. The following examples illustrate only two of the possible proteins which are suitable ; namely betagalactosidase and prochymosin.

EXAMPLE 27

EXPRESSION OF THE NA4 PROTEIN AS A BETA-GALACTOSIDASE FUSION PROTEIN IN E. COLI

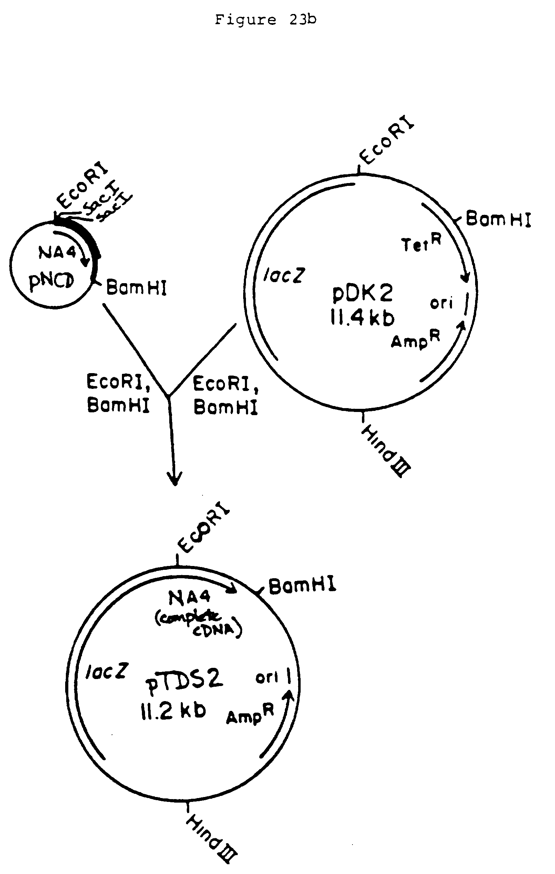

Construction of Beta-Galactosidase - NA4 Expression Plasmids. NA4 gene fusion plasmids were constructed because attachment of the NA4 protein to a large protein can stabilized it in E. coli. Several eukaryotic proteins are more stable in bacteria as fused proteins (17, 27). Recombinant plasmids pTDS1 and pTDS2 are hybrids constructed for expression of a beta-galactosidase-NA4 antigen fusion protein. These were derived from a plasmid pDK2 which contains the lac regulatory region and the whole beta-galactosidase gene from lambda plac (22, 63) inserted into the EcoRI site of plasmid pBR328, and from the cDNA clones pSMAC and pNCD. Suitable plasmids other than pDK2 can also be used. Plasmid pDK2 is one example of a suitable plasmid. The 1.3 kb EcoRI-BamHI fragments from pSMAC and pNCD, containing either 90 % or the entire NA4 cDNA sequence respectively, were cloned into pDK2 plasmid DNA that had been digested with EcoRI and BamHI to generate plasmids pTDS1 and pTDS2 respectively. Clones pTDS1 and pTDS2 contained the expected plasmids in which either 90 % or the entire NA4 cDNA sequence was fused in reading frame to the C-terminal region of the beta-galactosidase coding sequence. The constructions of pTDS1 and pTDS2 are illustrated in Figures 23A and 23B.

The recombinant DNAs and their host microorganisms described herein as MH1/pTDS1 and MH1/pTDS2 were deposited with the American Type Culture Collection, Rockville, MD and assigned ATCC Accession Numbers 67 240 and 67 264 respectively. These deposits were made pursuant to the Budapest Treaty. The pTDS1 and pTDS2 proteins are synthesized in E. coli at high levels, but are insoluble and do not react with monoclonal antibody Ptn 7.2 A4/4.

EXAMPLE 28

EXPRESSION OF THE NA4 PROTEIN AS A PROCHYMOSIN FUSION PROTEIN IN E. COLI

The proteins made by cells containing pTDS1 and pTDS2 are largely or totally insoluble, and thereby are apparently not reactive with monoclonal antibody Ptn 7.2 A4/4. It was observed that other eukaryotic proteins that are made in E. coli in an insoluble, inactive from can be solubilized and their activity recovered. One such protein is bovine prochymosin. The NA4 cDNA sequence was fused to the bovine prochymosin gene to produce an insoluble fusion protein that could be solubilized and made active by procedures developed for prochymosin alone. The extent of proper renaturation of the fusion protein could then be monitored by immunoreactivity with monoclonal antibody Ptn 7.2 A4/4 in a plate ELISA.

A plasmid-encoded prochymosin-NA4 fusion protein was created by joining the NA4 cDNA sequence to the cloned bovine prochymosin gene of pWHA93 (described in Example 12). Other plasmids may also be utilized. One suitable plasmid is pWHA93.

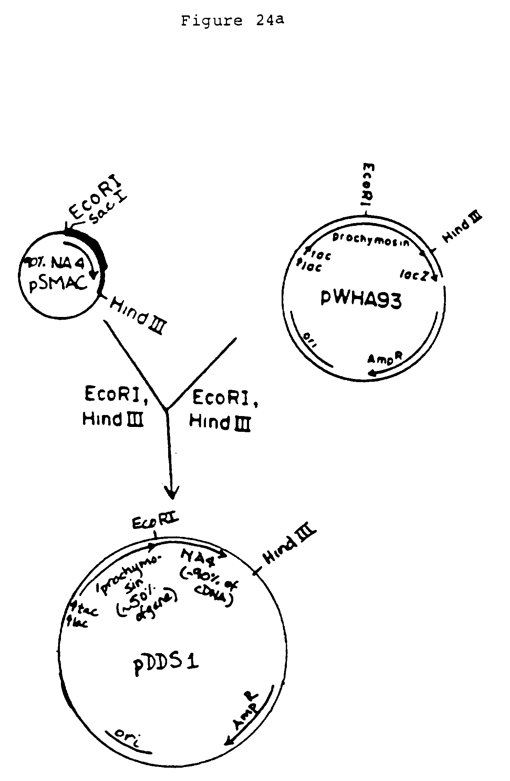

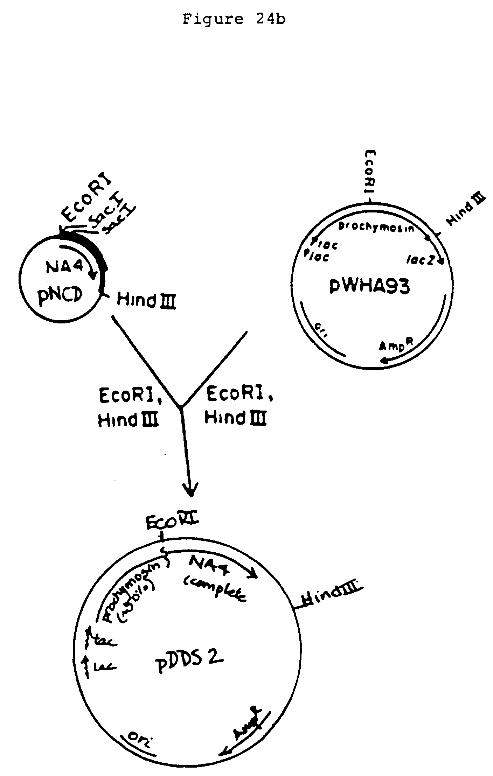

In the construction of the prochymosin-NA4 gene fusion, pDDS1 and pDDS2, an approximately 1.3 kb fragment was removed from each of the cDNA clones pSMAC and pNCD by digestion with the enzymes EcoRI and HindIII. The plasmid pWHA93 was similarly digested with EcoRI and HindIII, the larger of the two fragments so generated was gel-purified and was ligated with each of the EcoRI-HindIII fragments containing the NA4 cDNA sequences from pSMAC and pNCD to generate the recombinant plasmids pDDS1 and pDDS2, respectively. The construction of pDDS1 and pDSS2 is diagrammatically shown in Figures 24A and 24B.

The recombinant DNAs and host microorganisms described herein as MH1/pDDS1 and JM83/pDDS2 were deposited with the American Type Culture Collection, Rockville, MD and assigned ATCC Accession Numbers 67 243 and 67 265 respectively. These deposits were made pursuant to the Budapest Treaty.

EXAMPLE 29

EXTRACTION OF THE NA4 PROTEIN FROM THE INSOLUBLE STATE AND DEMONSTRATION OF IMMUNOREACTIVITY WITH MONOCLONAL ANTIBODY PTN 7.2 A4/4

The E. Coli products of expression plasmids pTDS1, pTDS2, pDDS1, and pDDS2 are all largely or totally insoluble. All can be solubilized by boiling in Laemmli sample buffer and will react with mouse antiserum raised against the 17,000 dalton TA4 antigen subunit which is highly homologous to that part of the NA4 antigen. However, none react with monoclonal antibody Ptn 7.2 A4/4 under these conditions. Therefore, it was necessary to and renature these E. coli synthesized proteins to produce antigens in a form that would react with monoclonal antibody Ptn 7.2 A4/4 and therefore could possibly raise neutralizing and protective antibody responses against E. necatrix and E. tenella in animals.

Extraction and Renaturation of Bacterially Produced NA4 Fusion Proteins

First the NA4-fusion proteins were solubilized and renatured by methods known to solubilize and renature bovine prochymosin to produce active enzyme (47). This procedure produced pure soluble NA4-fusion proteins that possessed Ptn 7.2 A4/4 immunoreactivity. Conditions were optimized for recovery of immunoreactivity and are described below.

Plasmids pTDS1, pTDS2, pDDS1 and pDDS2 were constructed, as described above. These plasmids were used to transform E. coli strain SG936 using standard techniques and ampicillin resistant colonies were purified and used culturing. In each case, an ampicillin resistant colony from a freshly streaked agar plate was used to inoculate a 100 ml liquid culture containing L-broth and ampicillin at 100 micrograms/ml. The culture was grown overnight at 30°C with shaking. The 100 ml culture was transferred to a flask containing 1 liter of L-broth/ampicillin. This culture was grown at 30°C with shaking to $OD_{600}$ of 1.0. IPTG was added to 2 mM and the culture was grown 2-3 hours more at 30°C. Cells were collected by centrifugation and stored frozen at -70°C. The frozen cell pastes of E. coli strain SG936, each containing one of the NA4 expression plasmids, were each suspended in 40 ml of 25 mM Tris-HCl pH 8, 10 mM EDTA, 0.5 mg/ml lysozyme. After a short incubation, the lysed cells were further disrupted by sonication. Because the NA4 fusion proteins synthesized in E. coli had been shown to be completely insoluble in cell lysates, the plasmid-encoded NA4 proteins were purified by centrifugation of the cell lysates at 100,000 x g for 1 hour, followed by a detergent extraction of the pelleted cell debris with a buffer solution containing 5 % Triton x-100 detergent (Sigma Chemical Co., St. Louis, MO), 20 mM EDTA, for 60 minutes at 25°C. The NA4 fusion proteins remained insoluble and were at 25°C. The NA4 fusion proteins remained insoluble and were collected by centrifugation at 100,000 x g.

The insoluble material was suspended in 12 ml, 10 mM Na phosphate (pH 7.5) and collected by centrifugation, to remove remaining detergent. The NA4 fusion proteins were suspended in 10 mM sodium phosphate buffer at pH 7.5 to a final volume of 7.7 ml. The suspensions were fully solubilized by the addition of 5.8 g solid urea to a final concentration of 8 M in a volume 12 ml, and then mixed for 16 hours at room temperature.

The resultant clear solutions were each diluted into 100 volumes of 10 mM sodium phosphate buffer adjusted to pH 11.0 to achieve final volumes of 1200 mls. The solutions were mixed thoroughly and allowed to stand for 10 minutes at 15°C. The pH of the solutions were then titrated to pH 8.5 by addition of 0.5N HCL over a period of 5 minutes.

The resultant solutions were left at room temperature for one hour or more prior to assay or storage. The sample was assayed for immunoreactivity with monoclonal antibody Ptn 7.2 A4/4. The preparation had activity comparable to renatured antigens from pC0C20 as described below.

Immunoassay of Renatured Samples

The immunoreactivity of the renatured pTDS1, pTDS2, pDDS1 and pDDS2 proteins with monoclonal antibody Ptn 7.2 A4/4 was measured. Each well of the microtiter plate (Immulon I microELISA flat-bottom well plates, Dynatech Laboratories, Inc., Alexandria VA) was coated with 100 microliters antigen diluted in 10 mM $Na_2HPO_4$, 150 mM NaCl, 0,01 % (w/v) Zwittergent® 3-12, pH 8.0. For renatured samples, 1:10 to 1:1000 dilutions of the antigen were assayed. Plates were coated with the antigens by incubation with the antigen solutions for 1 hour at room temperature and then overnight at 4°C. Wells were emptied and then washed three times with phosphate buffered saline pH 7.2 containing 0.02 % (v/v) Tween-20 (PBST). The plates were treated with 3 % (w/v) gelatin, 10 mM Tris-HCl pH 7.5, 150 mM NaCl, 0.05 % (v/w) $NaN_3$ for 30 minutes at room temperature to block any remaining protein binding sites. Plates were then incubated with 100 microliters of monoclonal antibody Ptn 7.2 A4/4 (30 micrograms/ml in 3 % [w/v] bovine serum albumin), 10 mM Tris-HCl pH 7.5, 150 mM NaCl, 0.05 % (w/v) $NaN_3$) for 2 hours at room temperature. After rinsing the wells three times with PBST, the bound monoclonal antibody Ptn 7.2 A4/4 was determined using the Vectastain® ABC Kit for mouse IgG (Vector Laboratories, Inc., Burlingame, CA). Each well of the plate was filled with 100 microliters of biotinylated horse antimouse IgG (40 microliters biotinylated anti-mouse antibody, 80 microliters normal horse serum in 10 ml PBST) and incubated 30 minutes at room temperature. Plates were rinsed three times with PBST. Plates were then incubated with 100 microliters/well of Vectastain® ABC Reagent for 30 minutes at room temperature (80 microliters Avidin DH Reagent A mixed with 80 microliters biotinylated horseradish peroxidase Reagent B in PEST preincubated for 30 minutes before addition to the plates). After five washes with PBST bound horseradish peroxidase was measured by the addition of 100 microliters substrate/well (0.1 mg/ml 2, 2'-azino-di-(3-ethyl-benzthiazoline) 6-sulfonic acid in 50 mM citrate/phosphate buffer pH 5.3, 0.015 % (v/v) hydrogen peroxide). Plates were incubated in the dark at room temperature. The absorbance at 414 nm was measured 10-60 minutes after substrate addition in a Titertek Multiscan® automatic plate reader (Flow Laboratories, Inc. McClean, VA).

The immunoreactivity of the NA4 fusion proteins was found comparable to the TA4 fusion protein pC0C20.

EXAMPLE 30

USE OF PURIFIED E. NECATRIX NA4 PROTEIN TO ELICIT A SPOROZOITE NEUTRALIZING SERUM RESPONSE AGAINST E. TENELLA IN CHICKENS

The antigen used in these experiments was prepared from sporocysts as described in Example 21. Prior to use in chickens, identity and purity of the protein was confirmed by SDS-PAGE and immunoreactivity with monoclonal antibody Ptn 7.2A4/4.

One part purified antigen diluted in 0.15 M phosphate buffered saline was emulsified to a final volume of one ml in three parts carrier consisting of about 5 % Arlacel A, 94 % Drakeol 6-VR, 1 % Tween-20. Chickens received 15 micrograms antigen/0.2 cc dose in the neck. Antigen was administered at 14-day intervals two additional times by the same route.

Three days prior to each administration of protein, and eleven days after the final administration, birds were bled for collection of serum samples. Heat inactivated sera were tested independently in the sporozoite microneutralization assay as described in Example 2.

The results as set forth in Table XIV below indicate that whereas nonvaccinated birds receiving carrier only had no demonstrable neutralizing antiserum titers against E. tenella sporozoites, birds receiving three doses of the antigen had demonstrable neutralizing antiserum titers.

Table XIV

| NA4 Antigen Induced Sporozoite Neutralization Assay Data Sporozoite Neutralization Titers (ND50 %)[c] | | | |
|---|---|---|---|
| Serum Sample | Highest | Lowest | Median Titer |
| Prebleed[a] | N.D. | N.D. | N.D. |
| Nonvaccinate Control (n=9) | N.D. | N.D. | N.D. |
| Carrier Only (n=14) | N.D. | N.D. | N.D. |
| Carrier/NA4 Protein Vaccine (n=15) | 1:32 | N.D. | 1:8 |
| Immune Serum[b] (Whole Sporozoite Vaccinates) | --- | --- | 1:32 |

[a] Serums from birds within each treatment group were pooled and tested

[b] Pooled serum from several birds

[c] 50 % neutralization dose.

N.D. not detectable

EXAMPLE 31

USE OF PURIFIED E. NECATRIX NA4 PROTEIN TO ELICIT A PROTECTIVE RESPONSE TO AN E. TENELLA CHALLENGE

On three occasions at 14 day intervals, 4 week-old white Leghorn chickens received 15 micrograms of immunoaffinity purified NA4 protein/0.2cc dose by intramuscular route in the neck muscle. The antigen was formulated in PBS and emulsified 60 micrograms/ml final volume in three parts of the aforementioned carrier. A second group of birds received the carrier substrate only. A third group was note vaccinated. A final group nonvaccinated birds housed with the NA4 vaccinated birds served as sentinels. Birds were randomly placed in an E. tenella contaminated battery. Ten days after exposure to E. tenella, birds were challenged with an oral dose of $1 \times 10^4$ E. tenella oocysts. Twenty-four hours later, birds received an additional $3 \times 10^4$ oocysts orally. All birds were sacrificed and lesions scored 5 days after receiving the last oral dose of E. tenella. The results are reported in Table XV below.

Table XV

| Treatment Groups | Lesions Scores (x+S.D.) |
|---|---|
| Carrier Only | 4.0 + 0.0 |
| Carrier/Protein | 2.4 + 1.3 |

Table XV   (continued)

| Treatment Groups | Lesions Scores (x+S.D.) |
|---|---|
| Nonvaccinated Controls | 3.4 + 0.6 |

These results would suggest to one skilled in the art that the birds receiving NA4 protein were measurably protected against disease due to severe challenge with E. tenella. The lesion scores for groups receiving the NA4 protein were lower than respective control groups.

EXAMPLE 32

RESPONSE IN CHICKEN TO EXPOSURE TO RECOMBINANT EIMERIA NECATRIX (NA4) ANTIGEN AND THEIR CROSS-REACTIVITY WITH E. TENELLA

Specific response of NA4 vaccinated chickens to Eimeria necatrix and E. tenella. An experiment was conducted to demonstrate the immunoreactivity of NA4 vaccinated chickens to the sporocyst derived membrane protein of E. necatrix and E. tenella, and to compare these to the reaction of TA4 vaccinated chickens against the same species. In this experiment, ten birds were vaccinated with pTDS1 (a beta galactosidase/NA4 fusion product, see Example 27) ten were vaccinated with pDDS1 (a prochymosin/NA4 fusion product, see Example 28). These were compared to ten pC0C20 (a prochymosin/TA4 fusion product, see Example 12) vaccinated birds. Immunoractivity of the proteins were assayed and confirmed with the monoclonal antibody Ptn 7.2A4/A prior to their incorporation into the experiment.

Antigen was prepared in a 3:1 ratio of 5 % Arlacel-A, 94 % Drakeol 6-Vr and 1 % Tween 80 carrier to 50 micrograms of antigen with 4 micrograms of LPS as an immunopotentiator. This formulation was delivered as a 0.5-ml subcutaneous dose behind the head. Vaccination regimen consisted of three doses at 10-day intervals, with birds bled and serum collected and stored frozen at each vaccination. Controls for the experiment consisted of pC0C20 TA4 antigen/carrier/ LPS ; carrier/LPS ; and nonvaccinated controls.

Sera from the vaccinates and controls were analyzed for immune reactivity against E. necatrix and E. tenella sporocyst protein by Western Blot as described in Example 13. As seen in Table XVI below, vaccination of chickens with pDDS1, pTDS1 and pC0C20 antigens confer specific serologic response to the homologous and heterologous species of parasite.

Table XVI

| NA4 Vaccinate Immunoreactivity Assay | | | | | | |
|---|---|---|---|---|---|---|
| Treatment Group | 1st Bleed Eimeria | | 2nd Bleed Eimeria | | 3rd Bleed Eimeria | |
| | Ten | Nec. | Ten. | Nec. | Ten. | Nec. |
| pDDS1 (n=10) | 2/8 | 0/8 | 6/8 | 7/8 | 7/8 | 8/8 |
| pTDS1 (n=10) | 1/6 | 2/6 | 1/8 | 2/8 | 1/7 | 3/8 |
| pCOC (n=15) | 7/9 | 5/9 | 6/6 | 4/6 | 6/6 | 6/6 |
| Adjuvant Control (n=10) | 0/5 | 0/5 | 0/5 | 0/5 | 0/5 | 0/5 |

Protective response in chickens vaccinated with the NA4 recombinant protein. Ten days following the vaccination schedule outlined above, chickens were inoculated with E. necatrix or E. tenella oocysts, and examined for pathognomonic lesions of the parasites. Birds of the various treatment groups were inoculated with 5,000 sporulated oocysts of E. tenella and 60,000 oocysts of E. necatrix. The inoculum had been previously titrated to result in the desired severity of lesions, which were scored five days post-challenge as in Example 18.

The results as shown below in Table XVII demonstrate a reduction in severity of lesions in the vaccinate groups as compared to the controls.

Table XVIII

| Lesion Scores of Recombinant NA4 Vaccinated Chickens When Challenged With E. necatrix and E. tenella Oocysts Lesion Score X+s.d. | | |
|---|---|---|
| Treatment Group (n=5) | E. Necatrix Challenge | E. tenella Challenge |
| pDDS1 | 2.0 ± 0.8 | 3.4 ± 0.9 |

Table XVIII   (continued)

| Lesion Scores of Recombinant NA4 Vaccinated Chickens When Challenged With E. necatrix and E. tenella Oocysts Lesion Score X+s.d. | | |
|---|---|---|
| Treatment Group (n=5) | E. Necatrix Challenge | E. tenella Challenge |
| pTDS1 | 1.75 ± 0.9 | 2.8 ± 0.6 |
| pC0C20 | 2.2 ± 0.4 | 3.0 ± 0.5 |
| Adjuvant | 2.6 ± 0.7 | ND |
| Challenge Cont. | 2.8 ± 0.8 | 3.8 ± 0.6 |

Sporozoite neutralizing serum response in chickens vaccinated with recombinant NA4 antigens. A sporozoite neutralization assay (SNA) was utilized to assess the ability of pDDS1 and pTDS1 to confer parasite neutralizing capability to the serum of vaccinated birds. Using the SNA protocol established in Example 18, sera from the aforementioned vaccinate and control groups were assayed for sporozoite neutralizing capability.

As described in Table XVIII below, sera from the pTDS1 and pDDS1 vaccinated birds which were collected following the third vaccination were effective at inhibiting the development of primary meronts.

Table XVIII

| E. tenella Sporozoite Neutralization Assay for Recombinant NA4 Vaccinated Chickens | | | | | |
|---|---|---|---|---|---|
| Treatment Group | Sporozoite Neutralization Titers | | | | |
| | <1:4 | 1:4 | 1:8 | 1:16 | 1:32 |
| pDDS1 (n=10) | 6/10 | 3/10 | 1/10 | --- | --- |
| pTDS1 (n=10) | 2/10 | --- | 4/10 | 4/10 | --- |
| pC0C20 (n=10) | 7/10 | --- | 1/10 | 2/10 | --- |
| Adjuvant Control (n=10) | 9/10 | 1/10 | --- | --- | --- |
| Sporozoite Immune Serum (n=2) | --- | --- | --- | 2/2 | --- |

REFERENCES

1. Ali, N.S., Binnerts, W.T. and Klimes, B. (1972). Immunization by (sic) irradiated Eimeria acervulina. J. Prot. 19, 177.

2. Aviv, H. and Leder, P. (1972). Purification of biologically active globin messenger RNA by chromatography on oligothymidylic acid cellulose. Proc. Natl. Acad. Sci. 69, 1408.

3. Benton, W.D. and Davis, R.W. (1977). Screening λ gt recombinant clones by hybridization to single plaques in situ. Science 196, 180-182.

4. Blobel, G. and Dobberstein, B. (1975). Transfer of proteins across membranes I. Presence of proteolytically processed and unprocessed nascent immunoglobulin light chains on membrane-bound ribosome of murine myeloma. J. Cell Biol. 67, 835-851.

5. Burnette, W.M. (1981). "Western Blotting" : Electrophoretic transfer of proteins from sodium dodecyl sulfate - polyacrylamide gels to unmodified nitro-cellulose and radiographic detection with antibody and radioiodinated protein A. Anal. Biochem. 112, 195.

6. Carlsson, M. ; Hedin, A. ; Inganaes, M. ; Haerfast, B. ; and Blomberg, F. (1985). Purification of in vitro produced mouse monoclonal antibodies. A two-step procedure using cation exchange chromatography and gel filtration. J. Immunol. Methods 79(1) : 89-98.

7. Chung, C.H. and Goldberg, A.L. (1981). The product of the lon(capR) gene in Escherichia coli is the ATP dependent protease, Protease La. Proc. Natl. Acad. Sci. USA 78, 4931.

8. Cohen, S.A., Tarvin, T.L. and Bidlingmeyer, B.A. (1984). Analysis of amino acids using precolumn derivatization with phenylisothiocyanate. American Laboratory 16, 48-59.

9. Danforth, H.D. (1982). Development of hybridomaproduced antibodies directed against Eimeria tenella and E. mitis. J. Parasitol. 68, 392.

10. Danforth, H.D. (1982). Use of monoclonal antibodies directed against Eimeria tenella sporozoites to determine stage specificity and in vitro effect on parasite penetration and development. Am J. Vet. Res 44, 1722.

11. Danforth, H.D. and Augustine P.C. (1983). Specificity and cross-reactivity of immune serum and hybridoma antibodies to various species of avian coccicia. Poultry Science 62, 2145.

12. Davis, P.J., Parry, S.H. and Porter, P. (1978). The role of secretory IgA in anti-coccidial immunity in the chicken. Immunology 34, 879.

13. Davis, P.J. and Porter, P. (1979). A mechanism for secretory IgA mediated inhibition of the cell penetration and intracellular development of Eimeria tenella. Immunology 36, 471.

14. Edman, P. and Begg, G. (1967). A protein sequenator. Automated Equipment for Sequence determination, Eur. J. Biochem 1, 80.

15. Giambrone, J.J., Klesius, P.H. and Edgar S.A. (1980). Avian Coccidiosis : Evidence for a cellmediated immune response. Poultry Sci. 59, 38.

16. Gibbons, R.A., Sellwood, R., Burrow, M. and Hunter, P.A. (1977). Inheritance of resistance to neonatal E. coli diarrhea in the pig : Examination of the genetic system. Theor. Appl. Genet. 51, 65.

17. Goeddel, D.V., Kleid, D.G., Bolivar, F., Heyneker, H.L., Yansura, D.G., Crea, R., Hirose, T., Kraszewski, A., Itakura, K. and Riggs, A.D. (1979). Expression in Escherichia coli of chemically synthesized genes for human insulin. Proc. Nat. Acad. Sci. USA 76, 106-110.

18. Goff, S.A. and Goldberg, A.L. (1985). Production of Abnormal Proteins in E. coli Stimulates Transcription of lon and Other Heat Shock Genes. Cell 41, 587-595.

19. Gore, T.C., Long, P.L., Kogut, M. and Johnson, J. (1983). Attenuation of Eimeria necatrix and E. tenella of U. S. origin by serial embryo passage. Avian Disease 27, 569.

20. Grunstein, M. and Hogness, D.S. (1975). Colony hybridization : A method for the isolation of cloned DNAs that contain a specific gene. Proc. Natl. Acad. Sci. USA 72, 3961.

21. Hagar, D.A. and Burgess, R.R. (1980). Elution of proteins from sodium dodecyl sulfate - polyacrylamide gels, removal of sodium dodecyl sulfate, and renaturation of enzymatic activity : Results with Sigma units of Escherichia coli RNA polymerase, wheat germ topoisomerase, and other enzymes. Anal. Biochem. 109:76.

22. Helling, R.B., Goodman, H.M. and Boyer, H.W. (1974) Analysis of Endonuclease EcoRI Fragments of DNA from Lambdoid Bacteriophages and other viruses by agarose gel electrophoresis. J. Virol. 14, 1235-1244.

23. Helms et al. (1985). DNA 4: 39-49.

24. Howard, R.J., Koushal, D.C. and Caster, R. (1982). Radioiodination of parasite antigen with 1,3,4,6-tetrachloro-3, alpha-6, alpha-diphenyl glycouril (I0D0-GEN™) Studies with zygotes of Plasmodium gallinarum. J. Protozol. 29:114.

25. Hunkapiller, M.W., Hewick, R.M., Dreyer, W.J. and Hood, L.E. (1983). High sensitivity sequencing with a gas phase sequenator. Methods in Enzymology 91, Academic Press, New York, 399-413.

26. Hunkapiller, M.W., Strickler, J.E. and Wilson, K.J. (1984). Contemporary Methodology for Protein Structure Determination. Science 226, 304-311.

27. Itakura, K., Hirose, T., Crea, R., Riggs, A.D., Heynechker, H.L., Bolivar, F. and Boyer, H.W. (1977). Expression in Escherichia coli of a chemically synthesized gene for the hormone somatostatin. Science 198, 1056-1063.

28. Jeffers, T.K. (1975). Attenuation of Eimeria tenella through selection for precociousness. J. Parasitol. 61, 1083.

29. Jeffers, T.K. (1976). Genetic recombination of precociousness and anticoccidial drug resistance on Eimeria tenella. Zeitschrift fur Parasitenkunde 50, 251.

30. Johnson, J. and Reid, W.M. (1970). Anticoccidial Drugs : Lesion scoring techniques in battery and floor pen experiments with chickens. Exp. Parasitology 38, 36.

31. Kasper, L.H., Crabb, J.H., and Pfefferkorn, E.R. (1983). Purification of a major membrane protein of Toxoplasma gondii by immunoabsorption with a monoclonal antibody. J. Immunol. 130, 2407.

32. Keusch, G.T. (1979). Specific membrane receptors : Pathogenic and therapeutic implications in infectious diseases. Rev. Inf. Dis. 1, 517.

33. Kleid D.G., Yansura, D., Small, B., Dowbenko, D., Moore, D.M., Grubman, M.J., McKercher, P.D., Morgan, D. O., Robertson, B.H. and Bachrach, H.L. (1981). Cloned viral protein vaccine for foot and mouth disease : responses in cattle and swine. Science 214, 1125.

34. Kriel, G. (1981). Transport of proteins across membranes. Ann. Rev. Biochem. 50, 317-348.

35. Laemmli, U.K. (1970). Cleavage of structural proteins during the assembly of the head of bacteriophage T4, Nature 227, 680.

36. Leder, P., Tiemeier, D. and Enquist, L. (1977). EK2 derivatives of bacteriophage lambda useful in cloning of DNA from higher organisms : the gt wes system. Science 196, 175-177.

37. Long, P.L. (1972) Eimeria mivati : Reproduction, pathogenicity and immunogenicity of a strain maintained in chick embryos by serial passage. J. Comp. Pathol. 82, 839.

38. Long, P.L. (1974). Further studies on the pathogenicity and immunogenicity of an embryo adapted strain of Eimeria tenella. Avian Pathology 3, 255.

39. Long, P.L. (1982). The Biology of the Coccidia. University Park Press, Baltimore. Pg. 44.

40. Long, P.L., Johnson, J., and Gore, T.C. (1982). Attenuation of a strain of Eimeria mivati of U.S. origin by serial embryo passage. Avian Diseases. 26, 305.

41. Long, P.L. and Rose, M.E. (1965). Active and passive immunization of chickens against induced infections of Eimeria tenella. Exp. Parasit. 16, 1.

42. Long, P.L., Millared, B.J., Joyner, L.P. and Norton, C.C. (1976). A guide to laboratory techniques used in the study and diagnosis of avian coccidiosis. Folia Vet. Lat. 6, 201.

43. Lowder, L.J. (1966). Artificial acquired immunity to Eimeria bovis infections in cattle. Proc. Int. Congr. Parasit. 1, 106.

44. Maniatis, T., Fritsch, E.F. and Sambrook, J. (1982). Molecular Cloning - A Laboratory Manual, Cold Springs Harbor Laboratory, New York.

45. Marquardt, W.C. (1980). Host and site specificity in the Coccidia ; a perspective. J. Protozool. 26, 243.

46. Maxam, A. and Gilbert, W. (1980). Sequencing endlabelled DNA with base-specific chemical change in : Methods in Enzymology, Vol. 65, part 1, Academic Press, New York, pp. 499-559.

47. McCaman, M.T., Andrews, W.H. and Files, J.G. (1985). Enzymatic properties and processing of bovine prochymosin synthesized in Escherichia coli. J Biotech. 2, 177.

48. McDonald, V. and Ballingall, S. (1982). Attenuation of Eimeria mivati (: mitis) by selection for precocious development. Parasitology 86, 371.

49. McDonald, V. and Ballingall, S. (1982). Further investigation of the pathogenicity, immunogenicity and stability of precocious Eimeria acervulina. Parasitology 86, 361.

50. McDonald, V., Ballingall, S. and Shirley, M.W. (1982). A preliminary study of the nature of infection and immunity in chickens given an attenuated line of Eimeria acervulina. Parasitology 84, 21.

51. McDougald, L.R. Status of coccidiosis : New products on way. Poult. Digest. Oct., 1981.

52. McDougald, L.R. New anticoccidial drugs : Better things to come or "endangered species ?" Feedstuffs Aug. 15, 1983.

53. Millar, W.T. and Smith, J.F.B. (1983). Protein iodination using I0D0-GEN™. Int. J. Appl. Radiot. Isol. 34:639.

54. Miller, L.H., Mason, S.J, Dvorak, J.A., McGinniss, M.H., and Rothman, I.K. (1975) Erythrocyte receptors of (Plamodium knowlesi) Malaria : Duffy blood group determinants. Science 189, 561.

55. Miller, J.H. ed. (1972). Experiments in Molecular Genetics. Cold Spring Harbor Laboratory, Cold Spring Harbor, New York, pages 54 and 431.

56. Norrander, J., Kempe, T. and Messing, J. (1983). Construction of improved M13 vectors using oligodeoxy-nucleotide - directed mutagenesis. Gene 26, 101.

57. Reid, W.M. (1978). Protozoa. In : Diseases of Poultry. 7th ed. M.S. Hofstad, ed. Iowa State Univ. Press. pp. 942-1054.

58. Riley, J.F. (1980). Screening for and evaluation of anticoccidial activity. Adv. Pharm. Chemo. 17, 1.

59. Rose, M.E. (1974). Immune responses to the Eimeria : Recent observations. Sympo. Coccidia and Related Organisms. pp. 92-118. Univ. Guelph, Ontario.

60. Rose, M.E. and Hesketh (1976). Immunity to Coccidiosis : Stages of the life cycle of Eimera maxima which induce and are affected by the host. Parasitology 27: 25-37.

61. Russell, D.R. and Bennett, G.N. (1982). Construction and analysis of in vivo activity of E. coli promoter hybrids and promoter mutants that alter the -35 to -10 spacing. Gene 20, 231-243.

62. Sanger, F. and Coulson, A.R. (1978). The use of thin polyacrylamide gels for DNA sequencing. FEBS Lett. 87, 107-110.

63. Shapiro, J., MacHattie, L., Eron, L., Ihler, G., Ippen, K. Beckwith, J., Arditti, R., Reznikoff, W., and MacGillivray, R. (1969). The isolation of pure lac operon DNA. Nature 224, 768-774.

64. Sharma, S.D., Mullenax, J., Araujo, F.G., Erlich, H.A. and Remington, J.S. (1983). Western blot analysis of the antigens of Toxoplasma gondii recognized by human IgM and IgG antibodies. J. Immunology 131, 977.

65. Sharp, P.A. (1981). Speculations on RNA splicing. Cell 23, 643-646.

66. Shirley, M.W. (1980) Eimeria necatrix : The development and characteristics of an egg-adapted (attenuated) line. Parasitology 81, 525.

67. Shirley, M.W. (1982). Features of an attenuated line of Eimeria praecox. Parasitology. Proceedings of the British Soc. for Parasitology 81, 525.

68. Shirley, M.W., Bellatti, M.A. and Millard, B.J. (1982). An egg-shaped (attenuated) line of Eimeria necatrix : further studies on its reproduction pathogenicity and immunogenicity. Parasitology 84, 215.

69. Speer, C.A., Wong, R.B. and Schenkel, R.H. (1983). Effects of monoclonal IgG antibodies on Eimeria tenella (coccidia) sporozoites. J. Parasitol. 69, 775.

70. Speer, C.A., Wong, R.B. and Schenkel, R.H. (1983). Ultra-structural localization of monoclonal IgG antibodies for antigenic sites of Eimeria tenella oocysts, sporocysts and sporozoites. J. Protozoal. 30, 548.

71. Steiner, D.F., Quinn, P.S., Chan, S.J., Marsh, J. and Tager, H.S. (1980). Processing mechanisms in the biosynthesis of proteins. Annals N.Y. Acad. Sci. 343, 1-16.

72. Stotish, R.L., Wang, C.C., Hichens, M., Vanden-Heuvel, W.J.A. and Gale, P. (1976). Studies of a glycoprotein in the oocysts of Eimeria tenella. J. Biol. Chem. 251, 302.

73. Stotish, R.L., Profous-Juichelka, H. and Dulski, P.M. (1985). Isolation and in vitro translation of mRNA from Eimeria tenella. Fed. Proc. 44, 1334.

74. Svennerholm, A., Lange, S. and Holmgrin, J. (1978). Correlation between intestinal synthesis of specific immunoglobulin A and protection against experimental cholera in mice. Inf. Imm. 21, 1.

75. Towbin, H., T. Staehelin and J. Gordon. (1979). Electrophoretic transfer of proteins from polyacrylamide gels to nitrocellulose sheets : Procedure and some applications. Proc. Nat'1. Acad. Sci. 76:4350.

76. Ullrich, A.J., Shine, J., Chirgwin, J., Pictec, R., Tischer, E., Rutter, W.J. and Goodman, H.M. (1977). Rat insulin genes : Construction of plasmids containing the coding sequences. Science 196, 1313.

77. Van Deusen, R.A. and Whetstone, C.A. (1981). Practical aspects of producing anti-viral monoclonal antibodies as diagnostic reagents. Proc. Amer. Assn. Vet. Lab. Diagnost. 24, 211.

78. Viera, J. and Messing, J. (1982). The pUC plasmids, an M13mp7-derived system for insertion mutagenesis and sequencing with synthetic universal primers. Gene 19, 259-268.

79. Wang, C.C. Biochemistry and physiology of Coccidia. In The Biology of the Coccidia, Long, P.L., ed., (1982). University Park Press, Baltimore, pp. 167-228.

80. Wang, C.C. and Stotish, R.L. (1975). Changes in nucleic acids and proteins in the oocysts of Eimeria tenella during sporulation. J. Protozool. 22(3), 438.

81. Wisher, M.H. (1983). Sporozoite antigens of Coccidia. J. Cellular Biochem., Supp. 7A, Abstract 0059.

82. Wong, R.B. and Schenkel, R.H. (1984). Monoclonal antibodies analysis of Eimeria tenella sporozoite antigens. Fed. Proc. 184, 43(6), 1630.

83. Wright, I.G., White, M., Tracey-Patte, P.D., Donaldson, R.A., Goodger, B.V., Waltisbuhl, O.J. and Mahoney, D. F. (1983). Babesia bovis : Isolation of a protective antigen by using monoclonal antibody. Infection and Immunity 41, 244.

## Claims

**Claims for the following Contracting States : BE, CH, DE, FR, GB, GR, IT, LI, LU, NL, SE**

1. A purified antigenic protein capable of inducing in a chicken an immune response conferring protection against infection by Eimeria necatrix or Eimeria tenella, the protein having a molecular weight of about 26,000 daltons and being composed of two polypeptides joined by a disulfide bond, one of the polypeptides being characterized by a molecular weight of about 18,000 daltons and by a blocked N-terminal amino acid and having the amino acid sequence set forth in Figure 17 and the other of the polypeptides being characterized by a molecular weight of about 8,000 daltons and having the amino acid sequence set forth in Figure 17.

2. A method of preparing the protein or the 18,000 dalton polypeptide of claim 1 which comprises :

   a. contacting sporocysts of Eimeria necatrix with a detergent under suitable non-reducing conditions in the presence of protease inhibitors so as to solubilize the sporocyst membrane proteins ; and
   b. separately recovering the protein or the polypeptide from the solubilized, sporocyst membrane proteins under suitable non-reducing conditions.

3. A method of preparing the protein or the 18,000 dalton polypeptide of claim 1 which comprises preparing a DNA molecule coding for the protein or the polypeptide inserting the DNA molecule into an appropriate expression vector, introducing the resulting expression vector into a suitable host under appropriate conditions permitting expression of the DNA and production of the protein and recovering the protein or the polypeptide so produced.

4. A nucleic acid molecule encoding the protein of claim 1.

5. A recombinant cloning vector which comprises cloning vector DNA and the cDNA molecule of claim 4, the cloning vector DNA being characterized by the presence of a first and a second restriction enzyme site and the cDNA being cloned into said sites.

6. A host cell which comprises the cloning vector of claim 5.

7. A method of producing a protein capable of inducing in a chicken an immune response confering protection against infection by Eimeria necatrix or Eimeria tenella which comprises growing host cells of claim 6 under suitable conditions permitting production of the protein and recovering the protein so produced.

8. A method for obtaining the DNA of claim 4 which comprises isolating total genomic DNA from Eimeria necatrix oocysts ; preparing DNA fragments from the genomic DNA so isolated ; ligating the fragments so prepared into an appropriate cloning vector ; subjecting the DNA of the clones so prepared to hybridization with oligonucleotides containing, or complementary to, nucleic acid sequences present within the nucleic acid sequence set forth in Figure 17 to identify appropriate clones ; and isolating from the appropriate clones DNA which encodes the protein and has the nucleic acid sequence set forth in Figure 17.

9. An antigenic polypeptide which has the amino acid sequence of the 18,000 dalton polypeptide component of the antigenic protein of claim 1 and a molecular weight of 18,000 daltons and which is capable of inducing in a chicken an immune response conferring protection against infection by Eimeria necatrix and Eimeria tenella.

10. An antigenic polypeptide which has the amino acid sequence of the 8,000 dalton polypeptide component of the antigenic protein of claim 1 and a molecular weight of 8,000 daltons and which is capable of inducing in a chicken an immune response conferring protection against infection by Eimeria necatrix and Eimeria tenella.

11. A vaccine for conferring upon a chicken active immunity against Eimeria necatrix or Eimeria tenella which comprises per dose an effective immunizing amount of any one of the polypeptides of claims 1, 9 or 10 and a pharmaceutically acceptable carrier.


**Claims for the following Contracting States : AT, ES**

1. A method of preparing a purified antigenic protein capable of inducing in a chicken an immune response conferring protection against infection by Eimeria necatrix or Eimeria tenella, the protein having a molecular weight of about 26,000 daltons and being composed of two polypeptides joined by a disulfide bond, one of the polypeptides being characterized by a molecular weight of about 18,000 daltons and by a blocked N-terminal amino acid and having the amino acid sequence set forth in Figure 17, and the other of the polypeptides being characterized by a molecular weight of about 8,000 daltons, and having the amino acid sequence set forth in Figure 17, which comprises :

   a. contacting sporocysts of Eimeria necatrix with a detergent under suitable non-reducing conditions in the presence of protease inhibitors so as to solubilize the sporocyst membrane proteins ; and
   b. separately recovering the protein from the solubilized, sporocyst membrane proteins under suitable non-reducing conditions.

2. A method according to claim 1, wherein the separately recovering comprises partially purifying the solubilized sporocyst membrane proteins by DEAE HPLC chromatography followed by preparative SDS gel electrophoresis under suitable non-reducing conditions.

3. A method according to claim 1, wherein the separately recovering comprises immunoprecipition or immunoaffinity chromatography with monoclonal antibody Ptn 7.2A4/4 (ATCC No. HB8561).

4. A method of preparing the 18,000 dalton polypeptide component of claim 1 which comprises :

   a. contacting sporocysts of Eimeria necatrix with a detergent under suitable conditions in the presence of protease inhibitors so as to solubilize the sporocyst membrane proteins ; and
   b. separately recovering the polypeptide from the solubilized, sporocyst membrane proteins under suitable reducing conditions.

5. A method according to claim 4, wherein the separately recovering comprises partially purifying the solubilized sporocyst membrane proteins by chromatography on DEAE-HPLC followed by preparative SDS gel electrophoresis under suitable reducing conditions.

6. A method of preparing the protein and the 18,000 dalton polypeptide component of claim 1 which comprises preparing a DNA molecule coding for the protein or the polypeptide, inserting the DNA molecule into an appropriate expression vector, introducing the resulting expression vector into a suitable host under appropriate conditions permitting expression of the DNA and production of the protein or the polypeptide and recovering the protein so produced.

7. A method of producing a protein capable of inducing in a chicken an immune response conferring protection against infection by Eimeria necatrix or Eimeria tenella which comprises

   1. growing host cells under suitable conditions permitting production of the protein, this host cells comprising a recombinant cloning vector with cloning vector DNA and a cDNA molecule encoding the protein of claim 1 and
   2. recovering the protein so produced.

8. A method for obtaining the DNA encoding the protein of claim 1 and comprises the nucleic acid sequence set forth in figure 17 which comprises isolating total genomic DNA from Eimeria necatrix oocysts ; preparing DMA fragments from the genomic DNA so isolated ; ligating the fragments so prepared into an appropriate cloning vector ; subjecting the DNA of the clones so prepared to hybridization with oligonucleotides containing, or complementary to, nucleic acid sequences present within the nucleic acid sequence set forth in Figure 17 to identify appropriate clones ; and isolating from the appropriate clones DNA which encodes the protein and has the nucleic acid sequence set forth in Figure 17.

9. A method of preparing a vaccine for conferring upon a chicken active immunity against infection by Eimeria tenella and/or Eimeria necatrix which comprises per dose an effective immunizing amount of the protein of claim 1 and a pharmaceutically acceptable carrier.

**Patentansprüche**

**Patentansprüche für folgende Vertragsstaaten : BE, CH, DE, FR, GB, GR, IT, LI, LU, NL, SE**

1. Gereinigtes antigenes Protein, welches imstande ist, in einem Hühnchen eine Immunantwort zu induzieren, die Schutz gegen eine Infektion durch Eimeria necatrix oder Eimeria tenella verleiht, wobei das Protein ein Molekulargewicht von etwa 26 000 Dalton aufweist und aus zwei Polypeptiden zusammengesetzt ist, die durch eine Disulfidbindung verknüpft sind, wobei eines der Polypeptide durch ein Molekulargewicht von etwa 18 000 Dalton und durch eine blockierte N-terminale Aminosäure und dadurch, daß es die in Figur 17 angegebene Aminosäuresequenz besitzt, gekennzeichnet ist und das andere Polypeptid durch ein Molekulargewicht von etwa 8 000 Dalton und dadurch, daß es die in Figur 17 angegebene Aminosäuresequenz besitzt, gekennzeichnet ist.

2. Verfahren zur Herstellung des Proteins oder des 18 000 Dalton-Polypeptids nach Anspruch 1, welches umfaßt:

a) das Kontaktieren von Sporocysten von <u>Eimeria necatrix</u> mit einem Detergens unter geeigneten nicht-reduzierenden Bedingungen in Gegenwart von Proteaseinhibitoren, um die Sporocysten-Membranproteine zu solubilisieren; und

b) die separate Gewinnung des Proteins oder des Polypeptids aus den solubilisierten Sporocysten-Membranproteinen unter geeigneten nicht-reduzierenden Bedingungen.

3.  Verfahren zur Herstellung des Proteins oder des 18 000 Dalton-Polypeptids nach Anspruch 1, welches umfaßt die Herstellung eines DNA-Moleküls, das für das Protein oder das Polypeptid kodiert, die Insertion des DNA-Moleküls in einen geeigneten Expressionsvektor, die Einführung des resultierenden Expressionsvektors in einen geeigneten Wirt unter geeigneten Bedingungen, welche die Expression der DNA und die Produktion des Proteins erlauben, und die Gewinnung des so produzierten Proteins oder Polypeptids.

4.  Nukleinsäuremolekül, welches für das Protein nach Anspruch 1 kodiert.

5.  Rekombinanter Klonierungsvektor, welcher Klonierungsvektor-DNA und das CDNA-Molekül nach Anspruch 4 umfaßt, wobei die Klonierungsvektor-DNA durch die Anwesenheit einer ersten und einer zweiten Restriktionsenzymstelle gekennzeichnet ist und die cDNA in diese Stellen kloniert ist.

6.  Wirtszelle, welche den Klonierungsvektor nach Anspruch 5 umfaßt.

7.  Verfahren zur Herstellung eines Proteins, das imstande ist, in einem Hühnchen eine Immunantwort zu induzieren, die Schutz gegen eine Infektion durch <u>Eimeria necatrix</u> oder <u>Eimeria tenella</u> verleiht, welches umfaßt die Züchtung von Wirtszellen nach Anspruch 6 unter geeigneten Bedingungen, welche die Produktion des Proteins erlauben, und die Gewinnung des so produzierten Proteins.

8.  Verfahren zur Gewinnung der DNA nach Anspruch 4, welches umfaßt die Isolierung von genomischer Gesamt-DNA aus <u>Eimeria</u> <u>necatrix</u>-Oocysten; die Herstellung von DNA-Fragmenten aus der so isolierten genomischen DNA; die Ligierung der so hergestellten Fragmente in einen geeigneten Klonierungsvektor; das Unterwerfen der DNA der so hergestellten Klone einer Hybridisierung mit Oligonukleotiden, welche Nukleinsäuresequenzen, die innerhalb der in Figur 17 angegebenen Nukleinsäuresequenz vorhanden sind, enthalten oder dazu komplementär sind, um geeignete Klone zu identifizieren; und die Isolierung von DNA, die für das Protein kodiert und die in Figur 17 angegebene Nukleinsäuresequenz aufweist, aus den geeigneten Klonen.

9.  Antigenes Polypeptid, welches die Aminosäuresequenz der 18 000 Dalton-Polypeptid-Komponente des antigenen Proteins nach Anspruch 1 und ein Molekulargewicht von 18 000 Dalton aufweist und imstande ist, in einem Hühnchen eine Immunantwort zu induzieren, die Schutz gegen eine Infektion durch <u>Eimeria necatrix</u> und <u>Eimeria tenella</u> verleiht.

10. Antigenes Polypeptid, welches die Aminosäuresequenz der 8 000 Dalton-Polypeptid-Komponente des antigenen Proteins nach Anspruch 1 und ein Molekulargewicht von 8 000 Dalton aufweist und imstande ist, in einem Hühnchen eine Immunantwort zu induzieren, die Schutz gegen eine Infektion durch <u>Eimeria necatrix</u> und <u>Eimeria tenella</u> verleiht.

11. Impfstoff, um einem Hühnchen aktive Immunität gegen <u>Eimeria necatrix</u> oder <u>Eimeria tenella</u> zu verleihen, welcher pro Dosis eine effektiv immunisierende Menge irgendeines der Polypeptide der Ansprüche 1, 9 oder 10 und einen pharmazeutisch annehmbaren Träger umfaßt.

**Patentansprüche für folgende Vertragsstaaten : AT, ES**

1.  Verfahren zur Herstellung eines gereinigten antigenen Proteins, welches imstande ist, in einem Hühnchen eine Immunantwort zu induzieren, die Schutz gegen eine Infektion durch <u>Eimeria</u> <u>necatrix</u> oder <u>Eimeria</u> <u>tenella</u> verleiht, wobei das Protein ein Molekulargewicht von etwa 26 000 Dalton aufweist und aus zwei Polypeptiden zusammengesetzt ist, die durch eine Disulfidbindung verknüpft sind, wobei eines der Polypeptide durch ein Molekulargewicht von etwa 18 000 Dalton und durch eine blockierte N-terminale Aminosäure und dadurch, daß es die in Figur 17 angegebene Aminosäuresequenz besitzt, gekennzeichnet ist und das andere Polypeptid durch ein Molekulargewicht von etwa 8 000 Dalton und dadurch, daß es die in Figur 17 angegebene Aminosäuresequenz besitzt, gekennzeichnet ist, welches Verfahren umfaßt:

a) das Kontaktieren von Sporocysten von <u>Eimeria</u> <u>necatrix</u> mit einem Detergens unter geeigneten nicht-reduzierenden Bedingungen in Gegenwart von Proteaseinhibitoren, um die Sporocysten-Membranproteine zu solubilisieren; und
b) die separate Gewinnung des Proteins aus den solubilisierten Sporocysten-Membranproteinen unter geeigneten nicht-reduzierenden Bedingungen.

2. Verfahren nach Anspruch 1, worin die separate Gewinnung umfaßt die partielle Reinigung der solubilisierten Sporocysten-Membranproteine durch DEAE-HPLC-Chrömatographie, gefolgt von präparativer SDS-Gelelektrophorese unter geeigneten nicht-reduzierenden Bedingungen.

3. Verfahren nach Anspruch 1, worin die separate Gewinnung eine Immunpräzipitation oder Immunaffinitätschromatographie mit monoklonalem Antikörper Ptn 7.2A4/4 (ATCC Nr. HB8561) umfaßt.

4. Verfahren zur Herstellung der 18 000 Dalton-Polypeptid-Komponente von Anspruch 1 , welches umfaßt:

a) das Kontaktieren von Sporocysten von <u>Eimeria</u> <u>necatrix</u> mit einem Detergens unter geeigneten Bedingungen in Gegenwart von Proteaseinhibitoren, um die Sporocysten-Membranproteine zu solubilisieren; und
b) die separate Gewinnung des Polypeptids aus den solubilisierten Sporocysten-Membranproteinen unter geeigneten reduzierenden Bedingungen.

5. Verfahren nach Anspruch 4, worin die separate Gewinnung umfaßt die partielle Reinigung der solubilisierten Sporocysten-Membranproteine durch DEAE-HPLC-Chromatographie, gefolgt von präparativer SDS-Gelelektrophorese unter geeigneten reduzierenden Bedingungen.

6. Verfahren zur Herstellung des Proteins und der 18 000 Dalton-Polypeptid-Komponente von Anspruch 1, welches umfaßt die Herstellung eines DNA-Moleküls, das für das Protein oder das Polypeptid kodiert, die Insertion des DNA-Moleküls in einen geeigneten Expressionsvektor, die Einführung des resultierenden Expressionsvektors in einen geeigneten Wirt unter geeigneten Bedingungen, welche die Expression der DNA und die Produktion des Proteins oder des Polypeptids erlauben, und die Gewinnung des so produzierten Proteins.

7. Verfahren zur Herstellung eines Proteins, welches imstande ist, in einem Hühnchen eine Immunantwort zu induzieren, die Schutz gegen eine Infektion durch <u>Eimeria</u> <u>necatrix</u> oder <u>Eimeria</u> <u>tenella</u> verleiht, welches umfaßt

1. die Züchtung von Wirtszellen unter geeigneten Bedingungen, welche die Produktion des Proteins erlauben, wobei die Wirtszellen einen rekombinanten Klonierungsvektor mit Klonierungsvektor-DNA und einem cDNA-Molekül, das für das Protein von Anspruch 1 kodiert, umfassen, und
2. die Gewinnung des so produzierten Proteins.

8. Verfahren zur Gewinnung der DNA, die für das Protein von Anspruch 1 kodiert und die in Figur 17 angegebene Nukleinsäuresequenz umfaßt, welches umfaßt die Isolierung von genomischer Gesamt-DNA aus <u>Eimeria necatrix</u>-Oocysten; die Herstellung von DNA-Fragmenten aus der so isolierten genomischen DNA; die Ligierung der so hergestellten Fragmente in einen geeigneten Klonierungsvektor; das Unterwerfen der DNA der so hergestellten Klone einer Hybridisierung mit Oligonukleotiden, welche Nukleinsäuresequenzen, die innerhalb der in Figur 17 angegebenen Nukleinsäuresequenz vorhanden sind, enthalten oder dazu komplementär sind, um geeignete Klone zu identifizieren; und die Isolierung von DNA, die für das Protein kodiert und die in Figur 17 angegebene Nukleinsäuresequenz aufweist, aus den geeigneten Klonen.

9. Verfahren zur Herstellung eines Impfstoffs, um einem Hühnchen aktive Immunität gegen eine Infektion durch <u>Eimeria</u> <u>tenella</u> und/oder <u>Eimeria</u> <u>necatrix</u> zu verleihen, welcher pro Dosis eine effektiv immunisierende Menge des Proteins von Anspruch 1 und einen pharmazeutisch annehmbaren Träger umfaßt.

**Revendications**

**Revendications pour les Etats contractants suivants : BE, CH, DE, FR, GB, GR, IT, LI, LU, NL, SE**

1. Protéine antigénique purifiée capable d'induire, chez un poulet, une réponse immunitaire conférant une protection contre une infection par <u>Eimeria</u> <u>necatrix</u> ou <u>Eimeria</u> <u>tenella</u>, la protéine ayant un poids moléculaire d'environ 26 000 daltons et étant composée de deux polypeptides joints par un pont disulfure, l'un des polypeptides étant caractérisé par un poids moléculaire d'environ 18 000 daltons, par un acide aminé N-terminal bloqué et ayant la séquence d'acides aminés donnée à la Figure 17, et l'autre des polypeptides étant caractérisé par un poids moléculaire d'environ 8 000 daltons et ayant la séquence d'acides aminés donnée à la Figure 17.

2. Procédé de préparation de la protéine ou du polypeptide de 18 000 daltons de la revendication 1, qui comprend :

   a. la mise de sporocystes de <u>Eimeria</u> <u>necatrix</u> en contact avec un détergent dans des conditions non réductrices appropriées, en présence d'inhibiteurs de protéase de manière à solubiliser les protéines de membrane des sporocystes, et
   b. la récupération séparée de la protéine ou du polypeptide à partir des protéines de membrane des sporocystes, solubilisées, dans des conditions non réductrices appropriées.

3. Procédé de préparation de la protéine ou du polypeptide de 18 000 daltons de la revendication 1, qui comprend la préparation d'une molécule d'ADN codant pour la protéine ou le polypeptide, l'insertion de la molécule d'ADN dans un vecteur d'expression approprié, l'introduction du vecteur d'expression résultant dans un hôte approprié, dans des conditions appropriées permettant l'expression de l'ADN et la production de la protéine et la récupération de la protéine ou du polypeptide ainsi produit.

4. Molécule d'acide nucléique codant la protéine de la revendication 1.

5. Vecteur de clonage recombiné qui comprend un ADN vecteur de clonage et la molécule ADNc de la revendication 4, l'ADN vecteur de clonage étant caractérisé par la présence de premier et deuxième sites d'enzyme de restriction et l'ADNc étant cloné dans les sites.

6. Cellule-hôte qui comprend le vecteur de clonage de la revendication 5.

7. Procédé de production d'une protéine capable d'induire, chez un poulet, une réponse immunitaire conférant une protection contre une infection par <u>Eimeria</u> <u>necatrix</u> ou <u>Eimeria</u> <u>tenella</u>, qui comprend la culture des cellules-hôtes de la revendication 6, dans des conditions appropriées permettant la production de la protéine et la récupération de la protéine ainsi produite.

8. Procédé d'obtention de l'ADN de la revendication 4, qui comprend l'isolement de l'ADN génomique total des oocystes de <u>Eimeria</u> <u>necatrix</u>; la préparation de fragments d'ADN à partir de l'ADN génomique ainsi isolé; la ligature des fragments ainsi préparés dans un vecteur de clonage approprié; la réalisation d'une hybridation de l'ADN des clones ainsi préparés avec des oligonucléotides contenant les, ou complémentaires aux, séquences d'acide nucléique présentes dans la séquence d'acide nucléique donnée à la Figure 17, afin d'identifier les clones appropriés, et l'isolement à partir des clones appropriés, de l'ADN qui code la protéine et qui a la séquence d'acide nucléique donnée à la Figure 17.

9. Polypeptide antigénique qui a la séquence d'acides aminés du composant polypeptidique de 18 000 daltons de la protéine antigénique de la revendication 1 et un poids moléculaire de 18 000 daltons et qui est capable d'induire, chez un poulet, une réponse immunitaire conférant une protection contre une infection par <u>Eimeria</u> <u>necatrix</u> ou <u>Eimeria</u> <u>tenella</u>.

10. Polypeptide antigénique qui a la séquence d'acides aminés du composant polypeptidique de 8 000 daltons de la protéine antigénique de la revendication et un poids moléculaire de 8 000 daltons et qui est capable d'induire, chez un poulet, une réponse immunitaire conférant une protection contre une infection par <u>Eimeria</u> <u>necatrix</u> ou <u>Eimeria</u> <u>tenella</u>.

11. Vaccin pour conférer, à un poulet, une immunité active contre <u>Eimeria</u> <u>necatrix</u> ou <u>Eimeria</u> <u>tenella</u>, qui comprend, par dose, une quantité efficace pour l'immunisation de l'un des polypeptides des revendications 1, 9 ou 10 et un excipient pharmaceutiquement acceptable.

**Revendications pour les Etats contractants suivants : ES, AT**

1. Procédé de préparation d'une protéine antigénique purifiée capable d'induire, chez un poulet, une réponse immunitaire conférant une protection contre une infection par Eimeria necatrix ou Eimeria tenella, la protéine ayant un poids moléculaire d'environ 26 000 daltons et étant composée de deux polypeptides joints par un pont disulfure, l'un des polypeptides étant caractérisé par un poids moléculaire d'environ 18 000 daltons, par un acide aminé N-terminal bloqué et ayant la séquence d'acides aminés donnée à la Figure 17, et l'autre des polypeptides étant caractérisé par un poids moléculaire d'environ 8 000 daltons et ayant la séquence d'acides aminés donnée à la Figure 17, qui comprend :

   a. la mise de sporocystes de Eimeria necatrix en contact avec un détergent dans des conditions non réductrices appropriées, en présence d'inhibiteurs de protéase de manière à solubiliser les protéines de membrane des sporocystes, et
   b. la récupération séparée de la protéine à partir des protéines de membrane des sporocystes, solubilisées, dans des conditions non réductrices appropriées.

2. Procédé suivant la revendication 1, dans lequel la récupération séparée comprend la purification partielle des protéines de membrane des sporocystes, solubilisées par chromatographie DEAE-HPLC, puis par électrophorèse préparative sur gel SDS dans des conditions non réductrices appropriées.

3. Procédé suivant la revendication 1, dans lequel la récupération séparée comprend une immunoprécipitation ou une chromatographie d'immunoaffinité avec l'anticorps monoclonal Ptn 7.2A4/4 (ATCC n° HB8561).

4. Procédé de préparation du composant polypeptidique de 18 000 daltons suivant la revendication 1, qui comprend :

   a. la mise de sporocystes de Eimeria necatrix en contact avec un détergent dans des conditions appropriées, en présence d'inhibiteurs de protéase de manière à solubiliser les protéines de membrane des sporocystes, et
   b. la récupération séparée des polypeptides à partir des protéines de membrane des sporocystes, solubilisées, dans des conditions réductrices appropriées.

5. Procédé suivant la revendication 4, dans lequel la récupération séparée comprend la purification partielle des protéines de membrane des sporocystes, solubilisées par chromatographie sur DEAE-HPLC, puis par électrophorèse préparative sur gel SDS dans des conditions réductrices appropriées.

6. Procédé de préparation de la protéine et du composant polypeptidique de 18000 daltons suivant la revendication 1, qui comprend la préparation d'une molécule d'ADN codant pour la protéine ou le polypeptide, l'insertion de la molécule d'ADN dans un vecteur d'expression approprié, l'introduction du vecteur d'expression résultant dans un hôte approprié, dans des conditions appropriées permettant l'expression de l'ADN et la production de la protéine ou du polypeptide et la récupération de la protéine ainsi produite.

7. Procédé de production d'une protéine capable d'induire, chez un poulet, une réponse immunitaire conférant une protection contre une infection par Eimeria necatrix ou Eimeria tenella, qui comprend :

   1. la culture de cellules-hôtes dans des conditions appropriées permettant la production de la protéine, ces cellules-hôtes comprenant un vecteur de clonage recombiné avec un ADN vecteur de clonage et une molécule d'ADNc codant la protéine de la revendication 1, et
   2. la récupération de la protéine ainsi produite.

8. Procédé d'obtention de l'ADN codant la protéine de la revendication 1 et comprenant la séquence d'acide nucléique donnée à la Figure 17, qui comprend l'isolement de l'ADN génomique total des oocystes de Eimeria necatrix ; la préparation de fragments d'ADN à partir de l'ADN génomique ainsi isolé; la ligature des fragments ainsi préparés dans un vecteur de clonage approprié; la réalisation d'une hybridation de l'ADN des clones ainsi préparés avec des oligonucléotides contenant les, ou complémentaires aux, séquences d'acide nucléique présentes dans la séquence d'acide nucléique donnée à la Figure 17, afin d'identifier les clones appropriés, et l'isolement à partir des clones appropriés, de l'ADN qui code la protéine et qui a la séquence d'acide nucléique donnée à la Figure 17.

9. Procédé de préparation d'un vaccin pour conférer, à un poulet, une immunité active contre une infection par Eimeria tenella et/ou Eimeria necatrix, qui comprend, par dose, une quantité de la protéine de la revendication 1 efficace pour l'immunisation et un excipient pharmaceutiquement acceptable.

Figure 1:  Amino Acid Sequence of the 17,000 Dalton
Polypeptide Component of the TA4 Antigen

```
          [gin]asp tyr pro thr ala val thr leu asp(cys)lys(glu)ala
              |-----------------PAP---------------------->
                                             |------CH3----------
                                                              |V7


met asn lys leu arg lys ala ala gly leu pro ala phe glu asp ala val gly
----------------------------------CH3--------------------------------------
----------------------------------V7---------------------------------------
     |----------------------------------CN1------------------------------


asp thr phe val leu pro ala tyr(ser his)glu glu ser arg ala ala pro val
--CH3-----| |-----------------------CH2'--------------------------------
-------V7----------------->                    |--------V6------------
-----------------------------CN1------------------------------------------
                                               |----R2---------


ala glu thr leu trp lys thr glu ile cys pro lys val leu gly gly gly arg
---CH2'--->                      |------------------V4---------------------
-V6---| |-----------------------------------------V2-----------------------
---------------------------------------R2---------------------------------
                                             |------CH5-----


ser arg asn val thr glu ala val lys leu thr gly asn phe ala tyr tyr pro
---------V4-----------| |----------------------V5---------------------
-------V2------------| |---------------------V1----------------------
--R2--|
-----------------------CH5------------------------>
|-----------------------------R1----------------------------------


val thr asp gly lys lys glu cys ser asp ala val glu tyr trp lys gly gly
---------V5----------------|                      |---CH2----
----------------------------V1--------------------------------------
----------------R1----------------------------->      |--R4--


leu ser gln phe asn asp thr ile pro pro thr phe gln ala leu asn asp pro
-----------------------------CH2-----------------------------------)
-----------V1--------------->
----------------------------R4-----------------------------------
                                                      |---R2'----


val val tyr asn asp arg ala val ser phe val ala leu tyr asn pro lys thr
--------R4----------------------->       |-----------CH1-----------------
              |---------CH4------------|
-----------------R2'------------------------------------------->


ser pro val val ser(cys)val leu leu gln(cys)pro asn ala gly val gly gly
-------------------------------CH1----------------------------------|
```

> indicates peptide sequence may continue, but too weak to follow
| indicates peptide C-terminus
() probable amino acids confirmed by DNA sequence
' indicates secondary sequence
CN: cyanogen bromide fragment; CH: chymotrypsin fragment; R: Arg-C
fragment; V: V8 fragment; PAP: pyroglutamate aminopeptidase treated
17kd protein.

Figure 2.    Restriction Map of the 5.5 kb Insert of Clone 108-1.

PstI and BalI sites shown are the rightmost sites, but not the only sites.

No sites for BamHI, EcoRV, NruI, SalI, SmaI and PvuI.

EP 0 453 054 B1

Figure 3a

```
                                          AGATCTATCAAGCAATAATCATCTA
CCTCCAAATATATGCTATGAAATGC7AAATT8CGTGAGAGTGATTCGTCACAGCAACGTC
TCATGCAGAGTGCCCGAGAACTGA5GGGAGAAACAGTGGAGTGACCGCGGGTCGCTGGTA
TTTTCTTGCTTTCATTOGCAAACGYGGCATTTTCAAGTGCCATTTTTCTTGTAATCACAT
TAGTTTGCCAGTAAATGAGGGGAATATTCTGGTGTAAGCTGTTCTTCTGGCAGTTTCACG
AGAGTCACACCGTCACCTGGOAGGTAACCTGGAAAGGGGCGGTGGCAGGAATGGCGCAAG
GCATGGAACAATGAAAGCTGAGAGCAGCGTCAAA3GGATGAATTTTCAATTTCACGTTTG
CCCTTAAATCCATTCAAGTGGGCCGAGACCGCTCTCGGAAGYGCAGTCTCGTTTGCGATT
GCATTMCCTGCACACACCTATGACGACGTACGGTGTTGGGCAGAACCTGAACATAGCGTT
TACGTCTAMAGCCGCAGCCCAAAGAAACTCTGCATACTTTTGCCAAGATATTTCAAATAA
AACCTCTTTGCCGAATTGTATTTTCACCCTCTATCTACTATTTCCTGCCCACTATGAGAG
GCAGCAAGC7GTAGCGTGCCTTCCAATGGCCAGCACCAGCGCGCCAG7TAGGGCAGCAGC
TGTCAACCTCGCTGTCATCTGTCAACAGGCCGCCAGAACTCTTCCCATATCTGTCAAAAC
ATATTTATCTGCTCACTTTACAGTTTCTGTACAGTCACTTTTGCATATTATACAATTACT

                            MetAlaArgLeuSerPheValSerLeuLeuSerLeuSer
GTACAGTCATATTTGCTCAAAATGGCTCGTCTTTCTTTTGTTTCTCTTCTTTCTCTGTCA
                                        •
LeuLeuPheGlyGlnGlnAlaValArgAlaGlnAspTyrProThrAlaV<----------
CTGCTCTTCGGGCAGCAAGCAGTCAGAGCTCAGGATTACCCAACAGCAGGTGGGCTTTTC

--------------------Intron A-------------------------------
CGCTAGCTGTTTTTGGTCCGATAGCATCGGAGCATCTCCCAAAACGAGGTGCATTCACC

-------------------------------->alThrLeuAspCysLysGluAlaMetAsn
TTTTGCATGTTGTGTGCGGAAATTTTATCAGTTACGCTGGACTGTAAAGAAGCGATGAAC

LysLeuArgLysAlaAlaGlyLeuProAlaPheGluAspAlaValGlyAspThrPheVal
AAGCTGAGAAAAGCAGCAGGACTTCCTGCATTCGAAGATGCTGTGGGAGACACATTTGTT

LeuProAlaTyrSerHisGluGluSerArgAlaAlaProValAlaGluThrLeuTrpLys
CTACCAGCATACTCGCATGAAGAGTCTAGGGCGGCACCAGTAGCTGAAACTCTCTGGAAG

ThrGluIleCysProLysValLeuGly<--------------------------------
ACGGAGATATGCCCCAAAGTCTTAGGAGTAAGCCGTCCACGGCCTTGCATCGTCATGATG
```

FIG 3

| FIG 3a | FIG 3b | FIG 3c |

Figure 3b

```
-----------------Intron B-------------------------------------
TAGTAGGTGTTCTGAGCAGCTTCGTTCTGTGGAACAAGGAACTACACTGTCCTTGAATTT


-------------------->GlyGlyArgSerArgAsnValThrGluAlaValLysLeu
TTAATCTTTTGTTACGTACAGGGCGGAAGGTCCAGGAACGTTACTGAAGCTGTCAAGTTA

ThrGlyAsnPheAlaTyrTyrProValThrAspGlyLysLysGluCysSerAspAlaVal
ACTGGCAATTTTGCCTACTACCCCGTCACAGACGGCAAAAAAGAGTGCAGCGATGCTGTG

GluTyrTrpLysGlyGlyLeuSerGlnPheAsnAspThrIleProProThrPheGlnAla
GAGTACTGGAAAGGCGGACTTTCTCAGTTCAACGACACAATTCCCCCAACGTTCCAAGCG

LeuAsnAspProValValTyrAsnAspArgAlaValSerPheValAlaLeuTyrAsnPro
TTGAACGACCCCGTTGTGTACAATGACAGGGCTGTTTCCTTTGTCGCCCTATACAACCCC
                                                          ••

LysThrSerProValValSerCysValLeuLeuGlnCysProAsnAlaGlyValGlyGly
AAAACCAGCCCCGTTGTCAGTTGCGTGCTCCTCCAGTGCCCTAATGCAGGTGTTGGTGGA

             ◆
ArgArgLeuAlaAlaGlyThrThrAspAlaValIleCysLeuThrAsnProAlaProLeu
CGCAGGCTTGCGGCAGGCACGACAGACGCTGTCATTTGCTTGACAAATCCGGCTCCTTTG

GluAlaArgSerGlnProPheAs<-------------------------------------
GAAGCAAGGTCACAACCATTCGAGTGAGAGTCAGCTGGTCGCCACTGCAACATGCATCAA

----------------------Intron C--------------------------------
TGCGGCAGGTTACACTGGGGGGTCTTGAGGTTGGTTGAAGCGCAATCTTCTAATACTTGTT

--------------------------->pAspGluGlnTrpLysLysIleValAspSerLe
TGTAATGTTTGTAATGTTTGCGTGCAGCGACGAGCAATGGAAGAAAATTGTTGACTTTTT

uSerLeuSerGluGluGluGluGluLysGlyGlyValSerProValValProSerValAl
ATCTCTCTCTGAGGAAGAGGAAGAGAAGGGCGGAGTTTCTTCAGTCGTCCCTTCAGTAGC

                                                        •◆
aLeuIleSerAlaAlaValIleSerAlaPheAlaLeuPhe
CCTCATCTCTGCGGCGGTCATCTCCGCTTTCGCTCTCTTTTAGGCGGGCGCCGGTTGTTA

GTGACACACCAGCATTGGACAGATATGGCGGCGCAAGTTCCTTCCTGAGTGAAATCCTTG

AGTGACAAACGAGCACCTCTCCTGGACGAAATGTGATGAATTAAGACAGCTTTGGTTGTT

TGAAGTGTATGCAAAAGCTACATTTGTAGGGCCCTTTTATAGGATAATCGGAGGAAGCGC

AATTTTATTTAAAACCCTTGCAGAGAGTCGCCACGTGCGAGTGCAAGTGTTGCGCAGTGT

GTGCTGCCAAATGAAATTCTCGATCTTTAGTGTACTCAAGCCAGAAGTTTCGGCGTTGAT

GTACCCGCCGGTGGTATCTGCCATGCCATGCCTGCCTGTTTGGGCAGTACAACCTCATAC

CAAGTGGCTTGTGTCATGGCATGTGTGGCCAAGCTACTTTTAGAGGGACAACAATGGGGA

TATTTTGAAGTATTTCGGATAAATACTCATCTGCTGTCCCTACCCACTGAGGCGCCATGG
```

Figure 3c

TGTTACCTTCCTCATTTGAAGGGGAAAACTTGGTTGATAATTTCTTGTCCTTCAACTTGT

CTTGATAAATCGAAGATTATATTGTAGATAGTATACGTGGTGAACAGTTTTTAGGGAAGA

CTGTAAACCACAAGTTAAACGTAGTCGGAATTC

Legend

* Initial amino acid of 17,000 Dalton peptide
** Final amino acid of 17,000 Dalton peptide
+ Initial amino acid of 8,000 Dalton peptide
++ Final amino acid of 8,000 Dalton peptide

Key to ambiguous bases

3 = Probably C
4 = Probably T
5 = Probably A
6 = Probably G
7 = Maybe C
8 = Maybe T
9 = Maybe A
0 = Maybe G

R = A or G
Y = C or T
J = C or A
K = T or G
L = T or A
M = C or G

Figure 4    Appearance of the TA4 Antigen During Sporulation

— 200 Kd

— 97

— 69

— 46

— 25.7

— 18.4

— 12.3

0   4   8   12   16   20   24   25   40   SPOR

time (hours)

EP 0 453 054 B1

Figure 5a

```
                                    AGATCTATCAAGCAATAATCATCTA
CCTCCAAATATATGCTATGAAATGC7AAATT8CGTGAGAGTGATTCGTCACAGCAACGTC
TCATGCAGAGTGCCCGAGAACTGA5GGGAGAAACAGTGGAGTGACCGCGGGTCGCTGGTA
TTTTCTTGCTTTCATTOGCAAACGYGGCATTTTCAAGTGCCATTTTTCTTGTAATCACAT
TAGTTTGCCAGTAAATGAGGGGAATATTCTGGTGTAAGCTGTTCTTCTGGCAGTTTCACG
AGAGTCACACCGTCACCTGGOAGGTAACCTGGAAAGGGGCGGTGGCAGGAATGGCGCAAG
GCATGGAACAATGAAAGCTGAGAGCAGCGTCAAA3GGATGAATTTTCAATTTCACGTTTG
CCCTTAAATCCATTCAAGTGGGCCGAGACCGCTCTCGGAAGYGCAGTCTCGTTTGCGATT
GCATTMCCTGCACACACCTATGACGACGTACGGTGTTGGGCAGAACCTGAACATAGCGTT
TACGTCTAMAGCCGCAGCCCAAAGAAACTCTGCATACTTTTGCCAAGATATTTCAAATAA
AACCTCTTTGCCGAATTGTATTTTCACCCTCTATCTACTATTTCCTGCCCACTATGAGAG
GCAGCAAGC7GTAGCGTGCCTTCCAATGGCCAGCACCAGCGCGCCAG7TAGGGCAGCAGC
TGTCAACCTCGCTGTCATCTGTCAACAGGCCGCCAGAACTCTTCCCATATCTGTCAAAAC
ATATTTATCTGCTCACTTTACAGTTTCTGTACAGTCACTTTTGCATATTATACAATTACT

                    MetAlaArgLeuSerPheValSerLeuLeuSerLeuSer
GTACAGTCATATTTGCTCAAAATGGCTCGTCTTTCTTTTGTTTCTCTTCTTTCTCTGTCA
                                                •

LeuLeuPheGlyGlnGlnAlaValArgAlaGlnAspTyrProThrAlaV<----------
CTGCTCTTCGGGCAGCAAGCAGTCAGAGCTCAGGATTACCCAACAGCAGGTGGGCTTTTC
                                 SacI

-----------------Intron A-----------------------------------------
CGCTAGCTGTTTTTGGTCCGATAGCATCGGAGCATCTCCCAAAACGAGGTGCATTCACC

------------------------------------>alThrLeuAspCysLysGluAlaMetAsn
TTTTGCATGTTGTGTGCGGAAATTTTTATCAGTTACGCTGGACTGTAAAGAAGCGATGAAC

LysLeuArgLysAlaAlaGlyLeuProAlaPheGluAspAlaValGlyAspThrPheVal
AAGCTGAGAAAAGCAGCAGGACTTCCTGCATTCGAAGATGCTGTGGGAGACACATTTGTT

LeuProAlaTyrSerHisGluGluSerArgAlaAlaProValAlaGluThrLeuTrpLys
CTACCAGCATACTCGCATGAAGAGTCTAGGGCGGCACCAGTAGCTGAAACTCTCTGGAAG

ThrGluIleCysProLysValLeuGly<-------------------------------------
ACGGAGATATGCCCCAAAGTCTTAGGAGTAAGCCGTCCACGGCCTTGCATCGTCATGATG
```

| | | | |
|---|---|---|---|
| FIG 5 | FIG 5a | FIG 5b | FIG 5c |

44

Figure 5b

```
-------------------Intron B--------------------------------
TAGTAGGTGTTCTGAGCAGCTTCGTTCTGTGGAACAAGGAACTACACTGTCCTTGAATTT

-------------------->GlyGlyArgSerArgAsnValThrGluAlaValLysLeu
TTAATCTTTTGTTACGTACAGGGCGGAAGGTCCAGGAACGTTACTGAAGCTGTCAAGTTA

ThrGlyAsnPheAlaTyrTyrProValThrAspGlyLysLysGluCysSerAspAlaVal
ACTGGCAATTTTGCCTACTACCCCGTCACAGACGGCAAAAAAGAGTGCAGCGATGCTGTG

GluTyrTrpLysGlyGlyLeuSerGlnPheAsnAspThrIleProProThrPheGlnAla
GAGTACTGGAAAGGCGGACTTTCTCAGTTCAACGACACAATTCCCCCAACGTTCCAAGCG

LeuAsnAspProValValTyrAsnAspArgAlaValSerPheValAlaLeuTyrAsnPro
TTGAACGACCCCGTTGTGTACAATGACAGGGCTGTTTCCTTTGTCGCCCTATACAACCCC

                                                          ••
LysThrSerProValValSerCysValLeuLeuGlnCysProAsnAlaGlyValGlyGly
AAAACCAGCCCCGTTGTCAGTTGCGTGCTCCTCCAGTGCCCTAATGCAGGTGTTGGTGGA

              •
ArgArgLeuAlaAlaGlyThrThrAspAlaValIleCysLeuThrAsnProAlaProLeu
CGCAGGCTTGCGGCAGGCACGACAGACGCTGTCATTTGCTTGACAAATCCGGCTCCTTTG

GluAlaArgSerGlnProPheAs<---------------------------------
GAAGCAAGGTCACAACCATTCGAGTGAGAGTCAGCTGGTCGCCACTGCAACATGCATCAA
                               PvuII

---------------------Intron C------------------------------
TGCGGCAGGTTACACTGGGGGGTC7TGAGGTTGGTTGAAGCGCAATCTTCTAATACTTGTT

------------------------->pAspGluGlnTrpLysLysIleValAspSerLe
TGTAATGTTTGTAATGTTTGCGTGCAGCGACGAGCAATGGAAGAAAATTGTTGACTCTCT

uSerLeuSerGluGluGluGluGluLysGlyGlyValSerProValValProSerValAl
ATCTCTCTCTGAGGAAGAGGAAGAGAAGGGCGGAGTTTCTCCAGTCGTCCCTTCAGTAGC

                                                      ••
aLeuIleSerAlaAlaValIleSerAlaPheAlaLeuPhe
CCTCATCTCTGCGGCGGTCATCTCCGCTTTCGCTCTCTTTTAGGCGGGGCGCCGGTTGTTA

GTGACACACCAGCATTGGACAGATATGGCGGCGCAAGTTCCTTCCTGAGTGAAATCCTTG

AGTGACAAACGAGCACCTCTCCTGGACGAAATGTGATGAATTAAGACAGCTTTGGTTGTT

TGAAGTGTATGCAAAAGCTACATTTGTAGGGCCCTTTTATAGGATAATCGGAGGAAGCGC

AATTTTATTTAAAACCCTTGCAGAGAGTCGCCACGTGCGAGTGCAAGTGTTGCGCAGTGT

GTGCTGCCAAATGAAATTCTCGATCTTTAGTGTACTCAAGCCAGAAGTTTCGGCGTTGAT

GTACCCGCCGGTGGTATCTGCCATGCCATGCCTGCCTGTTTGGGCAGTACAACCTCATAC
```

Figure 5c


CAAGTGGCTTGTGTCATGGCATGTGTGGCCAAGCTACTTTTAGAGGGACAACAATGGGGA

TATTTTGAAGTATTTCGGATAAATACTCATCTGCTGTCCCTACCCACTGAGGCGCCATGG

TGTTACCTTCCTCATTTGAAGGGGAAAACTTGGTTGATAATTTCTTGTCCTTCAACTTGT

CTTGATAAATCGAAGATTATATTGTAGATAGTATACGTGGTGAACAGTTTTTAGGGAAGA

CTGTAAACCACAAGTTAAACGTAGTCGGAATTC


**Legend**

- • Initial amino acid of 17,000 dalton peptide
- •• Final amino acid of 17,000 dalton peptide
- • Initial amino acid of 8,000 dalton peptide
- •• Final amino acid of 8,000 dalton peptide

**Key to ambiguous bases**

    3 = Probably C
    5 = Probably A
    7 = Maybe C
    8 = Maybe T
    0 = Maybe G
    Y = C or T
    M = C or G

Figure 6

Occurrence of the TA4 Antigen mRNA During Sporulation

**4   8  12 16 20 24    36**

**Hours  of  Sporulation**

Figure 7

DNA and Predicted Amino Acid Sequence of cDNA Clone pTCD26.

```
    GlnAspTyrProThrAlaValThrLeuAspCysLysGluAlaMetAsnLys
GAGCTCAGGATTACCCAACAGCAGTTACGCTGGACTGTAAAGAAGCGATGAACAAG
  SacI

 LeuArgLysAlaAlaGlyLeuProAlaPheGluAspAlaValGlyAspThrPheValLeu
CTGAGAAAAGCAGCAGGACTTCCTGCATTCGAAGATGCTGTGGGAGACACATTTGTTCTA

 ProAlaTyrSerHisGluGluSerArgAlaAlaProValAlaGluThrLeuTrpLysThr
CCAGCATACTCGCATGAAGAGTCTAGGGCGGCACCAGTAGCTGAAACTCTCTGGAAGACG

 GluIleCysProLysValLeuGlyGlyGlyArgSerArgAsnValThrGluAlaValLys
GAGATATGCCCCAAAGTCTTAGGAGGCGGAAGGTCCAGGAACGTTACTGAAGCTGTCAAG

 LeuThrGlyAsnPheAlaTyrTyrProValThrAspGlyLysLysGluCysSerAspAla
TTAACTGGCAATTTTGCCTACTACCCCGTCACAGACGGCAAAAAAGAGTGCAGCGATGCT

 ValGluTyrTrpLysGlyGlyLeuSerGlnPheAsnAspThrIleProProThrPheGln
GTGGAGTACTGGAAAGGCGGACTTTCTCAGTTCAACGACACAATTCCCCCAACGTTCCAA

 AlaLeuAsnAspProValValTyrAsnAspArgAlaValSerPheValAlaLeuTyrAsn
GCGTTGAACGACCCCGTTGTGTACAATGACAGGGCTGTTTCCTTTGTCGCCCTATACAAC

 ProLysThrSerProValValSerCysValLeuLeuGlnCysProAsnAlaGlyValGly
CCCAAAACCAGCCCCGTTGTCAGTTGCGTGCTCCTCCAGTGCCCTAATGCAGGTGTTGGT

 GlyArgArgLeuAlaAlaGlyThrThrAspAlaValIleCysLeuThrAsnProAlaPro
GGACGCAGGCTTGCGGCAGGCACGACAGACGCTGTCATTTGCTTGACAAATCCGGCTCCT

 LeuGluAlaArgSerGlnProPheAspAspGluGlnTrpLysLysIleValAspSerLeu
TTGGAAGCAAGGTCACAACCATTCGACGACGAGCAATGGAAGAAAATTGTTGACTCTCTA

 SerLeuSerGluGluGluGluGluLysGlyGlyValSerProValValProSerValAla
TCTCTCTCTGAGGAAGAGGAAGAGAAGGGCGGAGTTTCTCCAGTCGTCCCTTCAGTAGCC

 LeuIleSerAlaAlaValIleSerAlaPheAlaLeuPheAM
CTCATCTCTGCGGCGGTCATCTCCGCTTTCGCTCTCTTTTAGGCGGGCGCCGGTTGTTAG

TGACACACCAGCATTGGACAGATATGGCGGCGCAAGTTCCTTCCTGAGTGAAATCCTTGA

GTGACAAACGAGCACCTCTCCTGGACGAAATGTGATGAATTAAGACAGCTTTGGTTGTTT

GAAGTGTATGCAAAAGCTACATTTGTAGGGCCCTTTTATAGGATAATCGGAGGAAGCGCA

ATTTTATTTAAAACCCTTGCAGAGAGTCGCCACGTGCGAGTGCAAGTGTTGCGCAGTGTG

TGCTGCCAAATGAAATTCTCGATCTTTAGTGTACTCAAGCCAGAAGTTTCGGCGTTGATG

TACCCGCCGGTGGTATCTGCCATGCCATGCCTGCCTGTTTGGGCAGTACAACCTCATACC

AAGTGGCTTGTGTCATGGCATGTGTGGCCAAGCTACTTTTAGAGGGACAACAATGGGGAT

ATTTTGAAGTATTTCGGATAAATACTCATCTGCTGTCCCTACCCACTGAGGCGCCATGGT

GTTACCTTCCTCATTTGAAGGGGAAAACTTGGTTGATAATTTCTTGTCCTTCAAAAAAAA

AAAAAAAAAAA
```

Figure 3

Figure 9

Analysis of pDET1 and pDET2 proteins

A. Stained gel, pDET1
B. Immunoblot, pDET1
C. Stained gel, pDET2

Figure 10

Figure 11

Analysis of pBGC23 protein

A. Stained gel

B. Immunoblot

Figure 12

Figure 13

Figure 14

Analysis of pCOCl2 and pCOC20 proteins

A. Stained gel, pCOCl2
B. Immunoblot, pCOCl2
C. Stained gel, pCOC20

Figure 15

Immunoreactivity (ELISA) of TA4 antigen and renatured TA4 proteins with monoclonal antibody Ptn 7.2 A4/4

Figure 16    Restriction Map of the 3.9 Kb Insert of E. Necatrix clone 7.

## Figure 17a

DNA sequence of the E. necatrix genomic clone encoding the NA4 antigen

```
CTGCTTCATGCAACGCCACATTTTCAAGCTTCACTTTTCTGATACTCACATTATTTTGCCAGCAAGAGA3GGATA

TGTTCGGTGTAAGCTGJTCTTCTGGCAGTTTCATGAGAGTGACAGCGTCACCTGGTGGTAACCTGCGCTGGGGGC

GGCGGCAGGAATGGCGCAAGGCGTGGAACAATGAACGCTGACAGGCAGCGTCAAAGAGATGAATTTTCAATTTCA

CTTTTGCCATTAAATCCATTCAAGTGGGCCGAGACCGCTTTCTGGAGTGCAGTCTCGTTTGCGTTGGCATTCCCT

GCACACACCTGATGATGACGTAGGGTGTTGCGCAGAACCTGAATATAGCGTTTAGGTCTAGAGCCGCAGCCCTAC

TAAATCTGCACATTCTTGCATGATATTTCAAATAAAACTCTTGCGAAATTATATTTTCACTTTCTATCTACTATT

TGCTGCCCACTATGCGAGGCAGCAAGCCGTAGCGTGCTTCCAATCGCCAGCACCGGCGCGCCAGCTAGGGCAGCA

GCTGTCAACCTCGCTGTCATCTGTCGACAGCCGCCACAACTCTTTTCATATCTGTCAAAACATATTTATCTGCAT

                                                             MetAlaArgLeu
TTTACAGTTTCTGTACAGTCATTTTTGCATTTTTATAGTTACTGTACAGTCATATTTGCTCAAAATGGCTCGTCTC
                                                              *
SerPheValSerLeuLeuSerLeuSerLeuLeuPheGlyGlnGlnAlaAlaArgAlaGlnGluThrTyrProThr
TCTTTTGTTTCTCTTCTTTCTCTGTCACTGCTCTTCGGGCAGCAAGCAGCCAGAGCTCAGGAAACATACCCAACA

AlaG|————————————————Intron A———————————————————————————————————————————————
GCAGGTGGGCTTTTCCGCTAGCCGTTTTTTGGTCTGACAGCATCTGAGTACTTCCCAAAACAGCGTGCATTCTTCT

——————————————————————————|luThrMetGluCysArgGluAlaMetAsnGluLeuArgLysAlaA
TTTGCATGTTGTGTGCGGAAATTTTATCAGAAACGATGGAGTGTAGAGAGGCGATGAACGAGCTCAGAAAAGCAG

laGlyLeuProGluPheGlyAsnAlaValGlyAspAlaValValLeuProAlaTyrSerHisGluAlaArgAlaA
CAGGGCTTCCTGAATTTGGAAATGCTGTTGGAGATGCAGTAGTTCTACCAGCATACTCGCACGAGGCCAGGGCGG

laProValAlaGluThrLeuTrpLysThrGluIleCysProLysValLeuGly|————————————————————
CACCAGTGGCTGAAACTCTGTGGAAGACGGAAATATGTCCCAAAGTCTTAGGAGTAAGCCGTCCTCTGCATTGTA

————————————————————Intron B—————————————————————————————————————————————
GTCGTCCACTGCATTGTCATGTAGCAGGTGTTCTGAGCAGCTTATCTCTTTAAACAAGGAACTACGCCCTCCTCA

——————————————————————————|GlyAlaArgAlaLysSerValThrGluAlaValLysLeuThrGlyAsnPh
ATTTCTAATCTTTCGCTGCGTACAGGGAGCAAGGGCCAAGAGTGTTACCGAAGCTGTCAAGCTAACTGGCAACTT

eAlaTyrTyrProValThrAspGlyLysLysGluCysSerAspAlaLeuGluTyrTrpLysGlyGlyLeuSerGl
TGCCTACTACCCCGTCACCGACGGCAAAAAAGAGTGCAGCGATGCTCTGGAGTACTGGAAAGGCGGACTTTCGCA

nPheAsnAspLysIleProProThrPheGlnAlaLeuAsnAsnProAlaValTyrAsnAspArgAlaValSerPh
GTTCAACGATAAAATTCCCCCAACATTTCAAGCGTTGAACAACCCCGCTGTGTACAATGACAGGGCCGTCTCCTT

eValAlaLeuTyrAsnProLysProSerProValValSerCysValLeuLeuGlnCysProAsnAlaGlyGlyGl
TGTCGCCCTATACAACCCCAAACCCAGCCCCGTTGTTAGTTGCGTACTACTCCAGTGCCCTAATGCAGGAGGTGG
```

F16 17

FIG 17a   FIG 17b

Figure 17b

```
**            +
yGlyArgArgLeuAlaAlaGlyThrThrAspAlaValIleCysLeuThrAsnProAlaProLeuAlaAlaGlySe
TGGACGCAGGCTTGCGGCAGGCACGACAGATGCTGTCATTTGCTTGACAAACCCTGCTCCTTTGGCAGCAGGCTC


rProProPheAs|-----------Intron C----------------------------------------
ACCACCATTCGAGTGAGAATCAGCTGTTCGCCACTGCAACATACATCAALGCGGCAGGATACACTGGGGGCACTT


------------------------------------------------------------|pAspGluGlnTrpLysLysIl
GAGGTTGGTTGAAGCGCAATCTTCGGTGA4GCTTGTTTTGTAATTTGCGTGCAGCGACGAGCAATGGAAGAAAAT


eValAspSerLeuSerGluLysLysGlyGlyValSerProValGlyProSerValAlaLeuIleSerAlaAlaVa
TGTTGACTCTCTATCTGAAAAGAAGGGTGGAGTTTCTCCAGTCGGCCCTTCAGTAGCCCTCATCTCTGCGGCGGT


lIleSerAlaPheAlaLeuPheAM
TATCTCCGCTTTCGCTCTCTTCTAGGCGGGCTACACGCAGCATTGGACAGATATGGCAGCGCAAACTCCTTCCTG

AGAGAAATCCTTAAATGACAAACGAGCACCTCTCCTGGACGAAGTGTGATCAAGATAGCTTATAGTGCTTTTGGT

TGTTCGAAGTGAAGTATGCAAAAGCTACATTTGTAGGGCCCTTTTATAGGATAATCGGGAGGAAGCGCAATTTTA

TTTAAAACCCTTGCAGAGAGTCGCCACGTGCGAGTGCAAGTGTTGCGCAGTGTGTGCTGCCAAGTGAAATTCTCG

ATATGTAGGTGTACTCATGCCAGAAGTTTCGGCGTTGATGTACCCACCGGTGGTATACGCCATGCCACGCCTGCC

TGTTTGGGCAGTACAACCTCATACCAAATGGGCATGCTTGTGTCACTGCACGTTTCTGTATCATTAGTGGCCAAG

CAACTGTTTAAAGGGGAAGCAGTGGGGATATTTTGAAGTATTTTGAATAAATACTTTATTTGCTATACCCACCCG

CTGAGGCGCCATGGTGTTGCTGTCCTCATTTGAAGGGGAGAAACTGATTGATAATTTCCTTGTCCTTCAACTTGT

CTTGGTAAATCGAGGAATACGTAGTGAGC3ATTTTTTAGGGAAGATTGTAAACCACAGTAAAACGTTTAGCCGAC

ATTTTCTACACTCGTACGTCGGAAAAGCGCATAAGCTAGA
```

LEGEND

| Nucleotide | Definitely | Probably |
|---|---|---|
| cytosine | C | 3 |
| thymine | T | 4 |
| adenine | A | 5 |
| guanine | G | 6 |
| unknown | N | N |

Either

      C or A    J

      T or A    L

\*   Putative initial amino acid of 18,000 dalton peptide

\*\* Putative final amino acid of 18,000 dalton peptide

\+   Putative initial amino acid of 8,000 dalton peptide

Figure 18

Amino Acid Sequence Homology Between E. tenella and E. necatrix A4 Antigens

```
                              +
                 Exon 1 ←------|-----→ Exon 2
E. tenella  MARLSFVSLLSLSLLFGQQAVRAQD  YPTAVTLDCKEAMNKLRKAAGLPAFEDAVGDTFVLPAYSHEESRAA
            ******************** ***   **** *   * **** ******** *   **** ******** ***
E. necatrix MARLSFVSLLSLSLLFGQQAARAQET YPTAETMECREAMNELRKAAGLPEFGNAVGDAVVLPAYSHEA RAA


            Exon 2 ←-----\-----→ Exon 3
            PVAETLAKTEICPKVLGGGRSRNVTEAVKLTGNFAYYPVTDGKKECSDAVEYWKGGLSQFNDTIPPTFQALN
            ***************** *     ******************************** ************ *********
            PVAETLAKTEICPKVLGGARAKSVTEAVKLTGNFAYYPVTDGKKECSDALEYWKGGLSQFNDKIPPTFQALN


                                                    ↑        Exon 3 ←------|------
            DPVVYNDRAVSFVALYNPKTSPVVSCVLLQCPNAGVGGRRLAAGTTDAVICLTNPAPLEARSQPFDDEQWKK
            * ***************** **************** ********************* * * *********
            NPAVYNDRAVSFVALYNPKPSPVVSCVLLQCPNAGGGGRRLAAGTTDAVICLTNPAPLAAGSPPFDDEQWKR


            ---→ Exon 4
            IVDSLSLSEEEEEKGGVSPVVPSVALISAAVISAFALP
            ******     * ******* ******************
            IVDSLS      EKKGGVSPVGPSVALISAAVISAFALP
```

* = Homologous amino acid
+ = Start of E. tenella 17,000 dalton polypeptide component of the A4 antigen
↑ = Start of E. tenella 8,000 dalton polypeptide component of the A4 antigen


| | | | |
|---|---|---|---|
| C = Cys | A = Ala | F = Phe | D = Asp |
| H = His | G = Gly | R = Arg | N = Asn |
| I = Ile | L = Leu | Y = Tyr | B = Asx |
| M = Met | P = Pro | W = Trp | E = Glu |
| S = Ser | T = Thr | | Q = Gln |
| V = Val | | | Z = Glx |
| | | | K = Lys |


Space in E. tenella amino acid sequence = additional amino acid in E. necatrix sequence compared to E. tenella sequence.

Space in E. necatrix amino acid sequence = amino acids not in E. necatrix sequence compared to E. tenella sequence.

Figure 19

Comparison Of The Three Introns Within The Gene Encoding The A4 Antigen In
E. tenella and E. necatrix

Alignment of Intron A from E. tenella with Intron A from E. necatrix

```
E. tenella   1 GTGGGCTTTTCCGCTAGCTGTTTTTGGTCCGATAGCATCGGAGCATCTCCCAAAACGAGGTGCATTCACCT
               ***************** ********* ** ****** *** *  *********  *******  **
E. necatrix  1 GTGGGCTTTTCCGCTAGCCGTTTTTGGTCTGACAGCATCTGAGTACTTCCCAAAACAGCGTGCATTCTTCT
```

```
            73 TTTGCATGTTGTGTGCGGAAATTTTATCAG
               ******************************
            73 TTTGCATGTTGTGTGCGGAAATTTTATCAG
```

Alignment of Intron B from E. tenella with Intron B from E. necatrix

```
E. tenella   1 GTAAGCCGTCCACGGCCTTGCA TCGTC        ATGATGTAGTAGGTGTTCTGAGCAGCTTCGTTCTG
               ***********  *  ** *** *  *****      *  ****** ******************  ***
E. necatrix  1 GTAAGCCGTCCTCTGCATTGTAGTCGTCCACTGCATTGTCATGTAGCAGGTGTTCTGAGCAGCTTATCTCTT
```

```
            73 TGGAACAAGGAACTACACTGTCCTTGAATTTTTAATCTTTTGTTACGTACAG
               ************* *   *** *  ***** ******* * *  *******
            73 TAAACAAGGAACTACGCCCTCC TCAATTTCTAATCTTTCGCTGCGTACAG
```

Alignment of Intron C from E. tenella with Intron C from E. necatrix

```
E. tenella   1 GTGAGAGTCAGCTGGTCGCCACTGCAACATGCATCAATGCGGCAGGTTACACTGGGGG TC7TGAGGTTGGT
               ****** ******* **************** ****** ******** ********** *  **********
E. necatrix  1 GTGAGAATCAGCTGTTCGCCACTGCAACATACATCAALGCGGCAGGATACACTGGGGGCACTTGAGGTTGGT
```

```
            73 TGAAGCGCAATCTTCTAATACTTGTTTGTAATGTTTGTAATGTTTGCGTGCAG
               ****************        *     *******  ***********
            73 TGAAGCGCAATCTTC        GGTGA4GCTTGTTTTGTAA TTTGCGTGCAG
```

Legend

* = Homology

Space in E. tenella DNA sequence = additional base in E. necatrix sequence compared to E. tenella sequence.

Space in E. necatrix DNA sequence = base not in E. necatrix sequence compared to E. tenella sequence.

Key To Ambiguous Bases

4 = Probably T
7 = Maybe C
L = T or A

Figure 20

coding region, ~650 bp

60 bp

SacI  SacI

untranslated   blunt end

full-length NA4 cDNA
~1.3 Kb
(not to scale)

EcoRI

Bam HI
Hind III

SacI  SmaI

Lac Z
α protein

pUC18

ori.

Amp R

EcoRI  SacI

Bam HI
Hind III

90% of the
coding sequence
for NA4

pSMAC

ori.

Amp R

Figure 21

Figure 22

Base-pair sequence of the RI-SacI region of pSMAC which was removed from pSMAC and replaced with the synthetic DNA of COD's 395 and 396

pSMAC

90% of the coding sequence for NA4

Bam HI
Hind III

Synthetic DNA fragment sequence:

eco RI

GAATTC C GAGCTCAGGAA etc. (same sequence as the SacI-SacI 60 bp fragment cloned into pSS33)

C nucleotide added to sequence immediately upstream of the SacI site

to yield:

" C"nucleotide added

entire mature NA4 coding sequence

pNCD

Bam HI
Hind III

Ori

Amp

Figure 23a

Figure 23b

Figure 24a

Figure 24b